# EUROPEAN PATENT APPLICATION

(11) **EP 3 587 385 A1**
(43) Date of publication of application: **01.01.2020**
(21) Application number: 18758063.4
(22) Date of filing: 21.02.2018
(51) Int. Cl.: C07C 39/15, C07C 39/17, G03F 7/023, G03F 7/11, G03F 7/20, G03F 7/26, G03F 7/039

(54) **COMPOUND, RESIN, COMPOSITION, PATTERN FORMING METHOD AND PURIFICATION METHOD**

(30) Priority: 23.02.2017 JP 2017032249
(71) Applicant: Mitsubishi Gas Chemical Company, Inc., Tokyo 100-8324 (JP)
(72) Inventor: MIKI, Yasushi, Hiratsuka-shi Kanagawa 254-0016 (JP); MAKINOSHIMA, Takashi, Hiratsuka-shi Kanagawa 254-0016 (JP); ECHIGO, Masatoshi, Tokyo 100-8324 (JP)
(74) Representative: Müller-Boré & Partner Patentanwälte PartG mbB
(86) International application number: PCT/JP2018/006239
(87) International publication number: WO 2018/155495

(57) **Abstract**

The present invention provides a compound, a resin and a composition that are excellent in solvent solubility, are applicable to a wet process, are excellent in heat resistance and etching resistance, and are useful for forming a film for lithography. The present invention also provides pattern formation methods using the composition. The present invention further provides a method for purifying the compound and the resin. A composition comprising a compound having a specific structure and/or a resin having a constituent unit derived from the compound is used.

## Description

### Technical Field

The present invention relates to a compound and a resin having a specific structure, a composition comprising the compound and/or the resin, and pattern formation methods using the composition, and a method for purifying a substance.

### Background Art

In the production of semiconductor devices, fine processing is practiced by lithography using photoresist materials. In recent years, further miniaturization based on pattern rules has been demanded along with increase in the integration and speed of LSI. Lithography using light exposure, which is currently used as a general purpose technique, is approaching the limit of essential resolution derived from the wavelength of a light source.

The light source for lithography used upon forming resist patterns has been shifted to ArF excimer laser (193 nm) having a shorter wavelength from KrF excimer laser (248 nm). However, as the miniaturization of resist patterns proceeds, the problem of resolution or the problem of collapse of resist patterns after development arises. Therefore, resists have been desired to have a thinner film. If resists merely have a thinner film in response to such a demand, it is difficult to obtain the film thicknesses of resist patterns sufficient for substrate processing. Therefore, there is a need for a process of preparing a resist underlayer film between a resist and a semiconductor substrate to be processed, and imparting functions as a mask for substrate processing to this resist underlayer film in addition to a resist pattern.

Various resist underlayer films for such a process are currently known. Examples thereof can include resist underlayer films for lithography having the selectivity of a dry etching rate close to that of resists, unlike conventional resist underlayer films having a fast etching rate. As a material for forming such resist underlayer films for lithography, an underlayer film forming material for a multilayer resist process containing a resin component having at least a substituent that generates a sulfonic acid residue by eliminating a terminal group under application of predetermined energy, and a solvent has been suggested (see, for example, Patent Literature 1). Another example thereof can include resist underlayer films for lithography having the selectivity of a dry etching rate smaller than that of resists. As a material for forming such resist underlayer films for lithography, a resist underlayer film material comprising a polymer having a specific repeat unit has been suggested (see, for example, Patent Literature 2). Further examples thereof can include resist underlayer films for lithography having the selectivity of a dry etching rate smaller than that of semiconductor substrates. As a material for forming such resist underlayer films for lithography, a resist underlayer film material comprising a polymer prepared by copolymerizing a repeat unit of an acenaphthylene and a repeat unit having a substituted or unsubstituted hydroxy group has been suggested (see, for example, Patent Literature 3).

Meanwhile, as materials having high etching resistance for this kind of resist underlayer film, amorphous carbon underlayer films formed by CVD using methane gas, ethane gas, acetylene gas, or the like as a raw material are well known. However, resist underlayer film materials that can form resist underlayer films by a wet process such as spin coating or screen printing have been demanded from the viewpoint of a process.

Recently, layers to be processed having a complicated shape have been desired to form a resist underlayer film for lithography. Thus, there is a demand for a resist underlayer film material that can form an underlayer film excellent in embedding properties or film surface flattening properties.

As for methods for forming an intermediate layer used in the formation of a resist underlayer film in a three-layer process, for example, a method for forming a silicon nitride film (see, for example, Patent Literature 4) and a CVD formation method for a silicon nitride film (see, for example, Patent Literature 5) are known. Also, as intermediate layer materials for a three-layer process, materials comprising a silsesquioxane-based silicon compound are known (see, for example, Patent Literature 6 and Patent Literature 7).

The present inventors have suggested an underlayer film forming composition for lithography comprising a specific compound or resin (see, for example, Patent Literature 8).

Various optical component-forming compositions have been suggested, and, for example, an acrylic resin (see, for example, Patent Literatures 9 and 10) and polyphenol having a specific structure derived from an allyl group (see, for example, Patent Literature 11) have been suggested.

### Citation List

### Patent Literature

Patent Literature 1: Japanese Patent Application Laid-Open No. 2004-177668
Patent Literature 2: Japanese Patent Application Laid-Open No. 2004-271838
Patent Literature 3: Japanese Patent Application Laid-Open No. 2005-250434
Patent Literature 4: Japanese Patent Application Laid-Open No. 2002-334869
Patent Literature 5: International Publication No. WO 2004/066377
Patent Literature 6: Japanese Patent Application Laid-Open No. 2007-226170
Patent Literature 7: Japanese Patent Application Laid-Open No. 2007-226204
Patent Literature 8: International Publication No. WO 2013/024779
Patent Literature 9: Japanese Patent Application Laid-Open No. 2010-138393
Patent Literature 10: Japanese Patent Application Laid-Open No. 2015-174877
Patent Literature 11: International Publication No. WO 2014/123005

### Summary of Invention

### Technical Problem

As mentioned above, a large number of underlayer film forming materials for lithography have heretofore been suggested. However, none of these materials not only have high solvent solubility that permits application of a wet process such as spin coating or screen printing but achieve both of heat resistance and etching resistance at high dimensions. Thus, the development of novel materials is required.

Also, a large number of compositions intended for optical members have heretofore been suggested. However, none of these compositions achieve all of heat resistance, transparency and an index of refraction at high dimensions. Thus, the development of novel materials is required.

The present invention has been made in light of the problems described above. Specifically, an object of the present invention is to provide a compound, a resin and a composition that are excellent in solvent solubility, are applicable to a wet process, are excellent in heat resistance and etching resistance, and are useful for forming a film for lithography. Another object of the present invention is to provide pattern formation methods using the composition.

### Solution to Problem

The inventors have, as a result of devoted examinations to solve the above problems, found out that use of a compound having a specific structure can solve the above problems, and reached the present invention.

More specifically, the present invention is as follows.
[1] A compound represented by the following formula (A): 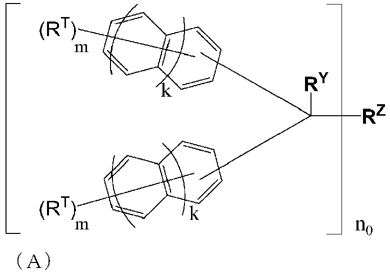 wherein
   R^{Y} is a hydrogen atom, a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent or an aryl group having 6 to 30 carbon atoms optionally having a substituent;
   R^{Z} is a no-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, a hydroxy group and a group in which a hydrogen atom of a hydroxy group is replaced with an acid dissociation group;
   each R^{T} is independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein at least one R^{T} is a hydroxy group or a group in which a hydrogen atom of a hydroxy group is replaced with an acid dissociation group;
   each m is independently an integer of 0 to 9, wherein at least one m is an integer of 1 to 9;
   n₀ is an integer of 1 to 4, wherein when n₀ is an integer of 2 or larger, n₀ structural formulae within the parentheses [ ] are the same or different; and
   each k is independently an integer of 0 to 2.
[2] The compound according to the above [1], wherein at least one of R^{Y} and R^{Z} has a hydroxy group or a group in which a hydrogen atom of a hydroxy group is replaced with an acid dissociation group.
[3] The compound according to the above [1] or [2], wherein the compound represented by the above formula (A) is a compound represented by the following formula (1): 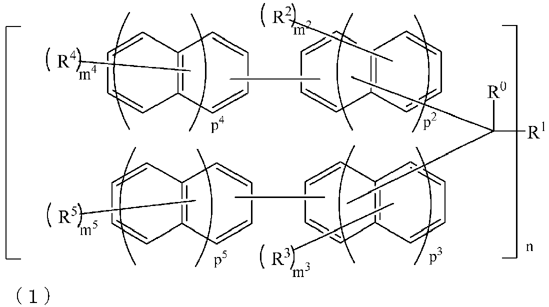 wherein
   R⁰ is as defined in the above R^{Y};
   R¹ is as defined in the above R^{Z};
   R² to R⁵ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein
   at least one of R² to R⁵ is a hydroxy group or a group in which a hydrogen atom of a hydroxy group is replaced with an acid dissociation group;
   m² and m³ are each independently an integer of 0 to 8;
   m⁴ and m⁵ are each independently an integer of 0 to 9,
   provided that m², m³, m⁴ and m⁵ are not 0 at the same time;
   n is as defined in the above n₀, wherein when n is an integer of 2 or larger, n structural formulae within the parentheses [ ] are the same or different; and
   p₂ to p₅ are each independently an integer of 0 to 2.
[4] The compound according to the above [3], wherein at least one of R⁰ and R¹ has a hydroxy group or a group in which a hydrogen atom of a hydroxy group is replaced with an acid dissociation group.
[5] The compound according to the above [3] or [4], wherein the compound represented by the above formula (1) is a compound represented by the following formula (1-1): 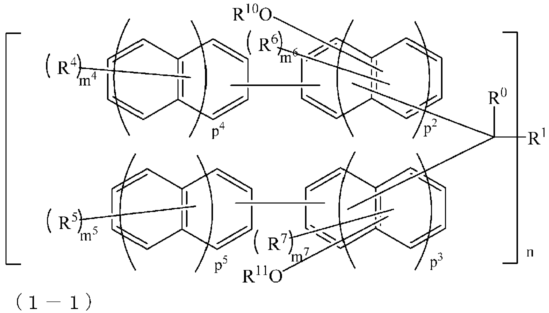 wherein
   R⁰, R¹ , R⁴, R⁵, n, p² to p⁵, m⁴ and m⁵ are as defined above;
   R⁶ and R⁷ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, a halogen atom, a nitro group, an amino group, a carboxyl group, or a thiol group;
   R¹⁰ and R¹¹ are each independently an acid dissociation group or a hydrogen atom, wherein at least one of R¹⁰ and R¹¹ is a hydrogen atom; and
   m⁶ and m⁷ are each independently an integer of 0 to 7.
[6] The compound according to the above [5], wherein the compound represented by the above formula (1-1) is a compound represented by the following formula (1-2): 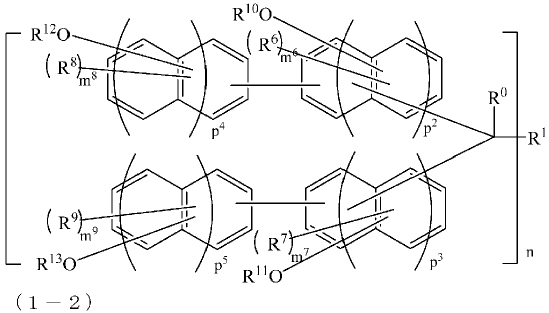 wherein
   R⁰, R¹, R⁶, R⁷, R¹⁰, R¹¹, n, p² to p⁵, m⁶ and m⁷ are as defined above;
   R⁸ and R⁹ are as defined in the above R⁶ and R⁷;
   R¹² and R¹³ are as defined in the above R¹⁰ and R¹¹; and
   m⁸ and m⁹ are each independently an integer of 0 to 8.
[7] The compound according to the above [6], wherein the compound represented by the above formula (1-2) is a compound selected from the group consisting of compounds represented by the following formulae: 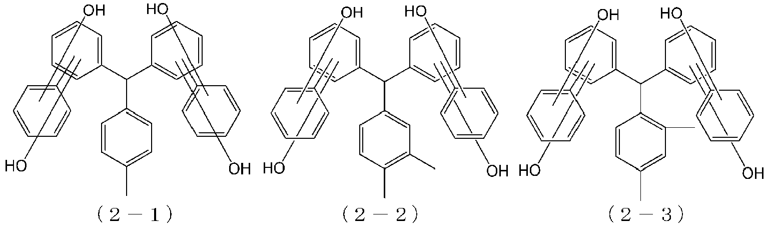 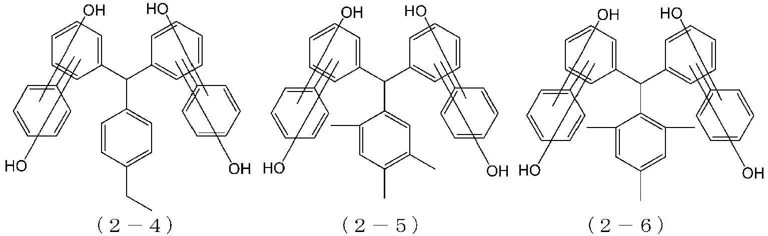 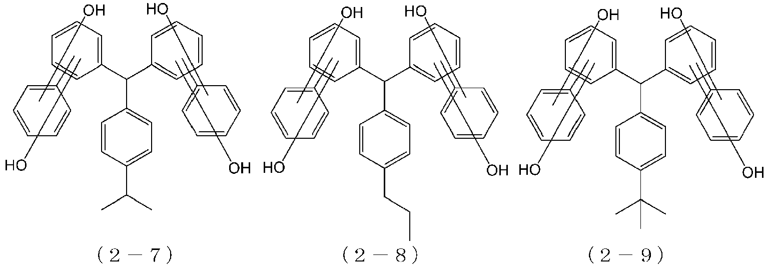 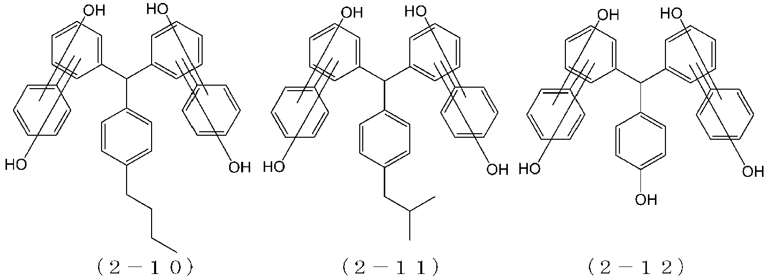 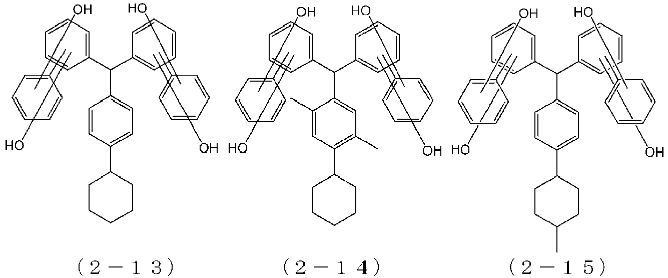 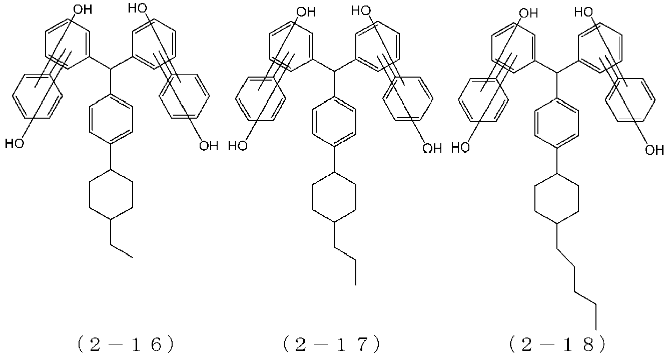 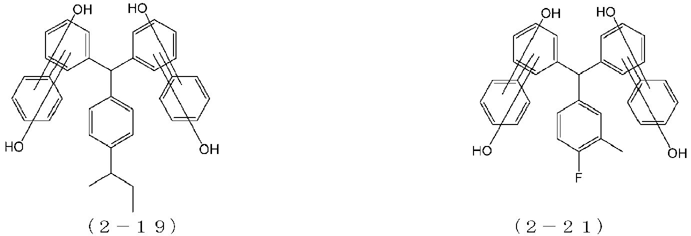 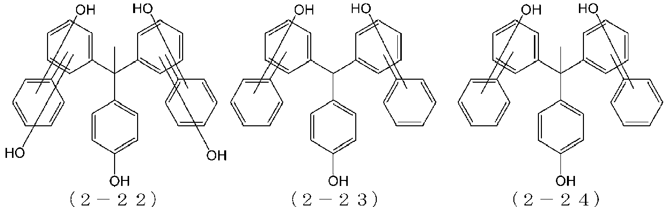 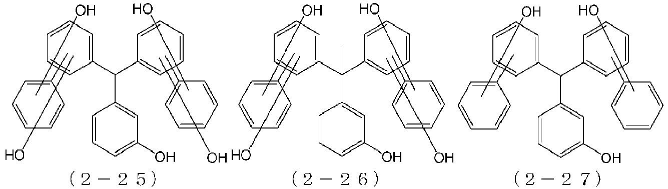 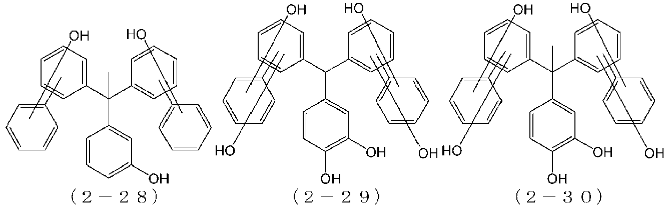 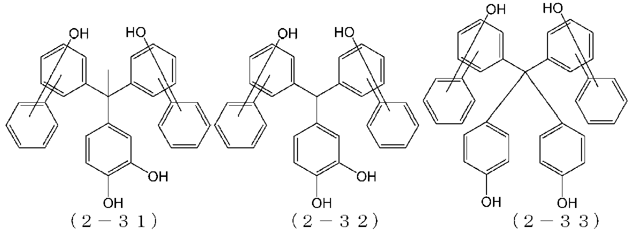 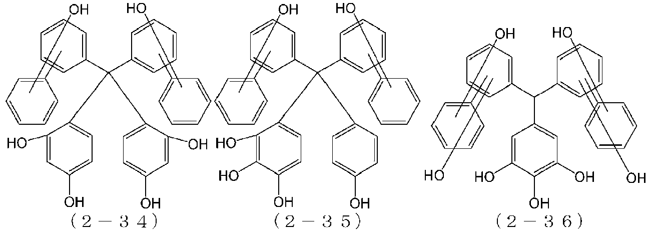
[8] A resin having a constituent unit derived from the compound according to any one of the above [1] to [7].
[9] A composition comprising one or more selected from the group consisting of the compound according to any one of the above [1] to [7] and the resin according to the above [8].
[10] A composition for forming a optical component comprising one or more selected from the group consisting of the compound according to any one of the above [1] to [7] and the resin according to [8].
[11] A film forming composition for lithography comprising one or more selected from the group consisting of the compound according to any one of the above [1] to [7] and the resin according to the above [8].
[12] A resist composition comprising one or more selected from the group consisting of the compound according to any one of the above [1] to [7] and the resin according to the above [8].
[13] The resist composition according to the above [12], further comprising a solvent.
[14] The resist composition according to the above [12] or [13], further comprising an acid generating agent.
[15] The resist composition according to any one of the above [12] to [14], further comprising an acid diffusion controlling agent.
[16] A method for forming a resist pattern, comprising the steps of:
   forming a resist film on a supporting material using the resist composition according to any one of the above [12] to [15];
   exposing at least a portion of the formed resist film; and
   developing the exposed resist film, thereby forming a resist pattern.
[17] A radiation-sensitive composition comprising
   a component (A) which is one or more selected from the group consisting of the compound according to any one of the above [1] to [7] and the resin according to the above [8],
   an optically active diazonaphthoquinone compound (B), and
   a solvent, wherein
   the content of the solvent is 20 to 99% by mass based on 100% by mass in total of the radiation-sensitive composition, and
   the content of components except for the solvent is 1 to 80% by mass based on 100% by mass in total of the radiation-sensitive composition.
[18] The radiation-sensitive composition according to the above [17], wherein the content ratio among the component (A), the optically active diazonaphthoquinone compound (B), and a further optional component (D) optionally comprised in the radiation-sensitive composition ((A)/(B)/(D)) is 1 to 99% by mass/99 to 1% by mass/0 to 98% by mass based on 100% by mass of solid components of the radiation-sensitive composition.
[19] The radiation-sensitive composition according to the above [17] or [18], wherein the radiation-sensitive composition is capable of forming an amorphous film by spin coating.
[20] A method for producing an amorphous film, comprising the step of forming an amorphous film on a supporting material using the radiation-sensitive composition according to any one of the above [17] to [19].
[21] A method for forming a resist pattern, comprising the steps of:
   forming a resist film on a supporting material using the radiation-sensitive composition according to any one of the above [17] to [19];
   exposing at least a portion of the formed resist film; and
   developing the exposed resist film, thereby forming a resist pattern.
[22] An underlayer film forming material for lithography comprising one or more selected from the group consisting of the compound according to any one of the above [1] to [7] and the resin according to the above [8].
[23] A composition for forming an underlayer film for lithography comprising the underlayer film forming material for lithography according to the above [22], and a solvent.
[24] The composition for forming an underlayer film for lithography according to the above [23], further comprising an acid generating agent.
[25] The composition for forming an underlayer film for lithography according to the above [23] or [24], further comprising a crosslinking agent.
[26] A method for producing an underlayer film for lithography, comprising the step of forming an underlayer film on a supporting material using the composition for forming an underlayer film for lithography according to any one of the above [23] to [25].
[27] A method for forming a resist pattern, comprising the steps of:
   forming an underlayer film on a supporting material using the composition for forming an underlayer film for lithography according to any one of the above [23] to [25] ;
   forming at least one photoresist layer on the underlayer film; and
   irradiating a predetermined region of the photoresist layer with radiation for development, thereby forming a resist pattern.
[28] A method for forming a circuit pattern, comprising the steps of:
   forming an underlayer film on a supporting material using the composition for forming an underlayer film for lithography according to any one of the above [23] to [25] ;
   forming an intermediate layer film on the underlayer film using a resist intermediate layer film material comprising a silicon atom;
   forming at least one photoresist layer on the intermediate layer film;
   irradiating a predetermined region of the photoresist layer with radiation for development, thereby forming a resist pattern;
   etching the intermediate layer film with the resist pattern as a mask, thereby forming an intermediate layer film pattern;
   etching the underlayer film with the intermediate layer film pattern as an etching mask, thereby forming an underlayer film pattern; and
   etching the supporting material with the underlayer film pattern as an etching mask, thereby forming a pattern on the supporting material.
[29] A purification method comprising the steps of:
   obtaining a solution (S) by dissolving one or more selected from the group consisting of the compound according to any one of the above [1] to [7] and the resin according to the above [8] in a solvent; and
   extracting impurities in the compound and/or the resin by bringing the obtained solution (S) into contact with an acidic aqueous solution (a first extraction step), wherein
   the solvent used in the step of obtaining the solution (S) comprises a solvent that is incompatible with water.
[30] The purification method according to the above [29], wherein
   the acidic aqueous solution is an aqueous mineral acid solution or an aqueous organic acid solution;
   the aqueous mineral acid solution is an aqueous mineral acid solution in which one or more selected from the group consisting of hydrochloric acid, sulfuric acid, nitric acid, and phosphoric acid is dissolved in water; and
   the aqueous organic acid solution is an aqueous organic acid solution in which one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid is dissolved in water.
[31] The purification method according to [29] or [30], wherein the solvent that is incompatible with water is one or more solvents selected from the group consisting of toluene, 2-heptanone, cyclohexanone, cyclopentanone, methyl isobutyl ketone, propylene glycol monomethyl ether acetate, and ethyl acetate.
[32] The purification method according to any one of the above [29] to [31], comprising the step of extracting impurities in the compound and/or the resin by further bringing a solution phase comprising the compound and/or the resin into contact with water after the first extraction step (a second extraction step).

The present invention can provide a compound, a resin and a composition that are excellent in solvent solubility, are applicable to a wet process, are excellent in heat resistance and etching resistance, and are useful for forming a film for lithography.

### Description of Embodiments

Hereinafter, embodiments of the present invention (hereinafter, also simply referred to as the "present embodiment") will be described. The embodiments described below are given merely for illustrating the present invention. The present invention is not limited only by these embodiments.

As for structural formulae described in the present specification, for example, when a line indicating a bond to C is in contact with ring A and ring B as described below, each C is meant to be bonded to any one or both of the ring A and the ring B.

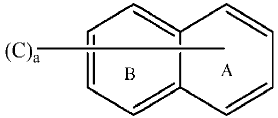

As for structural formulae described in the present specification, for example, the following structure: 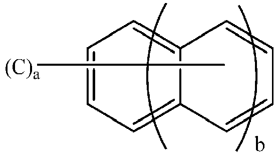 represents the following structure: 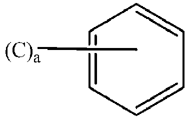 when b is 0, and the following structure: 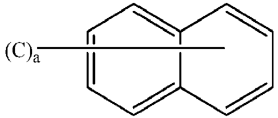 when b is 1.

### [Compound represented by formula (A)]

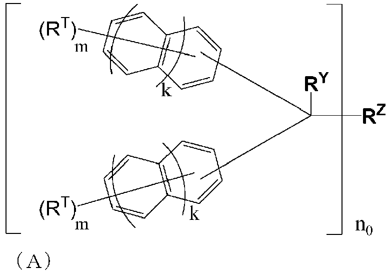

wherein
R^{Y} is a hydrogen atom, a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent or an aryl group having 6 to 30 carbon atoms optionally having a substituent;
R^{Z} is a m-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, a hydroxy group and a group in which a hydrogen atom of a hydroxy group is replaced with an acid dissociation group;
each R^{T} is independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein at least one R^{T} is a hydroxy group or a group in which a hydrogen atom of a hydroxy group is replaced with an acid dissociation group;
each m is independently an integer of 0 to 9, wherein at least one m is an integer of 1 to 9;
n₀ is an integer of 1 to 4, wherein when n₀ is an integer of 2 or larger, n₀ structural formulae within the parentheses [ ] are the same or different; and
each k is independently an integer of 0 to 2.

At least one of R^{Y} and R^{Z} preferably has a hydroxy group or a group in which a hydrogen atom of a hydroxy group is replaced with an acid dissociation group, from the viewpoint of curability.

R^{Y} in the above formula (A) is a hydrogen atom, a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent or an aryl group having 6 to 30 carbon atoms optionally having a substituent. The alkyl group and the aryl group may each have an ether bond, a ketone bond or an ester bond. The above cyclic alkyl group also includes bridged alicyclic alkyl groups.

Herein, examples of the linear alkyl group of 1 to 30 carbon atoms optionally having a substituent include a methyl group, an ethyl group, a propyl group, a butyl group, a pentyl group, a hexyl group, a heptyl group, an octyl group, a nonyl group, a decyl group, an icosyl group, a triacontyl group, a cyclopropylmethyl group, a cyclohexylmethyl group, and an adamantylmethyl group.

Examples of the branched alkyl group of 3 to 30 carbon atoms optionally having a substituent include a 1-methylethyl group, a 2-methylpropyl group, a 2-methylbutyl group, a 2-methylpentyl group, a 2-methylhexyl group, a 2-methylheptyl group, a 2-methyloctyl group, a 2-methylnonyl group, a 2-methyldecyl group, a 2-methylicosyl group, and a 2-methylnonacosyl group.

Examples of the cyclic alkyl group having 3 to 30 carbon atoms optionally having a substituent include a cyclopropyl group, a cyclobutyl group, a cyclopentyl group, a cyclohexyl group, a cycloheptyl group, a cyclooctyl group, a cyclononyl group, a cyclodecyl group, a cyclooctadecylene group, and an adamantyl group.

Examples of the aryl group having 6 to 30 carbon atoms optionally having a substituent include a methylphenyl group, an ethylphenyl group, a propylphenyl group, a butylphenyl group, a pentylphenyl group, a hexylphenyl group, a heptylphenyl group, an octylphenyl group, a nonylphenyl group, a decylphenyl group, an icosylphenyl group, a pentacosylphenyl group, a dimethylphenyl group, a diethylphenyl group, a dipropylphenyl group, a dibutylphenyl group, a dipentylphenyl group, a dihexylphenyl group, a diheptylphenyl group, a dioctylphenyl group, a dinonylphenyl group, a didecylphenyl group, a didodecylphenyl group, a trimethylphenyl group, a triethylphenyl group, a butylmethylphenyl group, a butylethylphenyl group, a hydroxyphenyl group, a dihydroxyphenyl group, a tetrahydroxyphenyl group, a fluoromethylphenyl group, a fluoroethylphenyl group, a cyclohexylphenyl group, a cyclohexylnaphthyl group, a methylcyclohexylphenyl group, an ethylcyclohexylphenyl group, a propylcyclohexylphenyl group, and a pentylcyclohexylphenyl group.

Among them, R^{Y} is preferably a hydrogen atom, a methyl group, an ethyl group, a propyl group, a butyl group, a 1-methylethyl group, a 2-methylpropyl group, a 2-methylbutyl group, a 2-methylpentyl group, a cyclopropyl group, a cyclobutyl group, a cyclopentyl group, a cyclohexyl group, a methylphenyl group, an ethylphenyl group, a propylphenyl group, or a butylphenyl group, and more preferably a hydrogen atom, a methyl group, an ethyl group, a propyl group, a butyl group, or a cyclohexyl group, from the viewpoint of solvent solubility and heat resistance. A hydrogen atom is particularly preferable from the viewpoint of solvent solubility.

R^{Z} in the above formula (A) is a no-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, a hydroxy group and a group in which a hydrogen atom of a hydroxy group is replaced with an acid dissociation group.

The above no-valent group refers to an alkyl group having 6 to 60 carbon atoms containing an aryl group having 6 to 30 carbon atoms having a substituent, an alkylene group having 6 to 60 carbon atoms containing an aryl group having 6 to 30 carbon atoms having a substituent, an alkanetriyl group having 6 to 60 carbon atoms containing an aryl group having 6 to 30 carbon atoms having a substituent, an alkanetetrayl group having 6 to 60 carbon atoms containing an aryl group having 6 to 30 carbon atoms having a substituent, a monovalent aromatic group having 6 to 60 carbon atoms containing an aryl group having 6 to 30 carbon atoms having a substituent, a divalent aromatic group having 6 to 60 carbon atoms containing an aryl group having 6 to 30 carbon atoms having a substituent, a trivalent aromatic group having 6 to 60 carbon atoms containing an aryl group having 6 to 30 carbon atoms having a substituent, or a tetravalent aromatic group having 6 to 60 carbon atoms containing an aryl group having 6 to 30 carbon atoms having a substituent.

Herein, the above aryl group has at least one linear alkyl group having 1 to 30 carbon atoms optionally having a substituent, branched alkyl group having 3 to 30 carbon atoms optionally having a substituent, cyclic alkyl group having 3 to 30 carbon atoms optionally having a substituent, hydroxy group, or group in which a hydrogen atom of a hydroxy group is replaced with an acid dissociation group. Also, the above no-valent group may have a double bond or a heteroatom and may further have an aromatic group having 6 to 30 carbon atoms. The above cyclic alkyl group also includes bridged alicyclic alkyl groups.

The above no-valent group is preferably an alkyl group having 6 to 60 carbon atoms containing an aryl group having 6 to 30 carbon atoms having a substituent, an alkylene group having 6 to 60 carbon atoms containing an aryl group having 6 to 30 carbon atoms having a substituent, a monovalent aromatic group having 6 to 60 carbon atoms containing an aryl group having 6 to 30 carbon atoms having a substituent, or a divalent aromatic group having 6 to 60 carbon atoms containing an aryl group having 6 to 30 carbon atoms having a substituent, from the viewpoint of heat resistance and flatness.

Examples of the 1-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, a hydroxy group and a group in which a hydrogen atom of a hydroxy group is replaced with an acid dissociation group include, but not particularly limited to, a methylphenyl group, a dimethylphenyl group, a trimethylphenyl group, an ethylphenyl group, a propylphenyl group, a butylphenyl group, a pentaphenyl group, a butylmethylphenyl group, a hydroxyphenyl group, a dihydroxyphenyl group, a fluoromethylphenyl group, and a cyclohexylphenyl group. Herein, for example, the propyl group includes a n-propyl group, an i-propyl group, a cyclopropyl group, and the like, and the butyl group includes a n-butyl group, a t-butyl group, an i-butyl group, a s-butyl group, a cyclobutyl group, and the like.

Examples of the 2-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms, a cyclic alkyl group having 3 to 30 carbon atoms, a hydroxy group and a group in which a hydrogen atom of a hydroxy group is replaced with an acid dissociation group include, but not particularly limited to, a methylphenylene group, a dimethylphenylene group, a trimethylphenylene group, an ethylphenylene group, a propylphenylene group, a butylphenylene group, a pentaphenylene group, a butylmethylphenylene group, a hydroxyphenylene group, dihydroxyphenylene, and a fluoromethylphenylene group.

Examples of the 3-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms, a cyclic alkyl group having 3 to 30 carbon atoms, a hydroxy group and a group in which a hydrogen atom of a hydroxy group is replaced with an acid dissociation group include, but not particularly limited to a methylbenzenetriyl group, a dimethylbenzenetriyl group, a trimethylbenzenetriyl group, an ethylbenzenetriyl group, a propylbenzenetriyl group, a butylbenzenetriyl group, a pentabenzenetriyl group, a butylmethylbenzenetriyl group, a hydroxybenzenetriyl group, dihydroxybenzenetriyl, and a fluoromethylbenzenetriyl group.

Examples of the 4-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms, a cyclic alkyl group having 3 to 30 carbon atoms, a hydroxy group and a group in which a hydrogen atom of a hydroxy group is replaced with an acid dissociation group include, but not particularly limited to, a methylbenzenetetrayl group, a dimethylbenzenetetrayl group, an ethylbenzenetetrayl group, a propylbenzenetetrayl group, a butylbenzenetetrayl group, a pentabenzenetetrayl group, a butylmethylbenzenetetrayl group, a hydroxybenzenetetrayl group, dihydroxybenzenetetrayl, and a fluoromethylbenzenetetrayl group.

Among them, a methylphenyl group, a dimethylphenyl group, a trimethylphenyl group, an ethylphenyl group, a propylphenyl group, a butylphenyl group, a pentaphenyl group, and a butylmethylphenyl group are preferable from the viewpoint of heat resistance. Among them, a methylphenyl group, a dimethylphenyl group, an ethylphenyl group, a propylphenyl group, and a butylphenyl group are particularly preferable from the viewpoint of the availability of raw materials.

Each R^{T} in the above formula (A) is independently a group selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group and a group in which a hydrogen atom of a hydroxy group is replaced with an acid dissociation group. Herein, at least one R^{T} is a hydroxy group or a group in which a hydrogen atom of a hydroxy group is replaced with an acid dissociation group. Each m is independently an integer of 0 to 9. Herein, at least one m is an integer of 1 to 9. n⁰ is an integer of 1 to 4, and each k is independently an integer of 0 to 2.

Herein, examples of the linear alkyl group having 1 to 30 carbon atoms optionally having a substituent include the same as those described above.

Examples of the branched alkyl group having 3 to 30 carbon atoms optionally having a substituent include the same as those described above.

Examples of the cyclic alkyl group having 3 to 30 carbon atoms optionally having a substituent include the same as those described above.

Examples of the aryl group having 6 to 30 carbon atoms optionally having a substituent include the same as those described above.

Examples of the alkenyl group having 2 to 30 carbon atoms optionally having a substituent include an ethenyl group, a propenyl group, a butenyl group, a pentenyl group, a hexenyl group, a heptenyl group, an octenyl group, a nonenyl group, a decenyl group, an icosenyl group, and a triacontenyl group.

Examples of the alkoxy group having 1 to 30 carbon atoms optionally having a substituent include a methoxy group, an ethoxy group, a n-propoxy group, an isopropoxy group, a n-butoxy group, an isobutoxy group, a t-butoxy group, a phenoxy group, a 1-naphthoxy group, and a 2-naphthoxy group.

In the present specification, the acid dissociation group refers to a characteristic group that is cleaved in the presence of an acid to cause a change such as an alkali soluble group. Examples of the alkali soluble group include a phenolic hydroxy group, a carboxyl group, a sulfonic acid group, and a hexafluoroisopropanol group. A phenolic hydroxy group and a carboxyl group are preferable, and a phenolic hydroxy group is particularly preferable. The acid dissociation group can be arbitrarily selected and used from among those proposed in hydroxystyrene-based resins, (meth)acrylic acid-based resins, and the like for use in chemically amplified resist compositions for KrF or ArF. For example, an acid dissociation group described in Japanese Patent Application Laid-Open No. 2012-136520 is used, though the acid dissociation group is not limited thereto.

The compound of the present embodiment has the structure as described above and therefore has both high heat resistance and high solvent solubility. The compound of the present embodiment is preferably a compound represented by the following formula (1) from the viewpoint of solubility in a solvent and heat resistance. 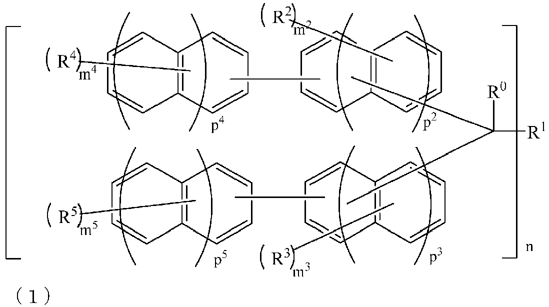 wherein
R⁰ is as defined in the above R^{Y};
R¹ is as defined in the above R^{Z};
R² to R⁵ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein
at least one of R² to R⁵ is a hydroxy group or a group in which a hydrogen atom of a hydroxy group is replaced with an acid dissociation group;
m² and m³ are each independently an integer of 0 to 8;
m⁴ and m⁵ are each independently an integer of 0 to 9,
provided that m², m³, m⁴ and m⁵ are not 0 at the same time;
n is as defined in the above n⁰, wherein when n is an integer of 2 or larger, n structural formulae within the parentheses [ ] are the same or different; and
p₂ to p₅ are each independently an integer of 0 to 2.

At least one of R⁰ and R¹ preferably has a hydroxy group or a group in which a hydrogen atom of a hydroxy group is replaced with an acid dissociation group, from the viewpoint of solubility in a solvent and curability.

The compound of the present embodiment is also preferably a compound represented by the following formula (1-1) from the viewpoint of solubility in a solvent and heat resistance. 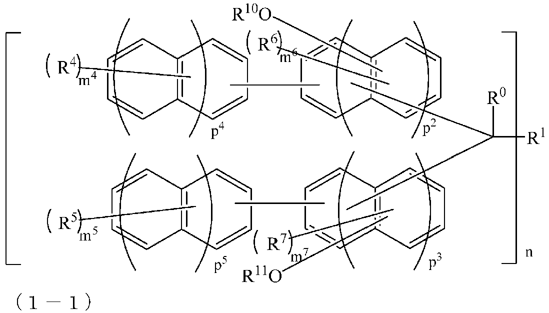 wherein
R⁰, R, R⁴, R⁵, n, p² to p⁵, m⁴ and m⁵ are as defined above;
R⁶ and R⁷ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, a halogen atom, a nitro group, an amino group, a carboxyl group, or a thiol group;
R¹⁰ and R¹¹ are each independently an acid dissociation group or a hydrogen atom, wherein at least one of R¹⁰ and R¹¹ is a hydrogen atom; and
m⁶ and m⁷ are each independently an integer of 0 to 7.

The compound of the present embodiment is also preferably a compound represented by the following formula (1-2) from the viewpoint of solubility in a solvent and heat resistance. 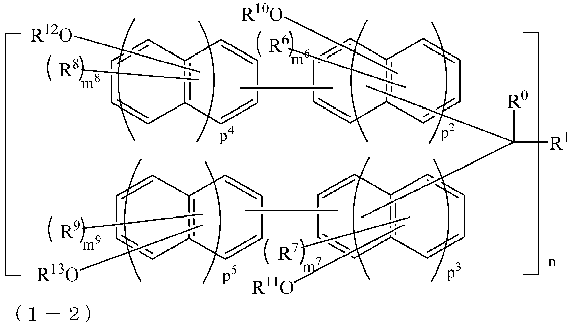 wherein
R⁰, R¹, R⁶, R⁷, R¹⁰, R¹¹, n, p² to p⁵, m⁶ and m⁷ are as defined above;
R⁸ and R⁹ are as defined in the above R⁶ and R⁷;
R¹² and R¹³ are as defined in the above R¹⁰ and R¹¹; and
m⁸ and m⁹ are each independently an integer of 0 to 8.

In the formula (1-2), preferably,
R⁰ is a hydrogen atom;
R¹ is a phenyl group substituted at least with a substituent selected from the group consisting of a linear alkyl group of 1 to 6 carbon atoms, a branched alkyl group having 3 to 6 carbon atoms, a cyclic alkyl group having 3 to 6 carbon atoms and a hydroxy group, and optionally further substituted with a substituent selected from the group consisting of an aryl group having 6 to 10 carbon atoms and a fluorine atom (wherein the cyclic alkyl group having 3 to 6 carbon atoms and the aryl group having 6 to 10 carbon atoms are each optionally substituted with a substituent selected from the group consisting of a linear alkyl group having 1 to 6 carbon atoms, a branched alkyl group having 3 to 6 carbon atoms, a cyclic alkyl group having 3 to 6 carbon atoms, and an aryl group having 6 to 10 carbon atoms);
each of R¹⁰ to R¹³ is a hydrogen atom;
n is 1;
each of p² to p⁵ is 0; and
each of m⁶ to m⁹ is 0.

In the formula (1-2), preferably,
R⁰ is a hydrogen atom;
R¹ is a phenyl group substituted with a substituent selected from the group consisting of a linear alkyl group having 1 to 6 carbon atoms, a branched alkyl group having 3 to 6 carbon atoms, a cyclic alkyl group having 3 to 6 carbon atoms and a hydroxy group;
each of R¹⁰ to R¹³ is a hydrogen atom;
n is 1;
each of p² to p⁵ is 0; and
each of m⁶ to m⁹ is 0.

In the present embodiment, specific examples of the compound represented by the above formula (1-2) include, but not limited to, those described below, from the viewpoint of heat resistance and solubility in an organic solvent. A compound selected from the group consisting of compounds represented by the following formulae (2-1) to (2-19) and the formulae (2-21) to (2-24) is preferable.

A compound selected from the group consisting of compounds represented by the following formula (2-12) and the formula (2-22) to the formula (2-24) is preferable from the viewpoint of curability.

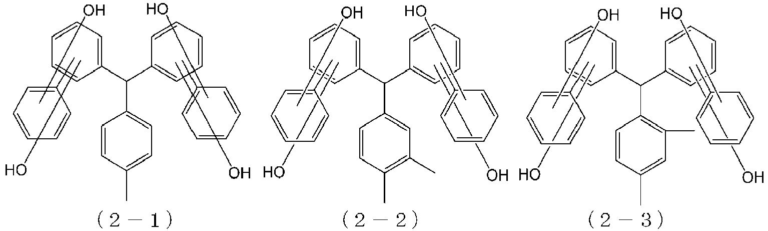

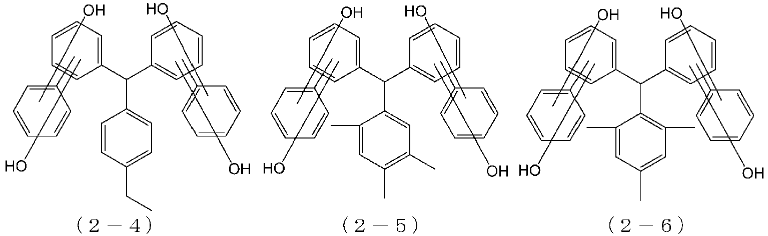

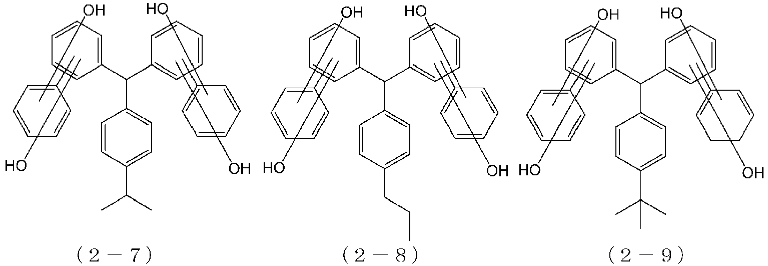

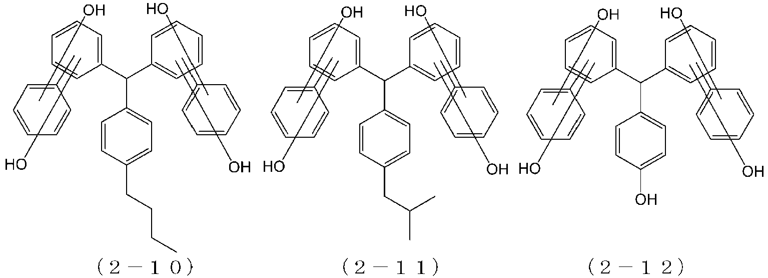

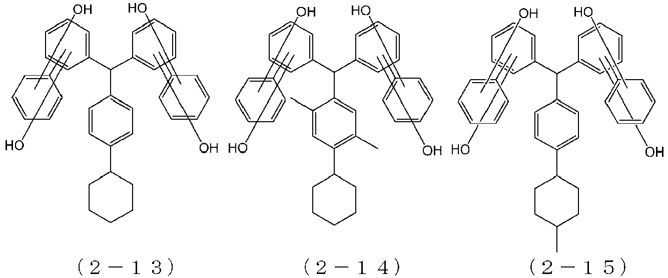

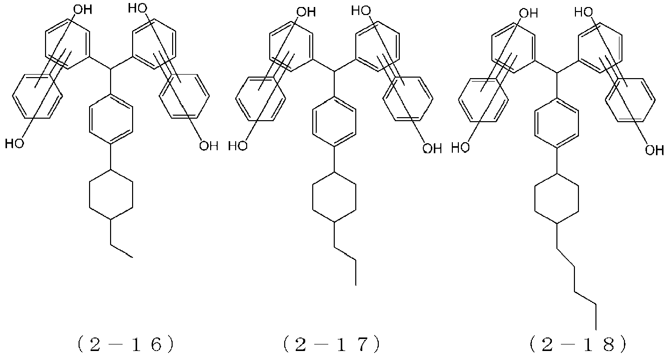

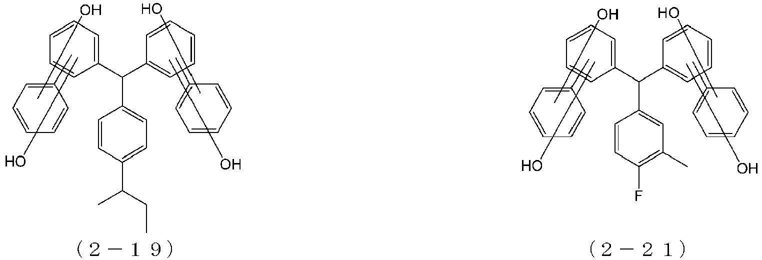

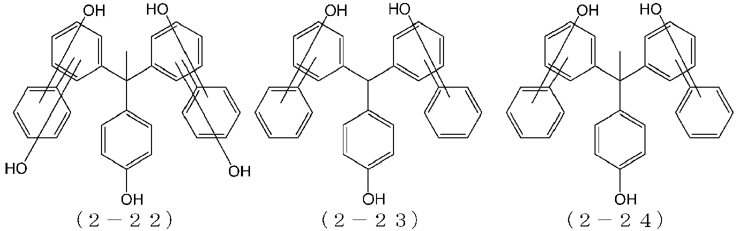

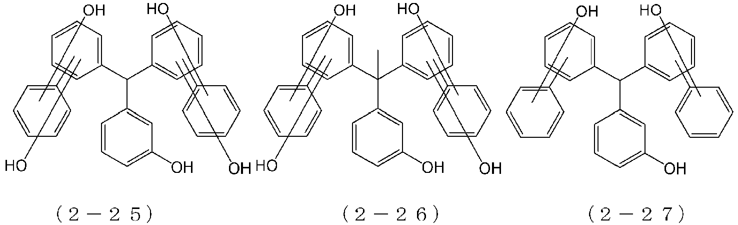

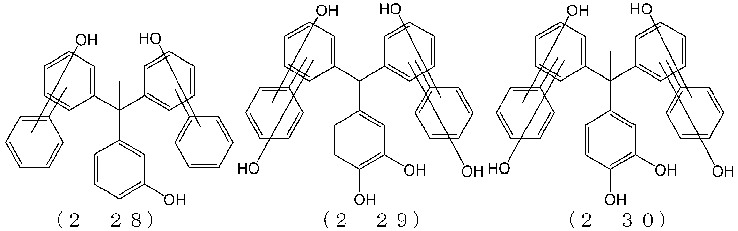

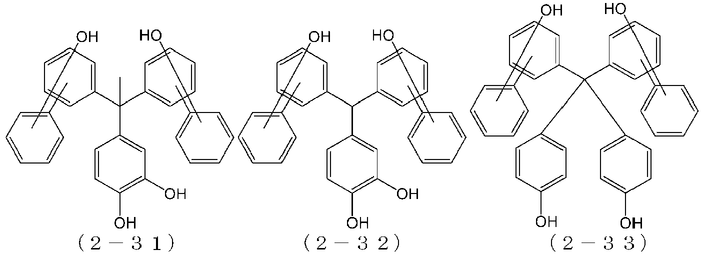

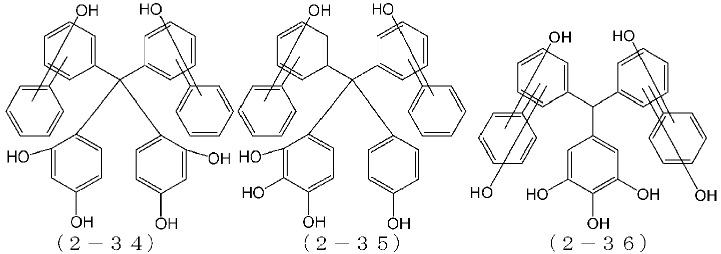

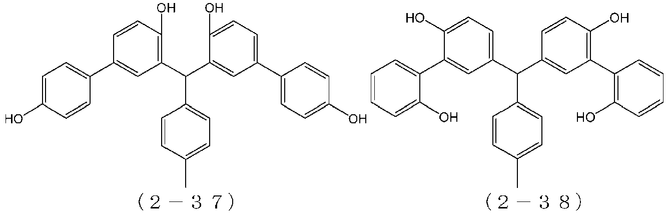

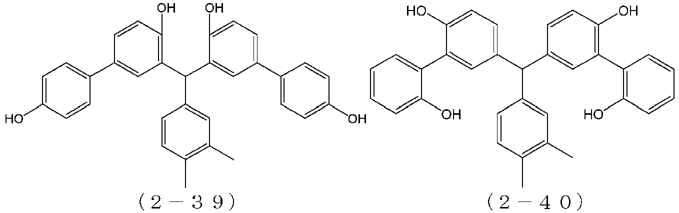

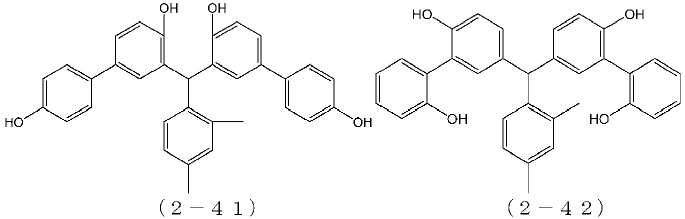

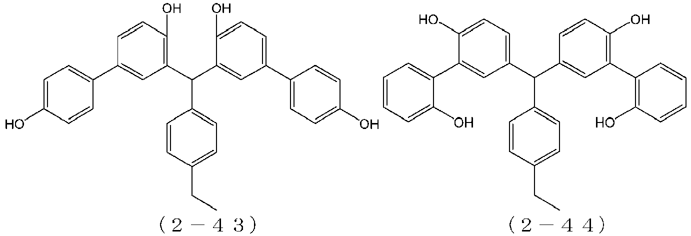

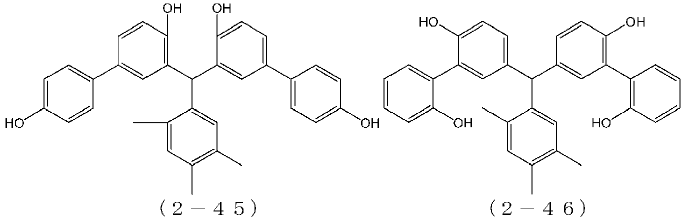

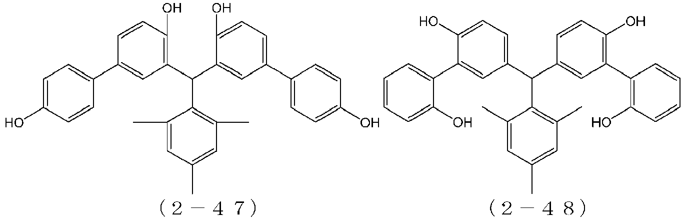

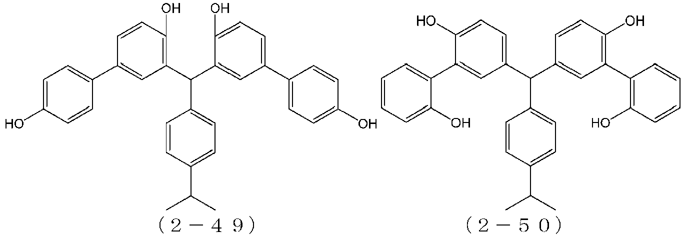

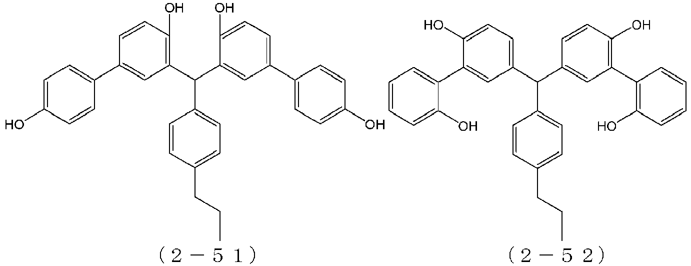

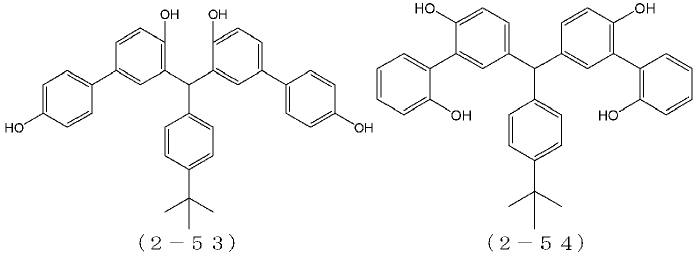

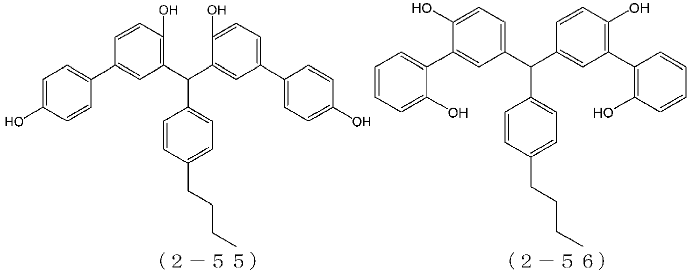

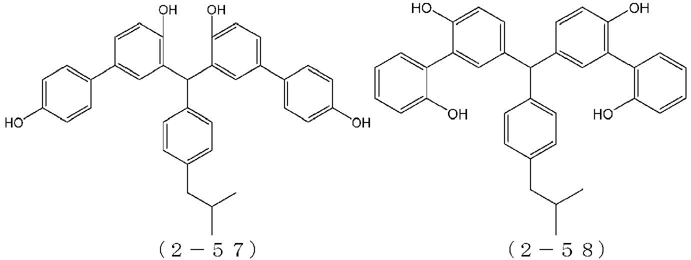

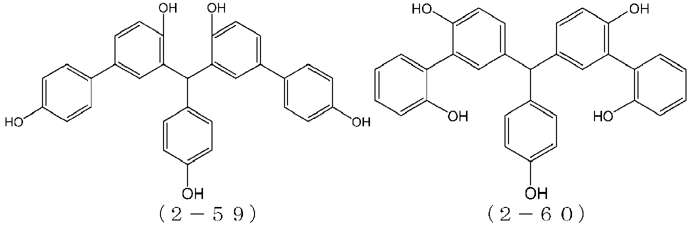

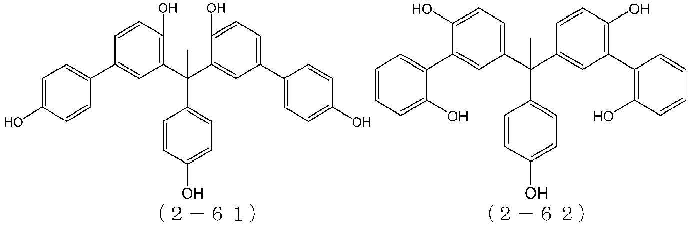

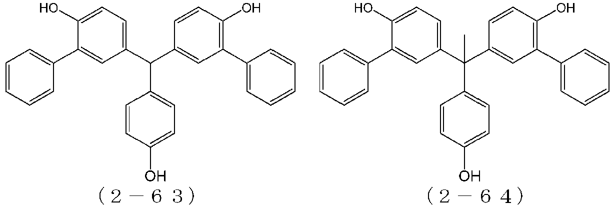

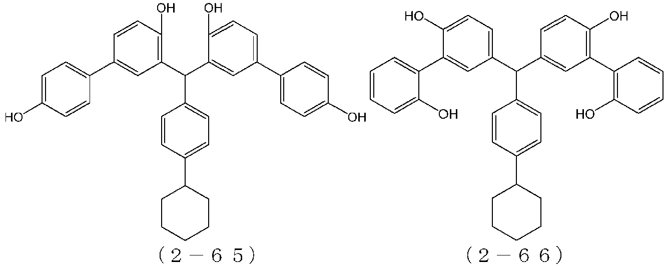

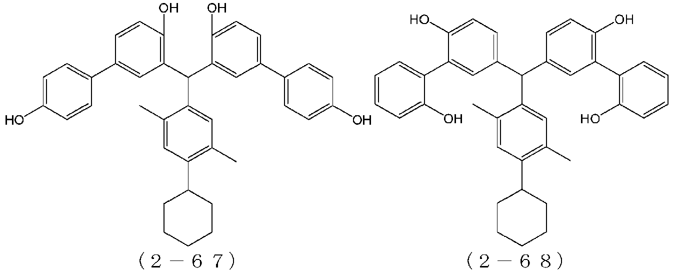

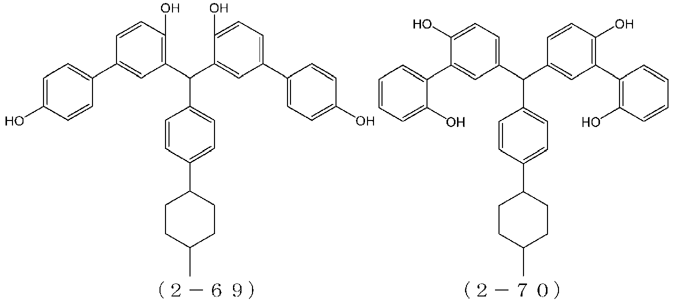

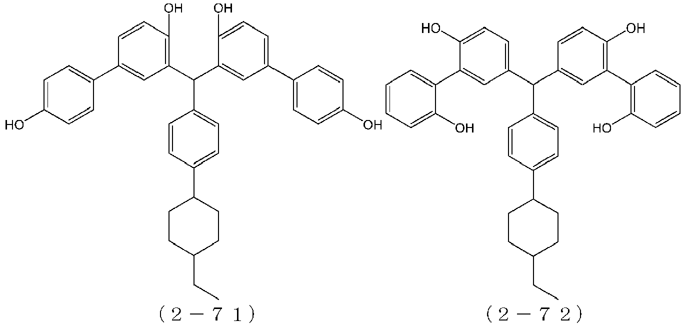

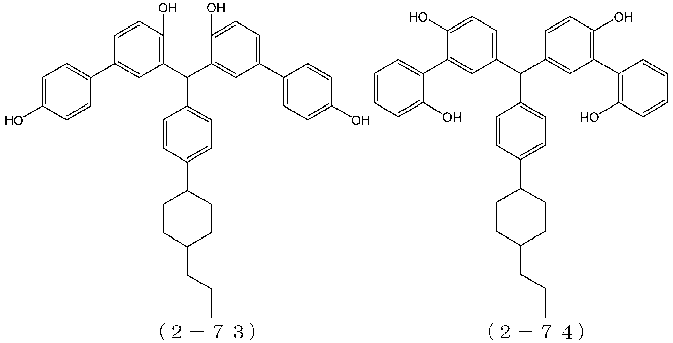

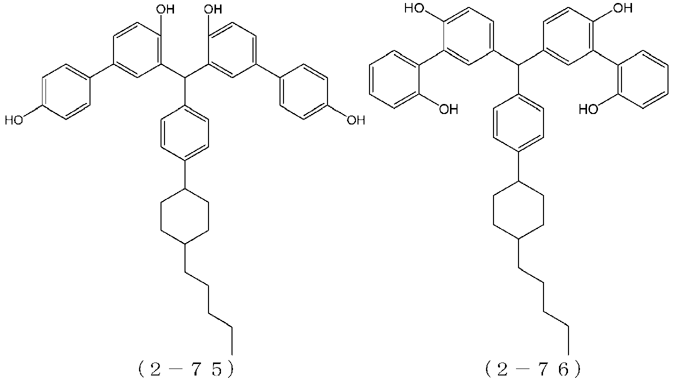

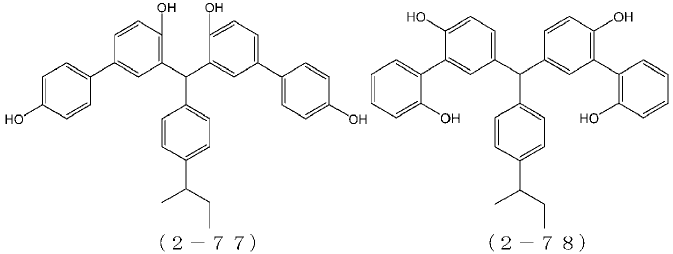

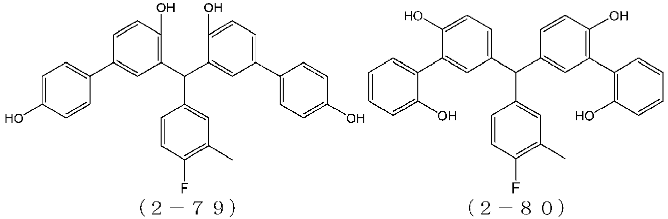

### [Method for producing compound represented by formula (A)]

The compound represented by the formula (A) of the present embodiment can be arbitrarily synthesized by the application of a publicly known approach, and the synthesis approach is not particularly limited. The compound represented by the general formula (A) can be obtained, for example, by subjecting a biphenol and an aldehyde or a ketone corresponding to the structure of the desired compound to polycondensation reaction in the presence of an acid catalyst at normal pressure. If necessary, this reaction can also be carried out under increased pressure. Furthermore, the target compound can also be obtained by introducing a publicly known protective group to a phenolic hydroxy group, and reacting the phenolic hydroxy group, followed by deprotection. An acetal or ketal form of a carbonyl compound can also be used as a raw material.

Examples of the biphenol include, but not particularly limited to, biphenol, thiodiphenol, oxydiphenol, methylbiphenol, methoxybinaphthol, binaphthol, thiodinaphthol, oxydinaphthol, methylbinaphthol, methoxybinaphthol, bianthracenol, thiodianthracenol, oxydianthracenol, methylbianthracenol, and methoxybianthracenol. The position of a hydroxy group is arbitrary. These biphenols can be used alone as one kind or can be used in combination of two or more kinds. Among them, 4,4'-biphenol is more preferably used from the viewpoint of heat resistance. Also, 2,2'-biphenol is more preferably used from the viewpoint of easy production.

Examples of the aldehyde include, but not particularly limited to, methylbenzaldehyde, dimethylbenzaldehyde, trimethylbenzaldehyde, ethylbenzaldehyde, propylbenzaldehyde, butylbenzaldehyde, pentabenzaldehyde, butylmethylbenzaldehyde, hydroxybenzaldehyde, dihydroxybenzaldehyde, fluoromethylbenzaldehyde, cyclopropylaldehyde, cyclobutylaldehyde, cyclohexylaldehyde, cyclodecylaldehyde, cycloundecylaldehyde, cyclopropylbenzaldehyde, cyclobutylbenzaldehyde, cyclohexylbenzaldehyde, cyclodecylbenzaldehyde, and cycloundecylbenzaldehyde. These aldehydes can be used alone as one kind or can be used in combination of two or more kinds. Among them, methylbenzaldehyde, dimethylbenzaldehyde, trimethylbenzaldehyde, ethylbenzaldehyde, propylbenzaldehyde, butylbenzaldehyde, pentabenzaldehyde, butylmethylbenzaldehyde, cyclohexylbenzaldehyde, cyclodecylbenzaldehyde, cycloundecylbenzaldehyde, or the like is preferably used from the viewpoint of imparting high heat resistance.

Examples of the ketone include, but not particularly limited to, acetylmethylbenzene, acetyldimethylbenzene, acetyltrimethylbenzene, acetylethylbenzene, acetylpropylbenzene, acetylbutylbenzene, acetylpentabenzene, acetylbutylmethylbenzene, acetylhydroxybenzene, acetyldihydroxybenzene, and acetylfluoromethylbenzene. These ketones can be used alone as one kind or can be used in combination of two or more kinds. Among them, acetylmethylbenzene, acetyldimethylbenzene, acetyltrimethylbenzene, acetylethylbenzene, acetylpropylbenzene, acetylbutylbenzene, acetylpentabenzene, or acetylbutylmethylbenzene is preferably used from the viewpoint of imparting high heat resistance.

The acid catalyst used in the reaction can be arbitrarily selected and used from publicly known catalysts and is not particularly limited. Inorganic acids, organic acids, Lewis acids, solid acids, and the like are widely known as such acid catalysts. Specific examples of the above acid catalyst include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferably used from the viewpoint of production such as easy availability and handleability. The acid catalysts can be used alone as one kind or can be used in combination of two or more kinds. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

Upon the reaction, a reaction solvent may be used. The reaction solvent is not particularly limited as long as the reaction of the aldehyde or the ketone used with the phenol proceeds, and can be arbitrarily selected and used from publicly known solvents. Examples include water, methanol, ethanol, propanol, butanol, tetrahydrofuran, dioxane, ethylene glycol dimethyl ether, ethylene glycol diethyl ether, and a mixed solvent thereof. The solvents can be used alone as one kind or can be used in combination of two or more kinds. Also, the amount of these solvents used can be arbitrarily set according to, for example, the kind of the raw materials used and the acid catalyst used and moreover the reaction conditions. The amount of the above solvent used is not particularly limited, but is preferably in the range of 0 to 2000 parts by mass based on 100 parts by mass of the reaction raw materials. Furthermore, the reaction temperature in the above reaction can be arbitrarily selected according to the reactivity of the reaction raw materials. The above reaction temperature is not particularly limited, but is usually preferably within the range of 10 to 200°C. In order to form the xanthene structure or the thioxanthene structure of the compound having the structure represented by the general formula (A) of the present embodiment, a higher reaction temperature is more preferable. Specifically, the range of 60 to 200°C is preferable. The reaction method can be arbitrarily selected and used from publicly known approaches and is not particularly limited, and there are a method of charging the phenol, the aldehyde or the ketone, and the acid catalyst in one portion, and a method of dropping the phenol and the aldehyde or the ketone in the presence of the acid catalyst. After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, the compound which is the target compound can be obtained.

As preferable reaction conditions, the reaction proceeds by using 1 mol to an excess of the phenol and 0.001 to 1 mol of the acid catalyst based on 1 mol of the aldehyde or the ketone, and reacting them at 50 to 200°C at normal pressure for about 20 minutes to 100 hours.

The target compound can be isolated by a publicly known method after the reaction terminates. The compound having the structure represented by the above general formula (A) which is the target compound can be obtained, for example, by concentrating the reaction solution, precipitating the reaction product by the addition of pure water, cooling the reaction solution to room temperature, then separating the precipitates by filtration, drying the solid matter obtained by filtration, then separating and purifying the solid matter from by-products by column chromatography, and distilling off the solvent, followed by filtration and drying.

The molecular weight of the compound having the structure represented by the above general formula (A) is not particularly limited, and the weight average molecular weight (Mw) in terms of polystyrene is preferably 350 to 30,000 and more preferably 500 to 20,000. The compound having the structure represented by the above general formula (A) preferably has dispersibility (weight average molecular weight Mw/number average molecular weight Mn) within the range of 1.1 to 7 from the viewpoint of enhancing crosslinking efficiency while suppressing volatile components during baking. The above Mw and Mn can be measured by a method described in Examples mentioned later.

The compound having the structure represented by the above general formula (A) preferably has high solubility in a solvent from the viewpoint of easier application to a wet process, etc. More specifically, in the case of using 1-methoxy-2-propanol (PGME) and/or propylene glycol monomethyl ether acetate (PGMEA) as a solvent, the compound and/or a resin preferably has a solubility of 10% by mass or more in the solvent.
Herein, the solubility in PGME and/or PGMEA is defined as "mass of the resin / (mass of the resin + mass of the solvent) × 100 (% by mass)". For example, 10 g of the compound represented by the above general formula (A) is evaluated as being dissolved in 90 g of PGMEA when the solubility of the compound represented by the general formula (A) in PGMEA is "10% by mass or more"; 10 g of the compound is evaluated as being not dissolved in 90 g of PGMEA when the solubility is "less than 10% by mass".

When the underlayer film forming material for lithography of the present embodiment comprises an organic solvent which is an optional component mentioned later, the content of the compound having the structure represented by the above general formula (A) is not particularly limited, but is preferably 1 to 33 parts by mass based on 100 parts by mass in total including the organic solvent, more preferably 2 to 25 parts by mass, and further preferably 3 to 20 parts by mass.

### [Resin having constituent unit derived from compound represented by formula (A)]

The resin of the present embodiment is a resin having a constituent unit derived from the compound represented by the above formula (A) (hereinafter, also referred to as "compound of the present embodiment"). The compound represented by the above formula (A) can be used directly as a film forming composition for lithography or the like. The resin having a constituent unit derived from the compound represented by the above formula (A) can also be used as a film forming composition for lithography or the like. The resin having a structural unit derived from the compound represented by the formula (A) includes a resin having a constituent unit derived from the compound represented by the formula (1), a resin having a constituent unit derived from the compound represented by the formula (1-1), and a resin having a constituent unit derived from the compound represented by the formula (1-2). Hereinafter, the "compound represented by the formula (A)" can be used interchangeably with the "compound represented by the formula (1)", the "compound represented by the formula (1-1)", or the "compound represented by the formula (1-2)".

### [Method for producing resin having constituent unit derived from compound represented by formula (A)]

The resin of the present embodiment is obtained by, for example, reacting the compound represented by the above formula (A) with a crosslinking compound.

As the crosslinking compound, a publicly known monomer can be used without particular limitations as long as it can oligomerize or polymerize the compound represented by the above formula (A). Specific examples thereof include, but not particularly limited to, aldehydes, ketones, carboxylic acids, carboxylic acid halides, halogen-containing compounds, amino compounds, imino compounds, isocyanates, and unsaturated hydrocarbon group-containing compounds.

Specific examples of the resin according to the present embodiment include resins that are made novolac by, for example, a condensation reaction between the compound represented by the above formula (A) with an aldehyde that is a crosslinking compound.

Herein, examples of the aldehyde used when making the compound represented by the above formula (A) novolac include, but not particularly limited to, formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarboaldehyde, phenanthrenecarboaldehyde, pyrenecarboaldehyde, and furfural. Among these, formaldehyde is more preferable. These aldehydes can be used alone as one kind or may be used in combination of two or more kinds. The amount of the above aldehydes used is not particularly limited, but is preferably 0.2 to 5 mol and more preferably 0.5 to 2 mol based on 1 mol of the compound represented by the above formula (A).

An acid catalyst can also be used in the condensation reaction between the compound represented by the above formula (A) and the aldehyde. The acid catalyst used herein can be arbitrarily selected and used from publicly known catalysts and is not particularly limited. Inorganic acids, organic acids, Lewis acids, and solid acids are widely known as such acid catalysts, and examples include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferable from the viewpoint of production such as easy availability and handleability. The acid catalysts can be used alone as one kind, or can be used in combination of two or more kinds.

Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

The aldehyde is not necessarily needed in the case of a copolymerization reaction with a compound having a non-conjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborn-2-ene, α-pinene, β-pinene, and limonene, as the crosslinking compound.

A reaction solvent can also be used in the condensation reaction between the compound represented by the above formula (A) and the aldehyde. The reaction solvent in the polycondensation can be arbitrarily selected and used from publicly known solvents and is not particularly limited, and examples include water, methanol, ethanol, propanol, butanol, tetrahydrofuran, dioxane, or a mixed solvent thereof. The reaction solvents can be used alone as one kind, or can be used in combination of two or more kinds.

Also, the amount of these reaction solvents used can be arbitrarily set according to, for example, the kind of the raw materials used and the catalyst used and moreover the reaction conditions and is not particularly limited, but is preferably in the range of 0 to 2000 parts by mass based on 100 parts by mass of the reaction raw materials. Furthermore, the reaction temperature can be arbitrarily selected according to the reactivity of the reaction raw materials and is not particularly limited, but is usually within the range of 10 to 200°C. The reaction method can be arbitrarily selected and used from publicly known approaches and is not particularly limited, and there are a method of charging the compound represented by the above formula (A), the aldehyde, and the catalyst in one portion, and a method of dropping the compound represented by the above formula (A) and the aldehyde in the presence of the catalyst.

After the polycondensation reaction terminates, isolation of the obtained resin can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, a novolac resin that is the target compound can be obtained.

Herein, the resin according to the present embodiment may be a homopolymer of a compound represented by the above formula (A), or may be a copolymer with a further phenol. Herein, examples of the copolymerizable phenol include, but not particularly limited to, phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol, and thymol.

The resin according to the present embodiment may be a copolymer with a polymerizable monomer other than the above-described further phenols. Examples of such a copolymerization monomer include, but not particularly limited to, naphthol, methylnaphthol, methoxynaphthol, dihydroxynaphthalene, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornene, pinene, and limonene. The resin according to the present embodiment may be a copolymer of two or more components (for example, a binary to quaternary system) composed of the compound represented by the above formula (A) and the above-described phenol, may be a copolymer of two or more components (for example, a binary to quaternary system) composed of the compound represented by the above formula (A) and the above-described copolymerization monomer, or may be a copolymer of three or more components (for example, a tertiary to quaternary system) composed of the compound represented by the above formula (A), the above-described phenol, and the above-described copolymerization monomer.

The molecular weight of the resin according to the present embodiment is not particularly limited, and the weight average molecular weight (Mw) in terms of polystyrene is preferably 500 to 30,000 and more preferably 750 to 20,000. The resin according to the present embodiment preferably has dispersibility (weight average molecular weight Mw/number average molecular weight Mn) within the range of 1.2 to 7 from the viewpoint of enhancing crosslinking efficiency while suppressing volatile components during baking. The above Mw and Mn can be measured by a method described in Examples mentioned later.

### [Composition]

The composition of the present embodiment comprises one or more substances selected from the group consisting of the compound represented by the above formula (A) and the resin having a constituent unit derived from the compound. Also, the composition of the present embodiment may comprise both of the compound of the present embodiment and the resin of the present embodiment. Hereinafter, the "one or more selected from the group consisting of the compound represented by the above formula (A) and the resin having a constituent unit derived from the compound" is also referred to as "compound and/or resin of the present embodiment" or "component (A)".

### [Composition for forming a optical component]

The composition for forming a optical component of the present embodiment comprises one or more substances selected from the group consisting of the compound represented by the above formula (A) and the resin having a constituent unit derived from the compound. Also, the composition for forming a optical component of the present embodiment may comprise both of the compound of the present embodiment and the resin of the present embodiment. Herein, the "optical component" refers to a component in the form of a film or a sheet as well as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, or a photosensitive optical waveguide. The compound and the resin of the present embodiment are useful for forming these optical components.

### [Film forming composition for lithography]

The film forming composition for lithography of the present embodiment comprises one or more substances selected from the group consisting of the compound represented by the above formula (A) and the resin having a constituent unit derived from the compound. Also, the film forming composition for lithography of the present embodiment may comprise both of the compound of the present embodiment and the resin of the present embodiment.

### [Resist composition]

The resist composition of the present embodiment comprises one or more substances selected from the group consisting of the compound represented by the above formula (A) and the resin having a constituent unit derived from the compound. Also, the resist composition of the present embodiment may comprise both of the compound of the present embodiment and the resin of the present embodiment.

It is preferable that the resist composition of the present embodiment should comprise a solvent. Examples of the solvent can include, but not particularly limited to, ethylene glycol monoalkyl ether acetates such as ethylene glycol monomethyl ether acetate, ethylene glycol monoethyl ether acetate, ethylene glycol mono-n-propyl ether acetate, and ethylene glycol mono-n-butyl ether acetate; ethylene glycol monoalkyl ethers such as ethylene glycol monomethyl ether and ethylene glycol monoethyl ether; propylene glycol monoalkyl ether acetates such as propylene glycol monomethyl ether acetate (PGMEA), propylene glycol monoethyl ether acetate, propylene glycol mono-n-propyl ether acetate, and propylene glycol mono-n-butyl ether acetate; propylene glycol monoalkyl ethers such as propylene glycol monomethyl ether (PGME) and propylene glycol monoethyl ether; ester lactates such as methyl lactate, ethyl lactate, n-propyl lactate, n-butyl lactate, and n-amyl lactate; aliphatic carboxylic acid esters such as methyl acetate, ethyl acetate, n-propyl acetate, n-butyl acetate, n-amyl acetate, n-hexyl acetate, methyl propionate, and ethyl propionate; other esters such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutylacetate, 3-methyl-3-methoxybutylacetate, butyl 3-methoxy-3-methylpropionate, butyl 3-methoxy-3-methylbutyrate, methyl acetoacetate, methyl pyruvate, and ethyl pyruvate; aromatic hydrocarbons such as toluene and xylene; ketones such as 2-heptanone, 3-heptanone, 4-heptanone, cyclopentanone (CPN), and cyclohexanone (CHN); amides such as N,N-dimethylformamide, N-methylacetamide, N,N-dimethylacetamide, and N-methylpyrrolidone; and lactones such as γ-lactone. These solvents can be used alone or in combination of two or more kinds.

The solvent used in the present embodiment is preferably a safe solvent, more preferably at least one selected from PGMEA, PGME, CHN, CPN, 2-heptanone, anisole, butyl acetate, ethyl propionate, and ethyl lactate, and still more preferably at least one selected from PGMEA, PGME, and CHN.

In the present embodiment, the amount of the solid component and the amount of the solvent are not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the amount of the solid component and the solvent.

### [Other components]

The resist composition of the present embodiment may contain other components such as a crosslinking agent, an acid generating agent, and an acid diffusion controlling agent, in addition to the compound and/or the resin of the present embodiment, if required. Hereinafter, these optional components will be described.

### [Acid generating agent (C)]

The resist composition of the present embodiment preferably comprises one or more acid generating agents (C) generating an acid directly or indirectly by irradiation of any radiation selected from visible light, ultraviolet, excimer laser, electron beam, extreme ultraviolet (EUV), X-ray, and ion beam. The acid generating agent (C) is not particularly limited, and, for example, an acid generating agent described in International Publication No. WO2013/024778 can be used. The acid generating agent (C) can be used alone or in combination of two or more kinds.

The amount of the acid generating agent (C) used is preferably 0.001 to 49% by mass of the total weight of the solid components, more preferably 1 to 40% by mass, still more preferably 3 to 30% by mass, and particularly preferably 10 to 25% by mass. By using the acid generating agent (C) within the above range, a pattern profile with high sensitivity and low edge roughness is obtained. In the present embodiment, the acid generation method is not limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

### [Acid crosslinking agent (G)]

The resist composition of the present embodiment preferably comprises one or more acid crosslinking agents (G). The acid crosslinking agent (G) is a compound capable of intramolecular or intermolecular crosslinking the component (A) in the presence of the acid generated from the acid generating agent (C). Examples of such an acid crosslinking agent (G) can include a compound having one or more groups (hereinafter, referred to as "crosslinkable group") capable of crosslinking the component (A).

Examples of such a crosslinkable group can include (i) a hydroxyalkyl group such as a hydroxy (C1-C6 alkyl group), a C1-C6 alkoxy (C1-C6 alkyl group), and an acetoxy (C1-C6 alkyl group), or a group derived therefrom; (ii) a carbonyl group such as a formyl group and a carboxy (C1-C6 alkyl group), or a group derived therefrom; (iii) a nitrogenous group-containing group such as a dimethylaminomethyl group, a diethylaminomethyl group, a dimethylolaminomethyl group, a diethylolaminomethyl group, and a morpholinomethyl group; (iv) a glycidyl group-containing group such as a glycidyl ether group, a glycidyl ester group, and a glycidylamino group; (v) a group derived from an aromatic group such as a C1-C6 allyloxy (C1-C6 alkyl group) and a C1-C6 aralkyloxy (C1-C6 alkyl group) such as a benzyloxymethyl group and a benzoyloxymethyl group; and (vi) a polymerizable multiple bond-containing group such as a vinyl group and an isopropenyl group. As the crosslinkable group of the acid crosslinking agent (G) according to the present embodiment, a hydroxyalkyl group and an alkoxyalkyl group or the like are preferable, and an alkoxymethyl group is particularly preferable.

The acid crosslinking agent (G) having the above crosslinkable group is not particularly limited, and, for example, an acid crosslinking agent described in International Publication No. WO2013/024778 can be used. The acid crosslinking agent (G) can be used alone or in combination of two or more kinds.

In the present embodiment, the amount of the acid crosslinking agent (G) used is preferably 0.5 to 49% by mass of the total weight of the solid components, more preferably 0.5 to 40% by mass, still more preferably 1 to 30% by mass, and particularly preferably 2 to 20% by mass. When the content ratio of the above acid crosslinking agent (G) is 0.5% by mass or more, the inhibiting effect of the solubility of a resist film in an alkaline developing solution is improved, and a decrease in the film remaining rate, and occurrence of swelling and meandering of a pattern can be inhibited. On the other hand, when the content is 50% by mass or less, a decrease in heat resistance as a resist can be inhibited.

### [Acid diffusion controlling agent (E)]

In the present embodiment, the resist composition may comprise an acid diffusion controlling agent (E) having a function of controlling diffusion of an acid generated from an acid generating agent by radiation irradiation in a resist film to inhibit any unpreferable chemical reaction in an unexposed region or the like. By using such an acid diffusion controlling agent (E), the storage stability of a resist composition is improved. Also, along with the improvement of the resolution, the line width change of a resist pattern due to variation in the post exposure delay time before radiation irradiation and the post exposure delay time after radiation irradiation can be inhibited, and the composition has extremely excellent process stability. Such an acid diffusion controlling agent (E) is not particularly limited, and examples include a radiation degradable basic compound such as a nitrogen atom-containing basic compound, a basic sulfonium compound, and a basic iodonium compound.

The above acid diffusion controlling agent (E) is not particularly limited, and, for example, an acid diffusion controlling agent described in International Publication No. WO2013/024778 can be used. The acid diffusion controlling agent (E) can be used alone or in combination of two or more kinds.

The content of the acid diffusion controlling agent (E) is preferably 0.001 to 49% by mass of the total weight of the solid component, more preferably 0.01 to 10% by mass, still more preferably 0.01 to 5% by mass, and particularly preferably 0.01 to 3% by mass. Within the above range, a decrease in resolution, and deterioration of the pattern shape and the dimension fidelity or the like can be prevented. Moreover, even though the post exposure delay time from electron beam irradiation to heating after radiation irradiation becomes longer, the shape of the pattern upper layer portion does not deteriorate. When the content is 10% by mass or less, a decrease in sensitivity, and developability of the unexposed portion or the like can be prevented. By using such an acid diffusion controlling agent, the storage stability of a resist composition improves, also along with improvement of the resolution, the line width change of a resist pattern due to variation in the post exposure delay time before radiation irradiation and the post exposure delay time after radiation irradiation can be inhibited, and the composition is extremely excellent process stability.

### [Further component (F)]

To the resist composition of the present embodiment, if required, as the further component (F), one kind or two kinds or more of various additive agents such as a dissolution promoting agent, a dissolution controlling agent, a sensitizing agent, a surfactant, and an organic carboxylic acid or an oxo acid of phosphor or derivative thereof can be added.

### [Dissolution promoting agent]

A low molecular weight dissolution promoting agent is a component having a function of increasing the solubility of a compound represented by the formula (A) in a developing solution to moderately increase the dissolution rate of the compound upon developing, when the solubility of the compound is too low. The low molecular weight dissolution promoting agent can be used, if required. Examples of the above dissolution promoting agent can include low molecular weight phenolic compounds, such as bisphenols and tris(hydroxyphenyl)methane. These dissolution promoting agents can be used alone or in mixture of two or more kinds.

The content of the dissolution promoting agent, which is arbitrarily adjusted according to the kind of the compound to be used, is preferably 0 to 49% by mass of the total weight of the solid component, more preferably 0 to 5% by mass, still more preferably 0 to 1% by mass, and particularly preferably 0% by mass.

### [Dissolution controlling agent]

The dissolution controlling agent is a component having a function of controlling the solubility of the compound represented by the formula (A) in a developing solution to moderately decrease the dissolution rate upon developing, when the solubility of the compound is too high. As such a dissolution controlling agent, the one which does not chemically change in steps such as calcination of resist coating, radiation irradiation, and development is preferable.

The dissolution controlling agent is not particularly limited, and examples can include aromatic hydrocarbons such as phenanthrene, anthracene, and acenaphthene; ketones such as acetophenone, benzophenone, and phenyl naphthyl ketone; and sulfones such as methyl phenyl sulfone, diphenyl sulfone, and dinaphthyl sulfone. These dissolution controlling agents can be used alone or in combination of two or more kinds.

The content of the dissolution controlling agent, which is arbitrarily adjusted according to the kind of the compound to be used, is preferably 0 to 49% by mass of the total weight of the solid component, more preferably 0 to 5% by mass, still more preferably 0 to 1% by mass, and particularly preferably 0% by mass.

### [Sensitizing agent]

The sensitizing agent is a component having a function of absorbing irradiated radiation energy, transmitting the energy to the acid generating agent (C), and thereby increasing the acid production amount, and improving the apparent sensitivity of a resist. Such a sensitizing agent is not particularly limited, and examples can include benzophenones, biacetyls, pyrenes, phenothiazines, and fluorenes. These sensitizing agents can be used alone or in combination of two or more kinds.

The content of the sensitizing agent, which is arbitrarily adjusted according to the kind of the compound to be used, is preferably 0 to 49% by mass of the total weight of the solid component, more preferably 0 to 5% by mass, still more preferably 0 to 1% by mass, and particularly preferably 0% by mass.

### [Surfactant]

The surfactant is a component having a function of improving coatability and striation of the resist composition of the present embodiment, and developability of a resist or the like. Such a surfactant may be any of anionic, cationic, nonionic, and amphoteric surfactants. A preferable surfactant is a nonionic surfactant. The nonionic surfactant has a good affinity with a solvent used in production of resist compositions and more effects. Examples of the nonionic surfactant include, but not particularly limited to, a polyoxyethylene higher alkyl ethers, polyoxyethylene higher alkyl phenyl ethers, and higher fatty acid diesters of polyethylene glycol. Examples of commercially available products include, hereinafter by trade name, EFTOP (manufactured by Jemco Inc.), MEGAFAC (manufactured by DIC Corporation), Fluorad (manufactured by Sumitomo 3M Limited), AsahiGuard, Surflon (hereinbefore, manufactured by Asahi Glass Co., Ltd.), Pepole (manufactured by Toho Chemical Industry Co., Ltd.), KP (manufactured by Shin-Etsu Chemical Co., Ltd.), and Polyflow (manufactured by Kyoeisha Chemical Co., Ltd.).

The content of the surfactant, which is arbitrarily adjusted according to the kind of the compound to be used, is preferably 0 to 49% by mass of the total weight of the solid component, more preferably 0 to 5% by mass, still more preferably 0 to 1% by mass, and particularly preferably 0% by mass.

### [Organic carboxylic acid or oxo acid of phosphor or derivative thereof]

For the purpose of prevention of sensitivity deterioration or improvement of a resist pattern shape and post exposure delay stability or the like, and as an additional optional component, the resist composition of the present embodiment can contain an organic carboxylic acid or an oxo acid of phosphor or derivative thereof. The organic carboxylic acid or the oxo acid of phosphor or derivative thereof can be used in combination with the acid diffusion controlling agent, or may be used alone. The organic carboxylic acid is, for example, suitably malonic acid, citric acid, malic acid, succinic acid, benzoic acid, salicylic acid, or the like. Examples of the oxo acid of phosphor or derivative thereof include phosphoric acid or derivative thereof such as ester including phosphoric acid, di-n-butyl ester phosphate, and diphenyl ester phosphate; phosphonic acid or derivative thereof such as ester including phosphonic acid, dimethyl ester phosphonate, di-n-butyl ester phosphonate, phenylphosphonic acid, diphenyl ester phosphonate, and dibenzyl ester phosphonate; and phosphinic acid and derivative thereof such as ester including phosphinic acid and phenylphosphinic acid. Among these, phosphonic acid is particularly preferable.

The organic carboxylic acid or the oxo acid of phosphor or derivative thereof can be used alone or in combination of two or more kinds. The content of the organic carboxylic acid or the oxo acid of phosphor or derivative thereof, which is arbitrarily adjusted according to the kind of the compound to be used, is preferably 0 to 49% by mass of the total weight of the solid component, more preferably 0 to 5% by mass, still more preferably 0 to 1% by mass, and particularly preferably 0% by mass.

### [Further additive agent other than above additive agents (dissolution promoting agent, dissolution controlling agent, sensitizing agent, surfactant, and organic carboxylic acid or oxo acid of phosphor or derivative thereof)]

Furthermore, the resist composition of the present embodiment can contain one kind or two kinds or more of additive agents other than the above dissolution promoting agent, dissolution controlling agent, sensitizing agent, surfactant, and organic carboxylic acid or oxo acid of phosphor or derivative thereof if required. Examples of such an additive agent include a dye, a pigment, and an adhesion aid. For example, the composition contains the dye or the pigment, and thereby a latent image of the exposed portion is visualized and influence of halation upon exposure can be alleviated, which is preferable. The composition contains the adhesion aid, and thereby adhesiveness to a supporting material can be improved, which is preferable. Furthermore, examples of other additive agent can include a halation preventing agent, a storage stabilizing agent, a defoaming agent, and a shape improving agent. Specific examples thereof can include 4-hydroxy-4'-methylchalkone.

In the resist composition of the present embodiment, the total content of the optional component (F) is preferably 0 to 99% by mass of the total weight of the solid component, more preferably 0 to 49% by mass, still more preferably 0 to 10% by mass, further preferably 0 to 5% by mass, still further preferably 0 to 1% by mass, and particularly preferably 0% by mass.

### [Content ratio of each component in resist composition]

In the resist composition of the present embodiment, the content of the compound and/or the resin of the present embodiment is not particularly limited, but is preferably 50 to 99.4% by mass of the total mass of the solid components (summation of solid components including the compound represented by the formula (A), the resin having the compound represented by the formula (A) as a constituent, and optionally used components such as acid generating agent (C), acid crosslinking agent (G), acid diffusion controlling agent (E), and further component (F) (also referred to as "optional component (F)"), hereinafter the same), more preferably 55 to 90% by mass, still more preferably 60 to 80% by mass, and particularly preferably 60 to 70% by mass. In the case of the above content, resolution is further improved, and line edge roughness (LER) is further decreased. When both the compound and the resin of the present embodiment are contained, the above content refers to the total amount of the compound and the resin of the present embodiment.

In the resist composition of the present embodiment, the content ratio of the compound and/or the resin of the present embodiment (component (A)), the acid generating agent (C), the acid crosslinking agent (G), the acid diffusion controlling agent (E), and the optional component (F) (the component (A)/the acid generating agent (C)/the acid crosslinking agent (G)/the acid diffusion controlling agent (E)/the optional component (F)) is preferably 50 to 99.4% by mass/0.001 to 49% by mass/0.5 to 49% by mass/0.001 to 49% by mass/0 to 49% by mass based on 100% by mass of the solid components of the resist composition, more preferably 55 to 90% by mass/1 to 40% by mass/0.5 to 40% by mass/0.01 to 10% by mass/0 to 5% by mass, still more preferably 60 to 80% by mass/3 to 30% by mass/1 to 30% by mass/0.01 to 5% by mass/0 to 1% by mass, and particularly preferably 60 to 70% by mass/10 to 25% by mass/2 to 20% by mass/0.01 to 3% by mass/0% by mass. The content ratio of each component is selected from each range so that the summation thereof is 100% by mass. By the above content ratio, performance such as sensitivity, resolution, and developability is excellent. The "solid components" refer to components except for the solvent. "100% by mass of the solid components" refer to 100% by mass of the components except for the solvent.

The resist composition of the present embodiment is generally prepared by dissolving each component in a solvent upon use into a homogeneous solution, and then if required, filtering through a filter or the like with a pore diameter of about 0.2 µm, for example.

The resist composition of the present embodiment can comprise an additional resin other than the resin of the present embodiment, if required. Examples of the resin include, but not particularly limited to, a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of the above resin is not particularly limited and is arbitrarily adjusted according to the kind of the component (A) to be used, and is preferably 30 parts by mass or less per 100 parts by mass of the component (A), more preferably 10 parts by mass or less, still more preferably 5 parts by mass or less, and particularly preferably 0 parts by mass.

### [Physical properties and the like of resist composition]

The resist composition of the present embodiment can form an amorphous film by spin coating. Also, the resist composition of the present embodiment can be applied to a general semiconductor production process. Any of positive type and negative type resist patterns can be individually prepared depending on the kind of a developing solution to be used.

In the case of a positive type resist pattern, the dissolution rate of the amorphous film formed by spin coating with the resist composition of the present embodiment in a developing solution at 23°C is preferably 5 angstrom/sec or less, more preferably 0.0005 to 5 angstrom/sec, and still more preferably 0.05 to 5 angstrom/sec. When the dissolution rate is 5 angstrom/sec or less, the above portion is insoluble in a developing solution, and thus the amorphous film can form a resist. When the amorphous film has a dissolution rate of 0.0005 angstrom/sec or more, the resolution may improve. It is presumed that this is because due to the change in the solubility before and after exposure of the component (A), contrast at the interface between the exposed portion being dissolved in a developing solution and the unexposed portion not being dissolved in a developing solution is increased. Also, there are effects of reducing LER and defects.

In the case of a negative type resist pattern, the dissolution rate of the amorphous film formed by spin coating with the resist composition of the present embodiment in a developing solution at 23°C is preferably 10 angstrom/sec or more. When the dissolution rate is 10 angstrom/sec or more, the amorphous film more easily dissolves in a developing solution, and is more suitable for a resist. When the amorphous film has a dissolution rate of 10 angstrom/sec or more, the resolution may improve. It is presumed that this is because the micro surface portion of the component (A) dissolves, and LER is reduced. Also, there are effects of reducing defects.

The dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 10 angstrom/sec or more. When the dissolution rate is 10 angstrom/sec or more, the amorphous film more easily dissolves in a developing solution, and is more suitable for a resist. When the amorphous film has a dissolution rate of 10 angstrom/sec or more, the resolution may improve. It is presumed that this is because the micro surface portion of the component (A) dissolves, and LER is reduced. Also, there are effects of reducing defects.

In the case of a negative type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 5 angstrom/sec or less, more preferably 0.0005 to 5 angstrom/sec, and still more preferably 0.05 to 5 angstrom/sec. When the dissolution rate is 5 angstrom/sec or less, the above portion is insoluble in a developing solution, and thus the amorphous film can form a resist. When the amorphous film has a dissolution rate of 0.0005 angstrom/sec or more, the resolution may improve. It is presumed that this is because due to the change in the solubility before and after exposure of the component (A), contrast at the interface between the unexposed portion being dissolved in a developing solution and the exposed portion not being dissolved in a developing solution is increased. Also, there are effects of reducing LER and defects.

### [Radiation-sensitive composition]

The radiation-sensitive composition of the present embodiment is preferably a radiation-sensitive composition comprising the compound of the present embodiment and/or the resin of the present embodiment (A), an optically active diazonaphthoquinone compound (B), and a solvent, wherein the content of the solvent is 20 to 99% by mass based on 100% by mass in total of the radiation-sensitive composition; and the content of components except for the solvent is 1 to 80% by mass based on 100% by mass in total of the radiation-sensitive composition.

The component (A) to be contained in the radiation-sensitive composition of the present embodiment is used in combination with the optically active diazonaphthoquinone compound (B) mentioned later and is useful as a base material for positive type resists that becomes a compound easily soluble in a developing solution by irradiation with g-ray, h-ray, i-ray, KrF excimer laser, ArF excimer laser, extreme ultraviolet, electron beam, or X-ray. Although the properties of the component (A) are not largely altered by g-ray, h-ray, i-ray, KrF excimer laser, ArF excimer laser, extreme ultraviolet, electron beam, or X-ray, the optically active diazonaphthoquinone compound (B) poorly soluble in a developing solution is converted to an easily soluble compound so that a resist pattern can be formed in a development step.

Since the component (A) to be contained in the radiation-sensitive composition of the present embodiment is a relatively low molecular weight compound as shown in the above formula (A), the obtained resist pattern has very small roughness.

The glass transition temperature of the component (A) to be contained in the radiation-sensitive composition of the present embodiment is preferably 100°C or higher, more preferably 120°C or higher, still more preferably 140°C or higher, and particularly preferably 150°C or higher. The upper limit of the glass transition temperature of the component (A) is not particularly limited and is, for example, 400°C. When the glass transition temperature of the component (A) falls within the above range, the resulting radiation-sensitive composition has heat resistance capable of maintaining a pattern shape in a semiconductor lithography process, and improves performance such as high resolution.

The heat of crystallization determined by the differential scanning calorimetry of the glass transition temperature of the component (A) to be contained in the radiation-sensitive composition of the present embodiment is preferably less than 20 J/g. (Crystallization temperature) - (Glass transition temperature) is preferably 70°C or more, more preferably 80°C or more, still more preferably 100°C or more, and particularly preferably 130°C or more. When the heat of crystallization is less than 20 J/g or (Crystallization temperature) - (Glass transition temperature) falls within the above range, the radiation-sensitive composition easily forms an amorphous film by spin coating, can maintain film formability necessary for a resist over a long period, and can improve resolution.

In the present embodiment, the above heat of crystallization, crystallization temperature, and glass transition temperature can be determined by differential scanning calorimetry using "DSC/TA-50WS" manufactured by Shimadzu Corp. For example, about 10 mg of a sample is placed in an unsealed container made of aluminum, and the temperature is raised to the melting point or more at a temperature increase rate of 20°C/min in a nitrogen gas stream (50 mL/min). After quenching, again the temperature is raised to the melting point or more at a temperature increase rate of 20°C/min in a nitrogen gas stream (30 mL/min). After further quenching, again the temperature is raised to 400°C at a temperature increase rate of 20°C/min in a nitrogen gas stream (30 mL/min). The temperature at the middle point (where the specific heat is changed into the half) of steps in the baseline shifted in a step-like pattern is defined as the glass transition temperature (Tg). The temperature of the subsequently appearing exothermic peak is defined as the crystallization temperature. The heat is determined from the area of a region surrounded by the exothermic peak and the baseline and defined as the heat of crystallization.

The component (A) to be contained in the radiation-sensitive composition of the present embodiment is preferably low sublimable at 100°C or lower, preferably 120°C or lower, more preferably 130°C or lower, still more preferably 140°C or lower, and particularly preferably 150°C or lower at normal pressure. The low sublimability means that in thermogravimetry, weight reduction when the resist base material is kept at a predetermined temperature for 10 minutes is 10% or less, preferably 5% or less, more preferably 3% or less, still more preferably 1% or less, and particularly preferably 0.1% or less. The low sublimability can prevent an exposure apparatus from being contaminated by outgassing upon exposure. In addition, a good pattern shape with low roughness can be obtained.

The component (A) to be contained in the radiation-sensitive composition of the present embodiment dissolves at preferably 1% by mass or more, more preferably 5% by mass or more, and still more preferably 10% by mass or more at 23°C in a solvent that is selected from propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate and exhibits the highest ability to dissolve the component (A). Particularly preferably, the component (A) dissolves at 20% by mass or more at 23°C in a solvent that is selected from PGMEA, PGME, and CHN and exhibits the highest ability to dissolve the resist base material (A). Particularly preferably, the component (A) dissolves at 20% by mass or more at 23°C in PGMEA. When the above conditions are met, the radiation-sensitive composition is easily used in a semiconductor production process at a full production scale.

### [Optically active diazonaphthoquinone compound (B)]

The optically active diazonaphthoquinone compound (B) to be contained in the radiation-sensitive composition of the present embodiment is a diazonaphthoquinone substance including a polymer or non-polymer optically active diazonaphthoquinone compound and is not particularly limited as long as it is generally used as a photosensitive component (sensitizing agent) in positive type resist compositions. One kind or two or more kinds can be optionally selected and used.

Such a sensitizing agent is preferably a compound obtained by reacting naphthoquinonediazide sulfonic acid chloride, benzoquinonediazide sulfonic acid chloride, or the like with a low molecular weight compound or a high molecular weight compound having a functional group condensable with these acid chlorides. Herein, examples of the above functional group condensable with the acid chlorides include, but not particularly limited to, a hydroxyl group and an amino group. Particularly, a hydroxyl group is preferable. Examples of the compound containing a hydroxyl group condensable with the acid chlorides can include, but not particularly limited to, hydroquinone, resorcin, hydroxybenzophenones such as 2,4-dihydroxybenzophenone, 2,3,4-trihydroxybenzophenone, 2,4,6-trihydroxybenzophenone, 2,4,4'-trihydroxybenzophenone, 2,3,4,4'-tetrahydroxybenzophenone, 2,2',4,4'-tetrahydroxybenzophenone, and 2,2',3,4,6'-pentahydroxybenzophenone, hydroxyphenylalkanes such as bis(2,4-dihydroxyphenyl)methane, bis(2,3,4-trihydroxyphenyl)methane, and bis(2,4-dihydroxyphenyl)propane, and hydroxytriphenylmethanes such as 4,4',3",4"-tetrahydroxy-3,5,3',5'-tetramethyltriphenylmethane and 4,4',2",3",4"-pentahydroxy-3,5,3',5'-tetramethyltriphenylmethane.

Preferable examples of the acid chloride such as naphthoquinonediazide sulfonic acid chloride or benzoquinonediazide sulfonic acid chloride include 1,2-naphthoquinonediazide-5-sulfonyl chloride and 1,2-naphthoquinonediazide-4-sulfonyl chloride.

The radiation-sensitive composition of the present embodiment is preferably prepared by, for example, dissolving each component in a solvent upon use into a homogeneous solution, and then if required, filtering through a filter or the like with a pore diameter of about 0.2 µm, for example.

### [Solvent]

Examples of the solvent that can be used in the radiation-sensitive composition of the present embodiment include, but not particularly limited to, propylene glycol monomethyl ether acetate, propylene glycol monomethyl ether, cyclohexanone, cyclopentanone, 2-heptanone, anisole, butyl acetate, ethyl propionate, and ethyl lactate. Among them, propylene glycol monomethyl ether acetate, propylene glycol monomethyl ether, or cyclohexanone is preferable. The solvent may be used alone as one kind or may be used in combination of two or more kinds.

The content of the solvent is, for example, 20 to 99% by mass based on 100% by mass in total of the radiation-sensitive composition, preferably 50 to 99% by mass, more preferably 60 to 98% by mass, and particularly preferably 90 to 98% by mass.

The content of components except for the solvent (solid components) is, for example, 1 to 80% by mass based on 100% by mass in total of the radiation-sensitive composition, preferably 1 to 50% by mass, more preferably 2 to 40% by mass, particularly preferably 2 to 10% by mass.

### [Properties of radiation-sensitive composition]

The radiation-sensitive composition of the present embodiment can form an amorphous film by spin coating. Also, the radiation-sensitive composition of the present embodiment can be applied to a general semiconductor production process. Any of positive type and negative type resist patterns can be individually prepared depending on the kind of a developing solution to be used.

In the case of a positive type resist pattern, the dissolution rate of the amorphous film formed by spin coating with the radiation-sensitive composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, the above portion is insoluble in a developing solution, and thus the amorphous film can form a resist. When the amorphous film has a dissolution rate of 0.0005 angstrom/sec or more, the resolution may improve. It is presumed that this is because due to the change in the solubility before and after exposure of the component (A), contrast at the interface between the exposed portion being dissolved in a developing solution and the unexposed portion not being dissolved in a developing solution is increased. Also, there are effects of reducing LER and defects.

In the case of a negative type resist pattern, the dissolution rate of the amorphous film formed by spin coating with the radiation-sensitive composition of the present embodiment in a developing solution at 23°C is preferably 10 angstrom/sec or more. When the dissolution rate is 10 angstrom/sec or more, the amorphous film more easily dissolves in a developing solution, and is more suitable for a resist. When the amorphous film has a dissolution rate of 10 angstrom/sec or more, the resolution may improve. It is presumed that this is because the micro surface portion of the component (A) dissolves, and LER is reduced. Also, there are effects of reducing defects.

The dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 10 angstrom/sec or more, more preferably 10 to 10000 angstrom/sec, and still more preferably 100 to 1000 angstrom/sec. When the dissolution rate is 10 angstrom/sec or more, the amorphous film more easily dissolves in a developing solution, and is more suitable for a resist. When the amorphous film has a dissolution rate of 10000 angstrom/sec or less, the resolution may improve. It is presumed that this is because the micro surface portion of the component (A) dissolves, and LER is reduced. Also, there are effects of reducing defects.

In the case of a negative type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, the above portion is insoluble in a developing solution, and thus the amorphous film can form a resist. When the amorphous film has a dissolution rate of 0.0005 angstrom/sec or more, the resolution may improve. It is presumed that this is because due to the change in the solubility before and after exposure of the component (A), contrast at the interface between the unexposed portion being dissolved in a developing solution and the exposed portion not being dissolved in a developing solution is increased. Also, there are effects of reducing LER and defects.

### [Content ratio of each component in radiation-sensitive composition]

In the radiation-sensitive composition of the present embodiment, the content of the component (A) is preferably 1 to 99% by mass of the total weight of the solid components (summation of the component (A), the optically active diazonaphthoquinone compound (B), and optionally used solid components such as further component (D), hereinafter the same), more preferably 5 to 95% by mass, still more preferably 10 to 90% by mass, and particularly preferably 25 to 75% by mass. When the content of the component (A) falls within the above range, the radiation-sensitive composition of the present embodiment can produce a pattern with high sensitivity and low roughness.

In the radiation-sensitive composition of the present embodiment, the content of the optically active diazonaphthoquinone compound (B) is preferably 1 to 99% by mass of the total weight of the solid components (summation of the component (A), the optically active diazonaphthoquinone compound (B), and optionally used solid components such as further component (D), hereinafter the same), more preferably 5 to 95% by mass, still more preferably 10 to 90% by mass, and particularly preferably 25 to 75% by mass. When the content of the optically active diazonaphthoquinone compound (B) falls within the above range, the radiation-sensitive composition of the present embodiment can produce a pattern with high sensitivity and low roughness.

### [Further component (D)]

To the radiation-sensitive composition of the present embodiment, if required, as a component other than the component (A) and the optically active diazonaphthoquinone compound (B), one kind or two kinds or more of various additive agents such as the above acid generating agent, acid crosslinking agent, acid diffusion controlling agent, dissolution promoting agent, dissolution controlling agent, sensitizing agent, surfactant, and organic carboxylic acid or oxo acid of phosphor or derivative thereof can be added. In the present specification, the further component (D) is also referred to as an optional component (D).

The content ratio of the component (A), the optically active diazonaphthoquinone compound (B), and the further optional component (D) that may be optionally contained in the radiation-sensitive composition ((A)/(B)/(D)) is preferably 1 to 99% by mass/99 to 1% by mass/0 to 98% by mass based on 100% by mass of the solid components of the radiation-sensitive composition, more preferably 5 to 95% by mass/95 to 5% by mass/0 to 49% by mass, still more preferably 10 to 90% by mass/90 to 10% by mass/0 to 10% by mass, particularly preferably 20 to 80% by mass/80 to 20% by mass/0 to 5% by mass, and most preferably 25 to 75% by mass/75 to 25% by mass/0% by mass.

The content ratio of each component is selected from each range so that the summation thereof is 100% by mass. When the content ratio of each component falls within the above range, the radiation-sensitive composition of the present embodiment is excellent in performance such as sensitivity and resolution, in addition to roughness.

The radiation-sensitive composition of the present embodiment may comprise a further resin in addition to the compound and/or the resin of the present embodiment. Examples of such a resin include a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of these resins, which is arbitrarily adjusted according to the kind of the component (A) to be used, is preferably 30 parts by mass or less per 100 parts by mass of the component (A), more preferably 10 parts by mass or less, still more preferably 5 parts by mass or less, and particularly preferably 0 parts by mass.

### [Method for producing amorphous film]

The method for producing an amorphous film according to the present embodiment comprises the step of forming an amorphous film on a supporting material using the above radiation-sensitive composition.

### [Resist pattern formation method using radiation-sensitive composition]

A resist pattern formation method using the radiation-sensitive composition of the present embodiment includes the steps of: forming a resist film on a supporting material using the above radiation-sensitive composition; exposing at least a portion of the formed resist film; and developing the exposed resist film, thereby forming a resist pattern. Specifically, the same operation as in the following resist pattern formation method using the resist composition can be performed.

### [Resist pattern formation method using resist composition]

A resist pattern formation method using the resist composition of the present embodiment includes the steps of: forming a resist film on a supporting material using the above resist composition of the present embodiment; exposing at least a portion of the formed resist film; and developing the exposed resist film, thereby forming a resist pattern. The resist pattern according to the present embodiment can also be formed as an upper layer resist in a multilayer process.

Examples of the resist pattern formation method include, but not particularly limited to, the following methods. A resist film is formed by coating a conventionally publicly known supporting material with the above resist composition of the present embodiment using a coating means such as spin coating, flow casting coating, and roll coating. The conventionally publicly known supporting material is not particularly limited. For example, a supporting material for electronic components, and the one having a predetermined wiring pattern formed thereon, or the like can be exemplified. More specific examples include a supporting material made of a metal such as a silicon wafer, copper, chromium, iron and aluminum, and a glass supporting material. Examples of a wiring pattern material include copper, aluminum, nickel, and gold. Also if required, the supporting material may be a supporting material having an inorganic and/or organic film provided thereon. Examples of the inorganic film include an inorganic antireflection film (inorganic BARC). Examples of the organic film include an organic antireflection film (organic BARC). Surface treatment with hexamethylene disilazane or the like may be conducted.

Next, the coated supporting material is heated if required. The heating conditions vary according to the compounding composition of the resist composition, or the like, but are preferably 20 to 250°C, and more preferably 20 to 150°C. By heating, the adhesiveness of a resist to a supporting material may improve, which is preferable. Then, the resist film is exposed to a desired pattern by any radiation selected from the group consisting of visible light, ultraviolet, excimer laser, electron beam, extreme ultraviolet (EUV), X-ray, and ion beam. The exposure conditions or the like are arbitrarily selected according to the compounding composition of the resist composition, or the like. In the present embodiment, in order to stably form a fine pattern with a high degree of accuracy in exposure, the resist film is preferably heated after radiation irradiation.

Next, by developing the exposed resist film in a developing solution, a predetermined resist pattern is formed. As the developing solution, a solvent having a solubility parameter (SP value) close to that of the component (A) to be used is preferably selected. For example, a polar solvent such as a ketone-based solvent, an ester-based solvent, an alcohol-based solvent, an amide-based solvent, and an ether-based solvent described in International Publication No. WO2013/024778; and a hydrocarbon-based solvent, or an alkaline aqueous solution can be used.

A plurality of above solvents may be mixed, or the solvent may be used by mixing the solvent with a solvent other than those described above or water within the range having performance. In order to sufficiently exhibit the effect of the present invention, the water content ratio as the whole developing solution is preferably less than 70% by mass and less than 50% by mass, more preferably less than 30% by mass, and further preferably less than 10% by mass. Particularly preferably, the developing solution is substantially moisture free. That is, the content of the organic solvent in the developing solution is not particularly limited, and is preferably 30% by mass or more and 100% by mass or less based on the total amount of the developing solution, preferably 50% by mass or more and 100% by mass or less, more preferably 70% by mass or more and 100% by mass or less, still more preferably 90% by mass or more and 100% by mass or less, and particularly preferably 95% by mass or more and 100% by mass or less.

Particularly, the developing solution containing at least one kind of solvent selected from a ketone-based solvent, an ester-based solvent, an alcohol-based solvent, an amide-based solvent, and an ether-based solvent improves resist performance such as resolution and roughness of the resist pattern, which is preferable.

The vapor pressure of the developing solution is preferably 5 kPa or less at 20°C, more preferably 3 kPa or less, and particularly preferably 2 kPa or less. The evaporation of the developing solution on the supporting material or in a developing cup is inhibited by setting the vapor pressure of the developing solution to 5 kPa or less, to improve temperature uniformity within a wafer surface, thereby resulting in improvement in size uniformity within the wafer surface.

Examples of the developing solution having a vapor pressure of 5 kPa or less include those described in International Publication NO. WO2013/024778.

Examples of the developing solution having a vapor pressure of 2 kPa or less which is a particularly preferable range include those described in International Publication NO. WO2013/024778.

To the developing solution, a surfactant can be added in an appropriate amount, if required. The surfactant is not particularly limited but, for example, an ionic or nonionic fluorine-based and/or silicon-based surfactant can be used. Examples of the fluorine-based and/or silicon-based surfactant can include the surfactants described in Japanese Patent Application Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but a fluorine-based surfactant or a silicon-based surfactant is further preferably used.

The amount of the surfactant used is usually 0.001 to 5% by mass based on the total amount of the developing solution, preferably 0.005 to 2% by mass, and further preferably 0.01 to 0.5% by mass.

The development method is, for example, a method for dipping a supporting material in a bath filled with a developing solution for a fixed time (dipping method), a method for raising a developing solution on a supporting material surface by the effect of a surface tension and keeping it still for a fixed time, thereby conducting the development (puddle method), a method for spraying a developing solution on a supporting material surface (spraying method), and a method for continuously ejecting a developing solution on a supporting material rotating at a constant speed while scanning a developing solution ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the resist pattern cured by crosslinking. A solution containing a general organic solvent or water may be used as the rinsing solution. As the rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still more preferably, after development, a step of rinsing the film by using a rinsing solution containing an alcohol-based solvent or an ester-based solvent is conducted. Still more preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, as the monohydric alcohol used in the rinsing step after development, for example, a monohydric alcohol described in International Publication No. WO2013/024778 can be used. As a particularly preferable monohydric alcohol of 5 or more carbon atoms, 1-hexanol, 2-hexanol, 4-methyl-2-pentanol, 1-pentanol, 3-methyl-1-butanol or the like can be used.

A plurality of these components may be mixed, or the component may be used by mixing the component with an organic solvent other than those described above.

The water content ratio in the rinsing solution is preferably 10% by mass or less, more preferably 5% by mass or less, and particularly preferably 3% by mass or less. By setting the water content ratio to 10% by mass or less, better development characteristics can be obtained.

The vapor pressure at 20°C of the rinsing solution used after development is preferably 0.05 kPa or more and 5 kPa or less, more preferably 0.1 kPa or more and 5 kPa or less, and most preferably 0.12 kPa or more and 3 kPa or less. By setting the vapor pressure of the rinsing solution to 0.05 kPa or more and 5 kPa or less, the temperature uniformity in the wafer surface is enhanced and moreover, swelling due to permeation of the rinsing solution is further inhibited. As a result, the dimensional uniformity in the wafer surface is further improved.

The rinsing solution may also be used after adding an appropriate amount of a surfactant to the rinsing solution.

In the rinsing step, the wafer after development is rinsed using the organic solvent-containing rinsing solution. The method for rinsing treatment is not particularly limited. However, for example, a method for continuously ejecting a rinsing solution on a supporting material spinning at a constant speed (spin coating method), a method for dipping a supporting material in a bath filled with a rinsing solution for a fixed time (dipping method), and a method for spraying a rinsing solution on a supporting material surface (spraying method), or the like can be applied. Above all, it is preferable to conduct the rinsing treatment by the spin coating method and after the rinsing, spin the supporting material at a rotational speed of 2,000 rpm to 4,000 rpm, to remove the rinsing solution from the supporting material surface.

After forming the resist pattern, a pattern wiring supporting material is obtained by etching. Examples of the etching method include publicly known methods such as dry etching using plasma gas, and wet etching with an alkaline solution, a cupric chloride solution, and a ferric chloride solution or the like.

After forming the resist pattern, plating can also be conducted. Examples of the above plating method include copper plating, solder plating, nickel plating, and gold plating.

The remaining resist pattern after etching can be peeled by an organic solvent. Examples of the above organic solvent include PGMEA (propylene glycol monomethyl ether acetate), PGME (propylene glycol monomethyl ether), and EL (ethyl lactate). Examples of the above peeling method include a dipping method and a spraying method. A wiring supporting material having a resist pattern formed thereon may be a multilayer wiring supporting material, and may have a small diameter through hole.

In the present embodiment, the wiring supporting material can also be formed by a method for forming a resist pattern, then depositing a metal in vacuum, and subsequently dissolving the resist pattern in a solution, i.e., a liftoff method.

### [Underlayer film forming material for lithography]

The underlayer film forming material for lithography of the present embodiment comprises the compound of the present embodiment and/or the resin of the present embodiment. The content of the compound of the present embodiment and/or the resin of the present embodiment in the underlayer film forming material for lithography is preferably 1 to 100% by mass, more preferably 10 to 100% by mass, still more preferably 50 to 100% by mass, particularly preferably 100% by mass, from the viewpoint of coatability and quality stability.

The underlayer film forming material for lithography of the present embodiment is applicable to a wet process and is excellent in heat resistance and etching resistance. Furthermore, the underlayer film forming material for lithography of the present embodiment employs the above substances and can therefore form an underlayer film that is prevented from deteriorating during high temperature baking and is also excellent in etching resistance against oxygen plasma etching or the like. Moreover, the underlayer film forming material for lithography of the present embodiment is also excellent in adhesiveness to a resist layer and can therefore produce an excellent resist pattern. The underlayer film forming material for lithography of the present embodiment may contain an already known underlayer film forming material for lithography or the like, within the range not deteriorating the effect of the present invention.

### [Composition for forming an underlayer film for lithography]

The composition for forming an underlayer film for lithography of the present embodiment comprises the above underlayer film forming material for lithography and a solvent.

### [Solvent]

A publicly known solvent can be arbitrarily used as the solvent in the composition for forming an underlayer film for lithography of the present embodiment as long as at least the above component (A) dissolves.

Specific examples of the solvent include, but not particularly limited to: ketone-based solvents such as acetone, methyl ethyl ketone, methyl isobutyl ketone, and cyclohexanone; cellosolve-based solvents such as propylene glycol monomethyl ether and propylene glycol monomethyl ether acetate; ester-based solvents such as ethyl lactate, methyl acetate, ethyl acetate, butyl acetate, isoamyl acetate, ethyl lactate, methyl methoxypropionate, and methyl hydroxyisobutyrate; alcohol-based solvents such as methanol, ethanol, isopropanol, and 1-ethoxy-2-propanol; and aromatic hydrocarbons such as toluene, xylene, and anisole. These solvents can be used alone as one kind or used in combination of two or more kinds.

Among the above solvents, cyclohexanone, propylene glycol monomethyl ether, propylene glycol monomethyl ether acetate, ethyl lactate, methyl hydroxyisobutyrate, or anisole is particularly preferable from the viewpoint of safety.

The content of the solvent is not particularly limited and is preferably 100 to 10,000 parts by mass per 100 parts by mass of the above underlayer film forming material, more preferably 200 to 5,000 parts by mass, and still more preferably 200 to 1,000 parts by mass, from the viewpoint of solubility and film formation.

### [Crosslinking agent]

The composition for forming an underlayer film for lithography of the present embodiment may contain a crosslinking agent, if required, from the viewpoint of, for example, suppressing intermixing. The crosslinking agent that may be used in the present embodiment is not particularly limited, but a crosslinking agent described in, for example, International Publication No. WO 2013/024779 can be used. In the present embodiment, the crosslinking agent can be used alone or in combination of two or more kinds.

Specific examples of the crosslinking agent that may be used in the present embodiment include, but not particularly limited to, phenol compounds, epoxy compounds, cyanate compounds, amino compounds, benzoxazine compounds, acrylate compounds, melamine compounds, guanamine compounds, glycoluril compounds, urea compounds, isocyanate compounds, and azide compounds. These crosslinking agents can be used alone as one kind or can be used in combination of two or more kinds. Among them, a benzoxazine compound, an epoxy compound or a cyanate compound is preferable, and a benzoxazine compound is more preferable from the viewpoint of improvement in etching resistance.

As the above phenol compound, a publicly known compound can be used. Examples of phenols include phenol as well as alkylphenols such as cresols and xylenols, polyhydric phenols such as hydroquinone, polycyclic phenols such as naphthols and naphthalenediols, bisphenols such as bisphenol A and bisphenol F, and polyfunctional phenol compounds such as phenol novolac and phenol aralkyl resins. Among them, an aralkyl-based phenol resin is preferred from the viewpoint of heat resistance and solubility.

As the above epoxy compound, a publicly known compound can be used and is selected from among compounds having two or more epoxy groups in one molecule. Examples thereof include, but not particularly limited to, epoxidation products of dihydric phenols such as bisphenol A, bisphenol F, 3,3',5,5'-tetramethyl-bisphenol F, bisphenol S, fluorene bisphenol, 2,2'-biphenol, 3,3',5,5'-tetramethyl-4,4'-dihydroxybiphenol, resorcin, and naphthalenediols, epoxidation products of trihydric or higher phenols such as tris-(4-hydroxyphenyl)methane, 1,1,2,2-tetrakis(4-hydroxyphenyl)ethane, tris(2,3-epoxypropyl) isocyanurate, trimethylolmethane triglycidyl ether, trimethylolpropane triglycidyl ether, triethylolethane triglycidyl ether, phenol novolac, and o-cresol novolac, epoxidation products of co-condensed resins of dicyclopentadiene and phenols, epoxidation products of phenol aralkyl resins synthesized from phenols and paraxylylene dichloride, epoxidation products of biphenyl aralkyl-based phenol resins synthesized from phenols and bischloromethylbiphenyl, and epoxidation products of naphthol aralkyl resins synthesized from naphthols and paraxylylene dichloride. These epoxy resins may be used alone or in combination of two or more kinds. An epoxy resin that is in a solid state at normal temperature, such as an epoxy resin obtained from a phenol aralkyl resin or a biphenyl aralkyl resin is preferable from the viewpoint of heat resistance and solubility.

The above cyanate compound is not particularly limited as long as the compound has two or more cyanate groups in one molecule, and a publicly known compound can be used. In the present embodiment, preferable examples of the cyanate compound include cyanate compounds having a structure where hydroxy groups of a compound having two or more hydroxy groups in one molecule are replaced with cyanate groups. Also, the cyanate compound preferably has an aromatic group, and a structure where a cyanate group is directly bonded to an aromatic group can be preferably used. Examples of such a cyanate compound include cyanate compounds having a structure where hydroxy groups of bisphenol A, bisphenol F, bisphenol M, bisphenol P, bisphenol E, a phenol novolac resin, a cresol novolac resin, a dicyclopentadiene novolac resin, tetramethylbisphenol F, a bisphenol A novolac resin, brominated bisphenol A, a brominated phenol novolac resin, trifunctional phenol, tetrafunctional phenol, naphthalene-based phenol, biphenyl-based phenol, a phenol aralkyl resin, a biphenyl aralkyl resin, a naphthol aralkyl resin, a dicyclopentadiene aralkyl resin, alicyclic phenol, phosphorus-containing phenol, or the like are replaced with cyanate groups. These cyanate compounds may be used alone or in arbitrary combination of two or more kinds. Also, the above cyanate compound may be in any form of a monomer, an oligomer and a resin.

Examples of the above amino compound include m-phenylenediamine, p-phenylenediamine, 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,4'-diaminodiphenylsulfone, 3,3'-diaminodiphenylsulfone, 4,4'-diaminodiphenyl sulfide, 3,4'-diaminodiphenyl sulfide, 3,3'-diaminodiphenyl sulfide, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, bis[4-(3-aminophenoxy)phenyl] ether, 9,9-bis(4-aminophenyl)fluorene, 9,9-bis(4-amino-3-chlorophenyl)fluorene, 9,9-bis(4-amino-3-fluorophenyl)fluorene, O-tolidine, m-tolidine, 4,4'-diaminobenzanilide, 2,2'-bis(trifluoromethyl)-4,4'-diaminobiphenyl, 4-aminophenyl-4-aminobenzoate, and 2-(4-aminophenyl)-6-aminobenzoxazole. Further examples thereof include aromatic amines such as 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,3'-diaminodiphenylsulfone, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, and bis[4-(3-aminophenoxy)phenyl] ether, alicyclic amines such as diaminocyclohexane, diaminodicyclohexylmethane, dimethyl-diaminodicyclohexylmethane, tetramethyl-diaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bisaminomethylcyclohexane, and isophoronediamine, and aliphatic amines such as ethylenediamine, hexamethylenediamine, octamethylenediamine, decamethylenediamine, diethylenetriamine, and triethylenetetramine.

Examples of the above benzoxazine compound include P-d-based benzoxazines obtained from difunctional diamines and monofunctional phenols, and F-a-based benzoxazines obtained from monofunctional diamines and difunctional phenols.

Specific examples of the melamine compounds include hexamethylolmelamine, hexamethoxymethylmelamine, a compound in which 1 to 6 methylol groups of hexamethylolmelamine are methoxymethylated or a mixture thereof, hexamethoxyethylmelamine, hexaacyloxymethylmelamine, and a compound in which 1 to 6 methylol groups of hexamethylolmelamine are acyloxymethylated or a mixture thereof.

Specific examples of the guanamine compounds include tetramethylolguanamine, tetramethoxymethylguanamine, a compound in which 1 to 4 methylol groups of tetramethylolguanamine are methoxymethylated or a mixture thereof, tetramethoxyethylguanamine, tetraacyloxyguanamine, and a compound in which 1 to 4 methylol groups of tetramethylolguanamine are acyloxymethylated or a mixture thereof.

Specific examples of the glycoluril compounds include tetramethylolglycoluril, tetramethoxyglycoluril, tetramethoxymethylglycoluril, a compound in which 1 to 4 methylol groups of tetramethylolglycoluril are methoxymethylated or a mixture thereof, and a compound in which 1 to 4 methylol groups of tetramethylolglycoluril are acyloxymethylated or a mixture thereof.

Specific examples of the urea compounds include tetramethylolurea, tetramethoxymethylurea, a compound in which 1 to 4 methylol groups of tetramethylolurea are methoxymethylated or a mixture thereof, and tetramethoxyethylurea.

In the present embodiment, a crosslinking agent having at least one allyl group may be used from the viewpoint of improvement in crosslinkability. Specific examples of the crosslinking agent having at least one allyl group include, but not limited to, allylphenols such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, and bis(3-allyl-4-hydroxyphenyl) ether, allyl cyanates such as 2,2-bis(3-allyl-4-cyanatophenyl)propane, 1,1,1,3,3,3-hexafluoro-2,2-bis(3-allyl-4-cyanatophenyl)propane, bis(3-allyl-4-cyanatophenyl)sulfone, bis(3-allyl-4-cyanatophenyl) sulfide, and bis(3-allyl-4-cyanatophenyl) ether, diallyl phthalate, diallyl isophthalate, diallyl terephthalate, triallyl isocyanurate, trimethylolpropane diallyl ether, and pentaerythritol allyl ether. These crosslinking agents may be alone, or may be a mixture of two or more kinds. Among them, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, or bis(3-allyl-4-hydroxyphenyl) ether is preferable.

In the composition for forming an underlayer film for lithography of the present embodiment, the content of the crosslinking agent is not particularly limited and is preferably 5 to 50 parts by mass per 100 parts by mass of the underlayer film forming material, and more preferably 10 to 40 parts by mass. By the above preferable range, a mixing event with a resist layer tends to be prevented. Also, an antireflection effect is enhanced, and film formability after crosslinking tends to be enhanced.

### [Crosslinking promoting agent]

In the underlayer film forming material of the present embodiment, if required, a crosslinking promoting agent for accelerating crosslinking and curing reaction can be used.

The crosslinking promoting agent is not particularly limited as long as the crosslinking promoting agent accelerates crosslinking or curing reaction, and examples thereof include amines, imidazoles, organic phosphines, and Lewis acids. These crosslinking promoting agents can be used alone as one kind or can be used in combination of two or more kinds. Among them, an imidazole or an organic phosphine is preferable, and an imidazole is more preferable from the viewpoint of decrease in crosslinking temperature.

Examples of the crosslinking promoting agent include, but not limited to, tertiary amines such as 1,8-diazabicyclo(5,4,0)undecene-7, triethylenediamine, benzyldimethylamine, triethanolamine, dimethylaminoethanol, and tris(dimethylaminomethyl)phenol; imidazoles such as 2-methylimidazole, 2-phenylimidazole, 2-ethyl-4-methylimidazole, 2-phenyl-4-methylimidazole, 2-heptadecylimidazole, and 2,4,5-triphenylimidazole; organic phosphines such as tributylphosphine, methyldiphenylphosphine, triphenylphosphine, diphenylphosphine, and phenylphosphine; tetra substituted phosphonium-tetra substituted borates such as tetraphenylphosphonium-tetraphenyl borate, tetraphenylphosphonium-ethyltriphenyl borate, and tetrabutylphosphonium-tetrabutyl borate; and tetraphenylboron salts such as 2-ethyl-4-methylimidazole-tetraphenyl borate and N-methylmorpholine-tetraphenyl borate.

The content of the crosslinking promoting agent is usually preferably 0.1 to 10 parts by mass based on 100 parts by mass of the total mass of the composition, and is more preferably 0.1 to 5 parts by mass, and still more preferably 0.1 to 3 parts by mass, from the viewpoint of easy control and cost efficiency.

### [Radical polymerization initiator]

The underlayer film forming material of the present embodiment can comprise, if required, a radical polymerization initiator. The radical polymerization initiator may be a photopolymerization initiator that initiates radical polymerization by light, or may be a thermal polymerization initiator that initiates radical polymerization by heat. The radical polymerization initiator can be at least one selected from the group consisting of a ketone-based photopolymerization initiator, an organic peroxide-based polymerization initiator and an azo-based polymerization initiator.

Such a radical polymerization initiator is not particularly limited, and a radical polymerization initiator conventionally used can be arbitrarily adopted. Examples thereof include ketone-based photopolymerization initiators such as 1-hydroxy cyclohexyl phenyl ketone, benzyl dimethyl ketal, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methylpropionyl)-benzyl]phenyl}-2-methylpropan-1-one, 2,4,6-trimethylbenzoyl-diphenyl-phosphine oxide, and bis(2,4,6-trimethylbenzoyl)-phenylphosphine oxide, and organic peroxide-based polymerization initiators such as methyl ethyl ketone peroxide, cyclohexanone peroxide, methylcyclohexanone peroxide, methyl acetoacetate peroxide, acetyl acetate peroxide, 1,1-bis(t-hexylperoxy)-3,3,5-trimethylcyclohexane, 1,1-bis(t-hexylperoxy)-cyclohexane, 1,1-bis(t-butylperoxy)-3,3,5-trimethylcyclohexane, 1,1-bis(t-butylperoxy)-2-methylcyclohexane, 1,1-bis(t-butylperoxy)-cyclohexane, 1,1-bis(t-butylperoxy)cyclododecane, 1,1-bis(t-butylperoxy)butane, 2,2-bis(4,4-di-t-butylperoxycyclohexyl)propane, p-menthane hydroperoxide, diisopropylbenzene hydroperoxide, 1,1,3,3-tetramethylbutyl hydroperoxide, cumene hydroperoxide, t-hexyl hydroperoxide, t-butyl hydroperoxide, α,α'-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexyne-3, isobutyryl peroxide, 3,5,5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexanoate, 1-cyclohexyl-1-methylethyl peroxy-2-ethylhexanoate, t-hexyl peroxy-2-ethylhexanoate, t-butyl peroxy-2-ethylhexanoate, t-hexyl peroxyisopropylmonocarbonate, t-butyl peroxyisobutyrate, t-butyl peroxymalate, t-butyl peroxy-3,5,5-trimethylhexanoate, t-butyl peroxylaurate, t-butyl peroxyisopropylmonocarbonate, t-butyl peroxy-2-ethylhexylmonocarbonate, t-butyl peroxyacetate, t-butyl peroxy-m-toluylbenzoate, t-butyl peroxybenzoate, bis(t-butylperoxy) isophthalate, 2,5-dimethyl-2,5-bis(m-toluylperoxy)hexane, t-hexyl peroxybenzoate, 2,5-dimethyl-2,5-bis(benzoylperoxy)hexane, t-butyl peroxyallylmonocarbonate, t-butyltrimethylsilyl peroxide, 3,3',4,4'-tetra(t-butylperoxycarbonyl)benzophenone, and 2,3-dimethyl-2,3-diphenylbutane.

Further examples of the radical polymerization initiator include azo-based polymerization initiators such as 2-phenylazo-4-methoxy-2,4-dimethylvaleronitrile, 1-[(1-cyano-1-methylethyl)azo]formamide, 1,1'-azobis(cyclohexane-1-carbonitrile), 2,2'-azobis(2-methylbutyronitrile), 2,2'-azobisisobutyronitrile, 2,2'-azobis(2,4-dimethylvaleronitrile), 2,2'-azobis(2-methylpropionamidine) dihydrochloride, 2,2'-azobis(2-methyl-N-phenylpropionamidine) dihydrochloride, 2,2'-azobis[N-(4-chlorophenyl)-2-methylpropionamidine]dihydride chloride, 2,2'-azobis[N-(4-hydrophenyl)-2-methylpropionamidine] dihydrochloride, 2,2'-azobis[2-methyl-N-(phenylmethyl)propionamidine] dihydrochloride, 2,2'-azobis[2-methyl-N-(2-propenyl)propionamidine] dihydrochloride, 2,2'-azobis[N-(2-hydroxyethyl)-2-methylpropionamidine] dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane] dihydrochloride, 2,2'-azobis[2-[1-(2-hydroxyethyl)-2-imidazolin-2-yl]propane] dihydrochloride, 2,2'-azobis[2-(2-imidazolin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)ethyl]propionamide], 2,2'-azobis[2-methyl-N-(2-hydroxyethyl)propionamide], 2,2'-azobis(2-methylpropionamide), 2,2'-azobis(2,4,4-trimethylpentane), 2,2'-azobis(2-methylpropane), dimethyl-2,2-azobis(2-methylpropionate), 4,4'-azobis(4-cyanopentanoic acid), and 2,2'-azobis[2-(hydroxymethyl)propionitrile]. As the radical polymerization initiator of the present embodiment, one kind thereof may be used alone, or two or more kinds may be used in combination. Alternatively, the radical polymerization initiator of the present embodiment may be used in further combination with an additional publicly known polymerization initiator.

The content of the radical polymerization initiator can be a stoichiometrically necessary amount and is preferably 0.05 to 25 parts by mass, and more preferably 0.1 to 10 parts by mass, based on 100 parts by mass of the total mass of the composition containing the above compound or resin. When the content of the radical polymerization initiator is 0.05% parts by mass or more, there is a tendency that curing can be prevented from being insufficient. On the other hand, when the content of the radical polymerization initiator is 25% parts by mass or less, there is a tendency that the long term storage stability of the underlayer film forming material at room temperature can be prevented from being impaired.

### [Acid generating agent]

The composition for forming an underlayer film for lithography of the present embodiment may contain an acid generating agent, if required, from the viewpoint of, for example, further accelerating crosslinking reaction by heat. An acid generating agent that generates an acid by thermal decomposition, an acid generating agent that generates an acid by light irradiation, and the like are known, any of which can be used.

The acid generating agent is not particularly limited, and, for example, an acid generating agent described in International Publication No. WO2013/024779 can be used. In the present embodiment, the acid generating agent can be used alone or in combination of two or more kinds.

In the composition for forming an underlayer film for lithography of the present embodiment, the content of the acid generating agent is not particularly limited and is preferably 0.1 to 50 parts by mass per 100 parts by mass of the underlayer film forming material, and more preferably 0.5 to 40 parts by mass. By the above preferable range, crosslinking reaction tends to be enhanced by an increased amount of an acid generated. Also, a mixing event with a resist layer tends to be prevented.

### [Basic compound]

The composition for forming an underlayer film for lithography of the present embodiment may further comprise a basic compound from the viewpoint of, for example, improving storage stability.

The basic compound plays a role as a quencher against acids in order to prevent crosslinking reaction from proceeding due to a trace amount of an acid generated by the acid generating agent. Examples of such a basic compound include, but not particularly limited to, primary, secondary or tertiary aliphatic amines, amine blends, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amide derivatives, and imide derivatives.

The basic compound used in the present embodiment is not particularly limited, and, for example, a basic compound described in International Publication No. WO2013/024779 can be used. In the present embodiment, the basic compound can be used alone or in combination of two or more kinds.

In the composition for forming an underlayer film for lithography of the present embodiment, the content of the basic compound is not particularly limited and is preferably 0.001 to 2 parts by mass per 100 parts by mass of the underlayer film forming material, and more preferably 0.01 to 1 parts by mass. By the above preferable range, storage stability tends to be enhanced without excessively deteriorating crosslinking reaction.

### [Further additive agent]

The composition for forming an underlayer film for lithography of the present embodiment may also comprises an additional resin and/or compound for the purpose of conferring thermosetting properties or controlling absorbance. Examples of such an additional resin and/or compound include, but not particularly limited to, naphthol resin, xylene resin naphthol-modified resin, phenol-modified resin of naphthalene resin, polyhydroxystyrene, dicyclopentadiene resin, resins containing (meth)acrylate, dimethacrylate, trimethacrylate, tetramethacrylate, a naphthalene ring such as vinylnaphthalene or polyacenaphthylene, a biphenyl ring such as phenanthrenequinone or fluorene, or a heterocyclic ring having a heteroatom such as thiophene or indene, and resins containing no aromatic ring; and resins or compounds containing an alicyclic structure, such as rosin-based resin, cyclodextrin, adamantine(poly)ol, tricyclodecane(poly)ol, and derivatives thereof. The composition for forming an underlayer film for lithography of the present embodiment may further contain a publicly known additive agent. Examples of the above publicly known additive agent include, but not limited to, ultraviolet absorbers, surfactants, colorants, and nonionic surfactants.

### [Formation method of underlayer film for lithography]

The method for forming an underlayer film for lithography according to the present embodiment includes the step of forming an underlayer film on a supporting material using the composition for forming an underlayer film for lithography of the present embodiment.

### [Resist pattern formation method using composition for forming an underlayer film for lithography]

A resist pattern formation method using the composition for forming an underlayer film for lithography of the present embodiment has the steps of: forming an underlayer film on a supporting material using the composition for forming an underlayer film for lithography of the present embodiment (step (A-1)); forming at least one photoresist layer on the underlayer film (step (A-2)); and irradiating a predetermined region of the photoresist layer with radiation for development, thereby forming a resist pattern (step (A-3)).

### [Circuit pattern formation method using composition for forming an underlayer film for lithography]

A circuit pattern formation method using the composition for forming an underlayer film for lithography of the present embodiment has the steps of: forming an underlayer film on a supporting material using the composition for forming an underlayer film for lithography of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); after the step (B-4), etching the intermediate layer film with the resist pattern as a mask, thereby forming an intermediate layer film pattern (step (B-5)); etching the underlayer film with the obtained intermediate layer film pattern as an etching mask, thereby forming an underlayer film pattern (step (B-6)); and etching the supporting material with the obtained underlayer film pattern as an etching mask, thereby forming a pattern on the supporting material (step (B-7)).

The underlayer film for lithography of the present embodiment is not particularly limited by its formation method as long as it is formed from the composition for forming an underlayer film for lithography of the present embodiment. A publicly known approach can be applied thereto. The underlayer film can be formed by, for example, applying the composition for forming an underlayer film for lithography of the present embodiment onto a supporting material by a publicly known coating method or printing method such as spin coating or screen printing, and then removing an organic solvent by volatilization or the like.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. The baking time is not particularly limited and is preferably in the range of 10 to 300 seconds. The thickness of the underlayer film can be arbitrarily selected according to required performance and is not particularly limited, but is preferably 30 to 20,000 nm, and more preferably 50 to 15,000 nm.

After preparing the underlayer film on the supporting material, a silicon-containing resist layer or a usual single-layer resist made of hydrocarbon thereon can be prepared on the underlayer film in the case of a two-layer process. In the case of a three-layer process, a silicon-containing intermediate layer can be prepared on the underlayer film, and a silicon-free single-layer resist layer can be further prepared on the silicon-containing intermediate layer. In these cases, a publicly known photoresist material can be arbitrarily selected and used for forming the resist layer, without particular limitations.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and if required, a basic compound or the like is preferably used, from the viewpoint of oxygen gas etching resistance. Herein, a publicly known polymer that is used in this kind of resist material can be used as the silicon atom-containing polymer.

A polysilsesquioxane-based intermediate layer is preferably used as the silicon-containing intermediate layer for a three-layer process. By imparting effects as an antireflection film to the intermediate layer, there is a tendency that reflection can be effectively suppressed. For example, use of a material containing a large amount of an aromatic group and having high supporting material etching resistance as the underlayer film in a process for exposure at 193 nm tends to increase a k value and enhance supporting material reflection. However, the intermediate layer suppresses the reflection so that the supporting material reflection can be 0.5% or less. The intermediate layer having such an antireflection effect is not limited, and polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly effective as an antireflection film prepared by CVD is not limited, and, for example, a SiON film is known. In general, the formation of an intermediate layer by a wet process such as spin coating or screen printing is more convenient and more advantageous in cost, as compared with CVD. The upper layer resist for a three-layer process may be positive type or negative type, and the same as a single-layer resist generally used can be used.

The underlayer film according to the present embodiment can also be used as an antireflection film for usual single-layer resists or an underlying material for suppression of pattern collapse. The underlayer film of the present embodiment is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally performed. This prebaking is preferably performed at 80 to 180°C in the range of 10 to 300 seconds. Then, exposure, post-exposure baking (PEB), and development can be performed according to a conventional method to obtain a resist pattern. The thickness of the resist film is not particularly limited and is preferably 30 to 500 nm, and more preferably 50 to 400 nm.

The exposure light can be arbitrarily selected and used according to the photoresist material to be used. General examples thereof can include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the above method, pattern collapse is suppressed by the underlayer film according to the present embodiment. Therefore, use of the underlayer film according to the present embodiment can produce a finer pattern and can reduce an exposure amount necessary for obtaining the resist pattern.

Next, etching is performed with the obtained resist pattern as a mask. Gas etching is preferably used as the etching of the underlayer film in a two-layer process. The gas etching is preferably etching using oxygen gas. In addition to oxygen gas, an inert gas such as He or Ar, or CO, CO₂, NH₃, SO₂, N₂, NO₂, or H₂ gas may be added. Alternatively, the gas etching may be performed with CO, CO₂, NH₃, N₂, NO₂, or H₂ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the above two-layer process is applicable. Particularly, it is preferable to process the intermediate layer in a three-layer process by using chlorofluorocarbon-based gas and using the resist pattern as a mask. Then, as mentioned above, for example, the underlayer film can be processed by oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for forming the nitride film is not limited, and, for example, a method described in Japanese Patent Laid-Open No. 2002-334869 or WO2004/066377 can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting effects as an antireflection film to the resist intermediate layer film, there is a tendency that reflection can be effectively suppressed. A specific material for the polysilsesquioxane-based intermediate layer is not limited, and, for example, a material described in Japanese Patent Laid-Open No. 2007-226170 or Japanese Patent Laid-Open No. 2007-226204 can be used.

The subsequent etching of the supporting material can also be performed by a conventional method. For example, the supporting material made of SiO₂ or SiN can be etched mainly using chlorofluorocarbon-based gas, and the supporting material made of p-Si, Al, or W can be etched mainly using chlorine- or bromine-based gas. In the case of etching the supporting material with chlorofluorocarbon-based gas, the silicon-containing resist of the two-layer resist process or the silicon-containing intermediate layer of the three-layer process is peeled at the same time with supporting material processing. On the other hand, in the case of etching the supporting material with chlorine- or bromine-based gas, the silicon-containing resist layer or the silicon-containing intermediate layer is separately peeled and in general, peeled by dry etching using chlorofluorocarbon-based gas after supporting material processing.

A feature of the underlayer film according to the present embodiment is that it is excellent in etching resistance of these supporting materials. The supporting material can be arbitrarily selected from publicly known ones and used and is not particularly limited. Examples thereof include Si, α-Si, p-Si, SiO₂, SiN, SiON, W, TiN, and Al. The supporting material may be a laminate having a film to be processed (supporting material to be processed) on a base material (support). Examples of such a film to be processed include various low-k films such as Si, SiO₂, SiON, SiN, p-Si, α-Si, W, W-Si, Al, Cu, and Al-Si, and stopper films thereof. A material different from that for the base material (support) is generally used. The thickness of the supporting material to be processed or the film to be processed is not particularly limited and is preferably 50 to 1,000,000 nm, and more preferably 75 to 500,000 nm.

### [Resist permanent film]

The above composition can also be used to prepare a resist permanent film. The resist permanent film prepared by coating with the above composition is suitable as a permanent film that also remains in a final product, if required, after formation of a resist pattern. Specific examples of the permanent film include in relation to semiconductor devices, solder resists, package materials, underfill materials, package adhesive layers for circuit elements and the like, and adhesive layers between integrated circuit elements and circuit supporting materials, and in relation to thin displays, thin film transistor protecting films, liquid crystal color filter protecting films, black matrixes, and spacers. Particularly, the permanent film made of the above composition is excellent in heat resistance and humidity resistance and furthermore, also has the excellent advantage that contamination by sublimable components is reduced. Particularly, for a display material, a material that achieves all of high sensitivity, high heat resistance, and hygroscopic reliability with reduced deterioration in image quality due to significant contamination can be obtained.

In the case of using the above composition for resist permanent film purposes, a curing agent as well as, if required, various additive agents such as other resins, a surfactant, a dye, a filler, a crosslinking agent, and a dissolution promoting agent can be added and dissolved in an organic solvent to prepare a composition for resist permanent films.

The above film forming composition for lithography or composition for resist permanent films can be prepared by adding each of the above components and mixing them using a stirrer or the like. When the above composition for resist underlayer films or composition for resist permanent films contains a filler or a pigment, it can be prepared by dispersion or mixing using a dispersion apparatus such as a dissolver, a homogenizer, and a three-roll mill.

### [Method for purifying compound and/or resin]

The method for purifying the compound and/or the resin of the present embodiment comprises the steps of: obtaining a solution (S) by dissolving the compound of the present embodiment and/or the resin of the present embodiment in a solvent; and extracting impurities in the compound and/or the resin by bringing the obtained solution (S) into contact with an acidic aqueous solution (a first extraction step), wherein the solvent used in the step of obtaining the solution (S) contains an organic solvent that does not mix with water.

In the first extraction step, the resin is preferably a resin obtained by a reaction between the compound represented by the above formula (A) and a crosslinking compound. According to the purification method of the present embodiment, the contents of various metals that may be contained as impurities in the compound or the resin having a specific structure described above can be reduced.

More specifically, in the purification method of the present embodiment, the compound and/or the resin is dissolved in an organic solvent that does not mix with water to obtain the solution (S), and further, extraction treatment can be carried out by bringing the solution (S) into contact with an acidic aqueous solution. Thereby, metals contained in the solution (S) containing the compound and/or the resin of the present embodiment are transferred to the aqueous phase, then the organic phase and the aqueous phase are separated, and thus the compound and/or the resin of the present embodiment having a reduced metal content can be obtained.

The compound and/or the resin of the present embodiment used in the purification method of the present embodiment may be alone, or may be a mixture of two or more kinds. Also, the compound and/or the resin of the present embodiment may contain various surfactants, various crosslinking agents, various acid generating agents, various stabilizers, and the like.

The solvent that does not mix with water used in the present embodiment is not particularly limited, but is preferably an organic solvent that is safely applicable to semiconductor manufacturing processes, and specifically it is an organic solvent having a solubility in water at room temperature of less than 30%, and more preferably is an organic solvent having a solubility of less than 20% and particularly preferably less than 10%. The amount of the organic solvent used is preferably 1 to 100 times the mass of the compound and/or the resin of the present embodiment to be used.

Specific examples of the solvent that does not mix with water include, but not limited to, those described in International Publication No. WO2015/080240. Among these, toluene, 2-heptanone, cyclohexanone, cyclopentanone, methyl isobutyl ketone, propylene glycol monomethyl ether acetate, ethyl acetate, and the like are preferable, methyl isobutyl ketone, ethyl acetate, cyclohexanone, and propylene glycol monomethyl ether acetate are more preferable, and methyl isobutyl ketone and ethyl acetate are still more preferable. Methyl isobutyl ketone, ethyl acetate, and the like have relatively high saturation solubility for the compound and the resin of the present embodiment and a relatively low boiling point, and it is thus possible to reduce the load in the case of industrially distilling off the solvent and in the step of removing the solvent by drying. These solvents can be each used alone, and can be used as a mixture of two or more kinds.

Examples of the acidic aqueous solution used in the purification method of the present embodiment include, but not particularly limited to, those described in International Publication No. WO2015/080240. These acidic aqueous solutions can be each used alone, and can be also used as a combination of two or more kinds. Among these acidic aqueous solutions, aqueous solutions of one or more mineral acids selected from the group consisting of hydrochloric acid, sulfuric acid, nitric acid, and phosphoric acid, or aqueous solutions of one or more organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are preferable, aqueous solutions of sulfuric acid, nitric acid, and carboxylic acids such as acetic acid, oxalic acid, tartaric acid, and citric acid are more preferable, aqueous solutions of sulfuric acid, oxalic acid, tartaric acid, and citric acid are still more preferable, and an aqueous solution of oxalic acid is further preferable. It is considered that polyvalent carboxylic acids such as oxalic acid, tartaric acid, and citric acid coordinate with metal ions and provide a chelating effect, and thus tend to be capable of more effectively removing metals. As for water used herein, it is preferable to use water, the metal content of which is small, such as ion exchanged water, according to the purpose of the purification method of the present embodiment.

The pH of the acidic aqueous solution used in the purification method of the present embodiment is not particularly limited, but it is preferable to regulate the acidity of the aqueous solution in consideration of an influence on the compound and/or the resin of the present embodiment. Normally, the pH range is about 0 to 5, and is preferably about pH 0 to 3.

The amount of the acidic aqueous solution used in the purification method of the present embodiment is not particularly limited, but it is preferable to regulate the amount from the viewpoint of reducing the number of extraction operations for removing metals and from the viewpoint of ensuring operability in consideration of the overall amount of fluid. From the above viewpoints, the amount of the acidic aqueous solution used is preferably 10 to 200 parts by mass, and more preferably 20 to 100 parts by mass, based on 100 parts by mass of the solution (S) .

In the purification method of the present embodiment, by bringing an acidic aqueous solution as described above into contact with the solution (S) containing the compound and/or the resin of the present embodiment and the solvent that does not mix with water, metals can be extracted from the compound or the resin in the solution (S).

In the purification method of the present embodiment, it is preferable that the solution (S) further contains an organic solvent that inadvertently mixes with water. When an organic solvent that mixes with water is contained, there is a tendency that the amount of the compound and/or the resin of the present embodiment charged can be increased, also the fluid separability is improved, and purification can be carried out at a high reaction vessel efficiency. The method for adding the organic solvent that mixes with water is not particularly limited. For example, any of a method involving adding it to the organic solvent-containing solution in advance, a method involving adding it to water or the acidic aqueous solution in advance, and a method involving adding it after bringing the organic solvent-containing solution into contact with water or the acidic aqueous solution. Among these, the method involving adding it to the organic solvent-containing solution in advance is preferable in terms of the workability of operations and the ease of managing the amount.

The organic solvent that mixes with water used in the purification method of the present embodiment is not particularly limited, but is preferably an organic solvent that is safely applicable to semiconductor manufacturing processes. The amount of the organic solvent used that mixes with water is not particularly limited as long as the solution phase and the aqueous phase separate, but is preferably 0.1 to 100 times, more preferably 0.1 to 50 times, and further preferably 0.1 to 20 times the mass of the compound and/or the resin of the present embodiment.

Specific examples of the organic solvent used in the purification method of the present embodiment that mixes with water include those described in International Publication No. WO2015/080240. Among these, N-methylpyrrolidone, propylene glycol monomethyl ether, and the like are preferable, and N-methylpyrrolidone and propylene glycol monomethyl ether are more preferable. These solvents can be each used alone, and can be used as a mixture of two or more kinds.

The temperature when extraction treatment is carried out is generally in the range of 20 to 90°C, and preferably 30 to 80°C. The extraction operation is carried out, for example, by thoroughly mixing the solution (S) and the acidic aqueous solution by stirring or the like and then leaving the obtained mixed solution to stand still. Thereby, metals contained in the solution containing the compound and/or the resin of the present embodiment and the organic solvents are transferred to the aqueous phase. Also, by this operation, the acidity of the solution is lowered, and the degradation of the compound and/or the resin of the present embodiment can be suppressed.

By being left to stand still, the mixed solution is separated into an aqueous phase and a solution phase containing the compound and/or the resin of the present embodiment and the solvents, and thus the solution phase containing the compound and/or the resin of the present embodiment and the solvents is recovered by decantation. The time for leaving the mixed solution to stand still is not particularly limited, but it is preferable to regulate the time for leaving the mixed solution to stand still from the viewpoint of attaining good separation of the solution phase containing the solvents and the aqueous phase. Normally, the time for leaving the mixed solution to stand still is 1 minute or longer, preferably 10 minutes or longer, and more preferably 30 minutes or longer. While the extraction treatment may be carried out once, it is effective to repeat mixing, leaving-to-stand-still, and separating operations multiple times.

It is preferable that the purification method of the present embodiment includes the step of extracting impurities in the compound or the resin by further bringing the solution phase containing the compound or the resin into contact with water after the first extraction step (the second extraction step). Specifically, for example, it is preferable that after the above extraction treatment is carried out using an acidic aqueous solution, the solution phase that is extracted and recovered from the aqueous solution and that contains the compound and/or the resin of the present embodiment and the solvents is further subjected to extraction treatment with water. The above extraction treatment with water is not particularly limited, and can be carried out, for example, by thoroughly mixing the solution phase and water by stirring or the like and then leaving the obtained mixed solution to stand still. The mixed solution after being left to stand still is separated into an aqueous phase and a solution phase containing the compound and/or the resin of the present embodiment and the solvents, and thus the solution phase containing the compound and/or the resin of the present embodiment and the solvents can be recovered by decantation.

Water used herein is preferably water, the metal content of which is small, such as ion exchanged water, according to the purpose of the present embodiment. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is possibly present in the thus-obtained solution containing the compound and/or the resin of the present embodiment and the solvents can be easily removed by performing vacuum distillation or a like operation. Also, if required, the concentration of the compound and/or the resin of the present embodiment can be regulated to be any concentration by adding a solvent to the above solution.

The method for isolating the compound and/or the resin of the present embodiment from the obtained solution containing the compound and/or the resin of the present embodiment and the solvents is not particularly limited, and publicly known methods can be carried out, such as reduced-pressure removal, separation by reprecipitation, and a combination thereof. Publicly known treatments such as concentration operation, filtration operation, centrifugation operation, and drying operation can be carried out if required.

### Examples

The embodiment of the present invention will be more specifically described with reference to examples below. However, the present embodiment is not particularly limited to these examples.

Methods for analyzing and evaluating a compound are as follows.

### <Molecular weight>

The molecular weight of the compound was measured by LC-MS analysis using Acquity UPLC/MALDI-Synapt HDMS manufactured by Waters Corp.

### <Measurement of thermal decomposition temperature>

EXSTAR 6000 DSC apparatus manufactured by SII NanoTechnology Inc. was used. About 5 mg of a sample was placed in an unsealed container made of aluminum, and the temperature was raised to 500°C at a temperature increase rate of 10°C/min in a nitrogen gas stream (30 ml/min). The temperature at which a decrease in baseline appeared was defined as the thermal decomposition temperature.

### <Method for testing heat resistance>

EXSTAR 6000 DSC apparatus manufactured by SII NanoTechnology Inc. was used. About 5 mg of a sample was placed in an unsealed container made of aluminum, and the temperature was raised to 500°C at a temperature increase rate of 10°C/min in a nitrogen gas stream (30 ml/min). The temperature at which a decrease in baseline appeared was defined as the thermal decomposition temperature (Tg). The heat resistance was evaluated according to the following criteria.
Evaluation A: The thermal decomposition temperature was ≥ 150°C
Evaluation C: The thermal decomposition temperature was < 150°C

### <Solubility>

The compound was added to a 50 ml screw bottle and stirred at 23°C for 1 hour using a magnetic stirrer. Then, the amount of the compound dissolved in propylene glycol monomethyl ether (PGME) was evaluated according to the following criteria.
Evaluation A: 10 wt% or more
Evaluation C: Less than 10 wt%

### (Synthesis Working Example 1) Synthesis of BisP-1

A container (internal capacity: 100 ml) equipped with a stirrer, a condenser tube, and a burette was prepared. To this container, 1.52 g (8.1 mmol) of 4,4'-biphenol (a reagent manufactured by Sigma-Aldrich), 0.56 g (4.7 mmol) of 4-methylbenzaldehyde (manufactured by Mitsubishi Gas Chemical Company Inc.), and 30 ml of 1,4-dioxane were added, and 0.4 g (2.3 mmol) of p-toluenesulfonic acid was added to prepare a reaction solution. The reaction solution was stirred at 90°C for 3.5 hours and reacted. Next, the reaction solution was cooled to 40°C, and 10 ml of hexane was dropped thereto. The reaction product was precipitated by cooling to 10°C, filtered, then washed with hexane, and then separated and purified by column chromatography to obtain 0.5 g of the objective compound (BisP-1) represented by the following formula.

The following peaks were found by 400 MHz-¹H-NMR, and the compound was confirmed to have a chemical structure of the following formula.
¹H-NMR: (d-DMSO, internal standard TMS)
δ (ppm) 9.67 (4H, O-H), 6.8-7.6 (18H, Ph-H), 5.48 (1H, C-H), 2.2 (3H, Ph-CH₃)

As a result of measuring the molecular weight of the obtained compound by the above method, it was 474.

### (Synthesis Working Examples 2 to 18) Synthesis of BisP-2 to BisP-18

The objective compounds BisP-2 to BisP-18 were obtained in the same way as in Synthesis Working Example 1 except that the raw materials 4,4'-biphenol and 4-methylbenzaldehyde were changed as shown in Table 1.

Each compound was identified using ¹H-NMR and a molecular weight.

### [Table 1]

**Table 1**

| Synthesis Working Example | Raw material 1 | Raw material 2 |
|---|---|---|
| 1 | 4,4'-Biphenol | 4-Methylbenzaldehyde |
| 2 | 2,2'-Biphenol | 4-Methylbenzaldehyde |
| 3 | 4,4'-Biphenol | 3,4-Dimethylbenzaldehyde |
| 4 | 2,2'-Biphenol | 3,4-Dimethylbenzaldehyde |
| 5 | 4,4'-Biphenol | 4-Isopropylbenzaldehyde |
| 6 | 2,2'-Biphenol | 4-Isopropylbenzaldehyde |
| 7 | 4,4'-Biphenol | 4-n-Propylbenzaldehyde |
| 8 | 2,2'-Biphenol | 4-n-Propylbenzaldehyde |
| 9 | 4,4'-Biphenol | 4-Isobutylbenzaldehyde |
| 10 | 2,2'-Biphenol | 4-Isobutylbenzaldehyde |
| 11 | 4,4'-Biphenol | 4-Hydroxybenzaldehyde |
| 12 | 2,2'-Biphenol | 4-Hydroxybenzaldehyde |
| 13 | 4,4'-Biphenol | 4-Cyclohexylbenzaldehyde |
| 14 | 2,2'-Biphenol | 4-Cyclohexylbenzaldehyde |
| 15 | 4,4'-Biphenol | 4'-Hydroxyacetophenone |
| 16 | 2,2'-Biphenol | 4'-Hydroxyacetophenone |
| 17 | 2-Phenylphenol | 4-Hydroxybenzaldehyde |
| 18 | 2-Phenylphenol | 4'-Hydroxyacetophenone |

### [Table 2]

**Table 2**

| Synthesis Working Example | Compound name | ¹H-NMR (d-DMSO, internal standard TMS) δ(ppm) | Molecular weight |
|---|---|---|---|
| 1 | BisP-1 | 9.67(4H,O-H), 6.8∼7.6(18H,Ph-H), 5.48(1 H,C-H)2.2(3H,Ph-CH₃) | 474 |
| 2 | BisP-2 | 8.98(4H,O-H), 7.0∼7.8(18H,Ph-H), 5.4(1H,C-H)2.2(3H,Ph-CH₃) | 474 |
| 3 | BisP-3 | 9.67(4H,O-H), 6.8∼7.6(18H,Ph-H), 5.48(1H,C-H)2.23,2.29(6H,Ph-CH₃) | 488 |
| 4 | BisP-4 | 8.98(4H,O-H), 7.0∼7.8(18H,Ph-H), 5.4(1H,C-H)2.23,2.29(6H,Ph-CH₃) | 488 |
| 5 | BisP-5 | 9.67(4H,O-H), 6.8∼7.6(18H,Ph-H), 5.48(1 H,C-H), 2.86(1 H,Ph-CH(CH₃)₂), 1.2(6H,-CH(CH₃)₂) | 502 |
| 6 | BisP-6 | 8.98(4H,O-H), 7.0∼7.8(18H,Ph-H), 5.4(1 H,C-H), 2.86(1 H,Ph-CH(CH₃)₂), 1.2(6H,-CH(CH₃)₂) | 502 |
| 7 | BisP-7 | 9.67(4H,O-H), 7.0∼7.6(18H,Ph-H), 5.48(1 H,C-H), 2.6(2H,Ph-CH₂), 1.6(2H,Ph-CH₂-CH₂), 0.9(3H ,Ph-CH₂-CH₂-CH₃) | 502 |
| 8 | BisP-8 | 8.98(4H,O-H), 7.0∼7.6(18H,Ph-H), 5.4(1 H,C-H), 2.6(2H,Ph-CH₂), 1.6(2H,Ph-CH₂-CH₂), 0.9(3H ,Ph-CH₂-CH₂-CH₃) | 502 |
| 9 | BisP-9 | 9.67(4H,O-H), 6.8∼7.6(18H,Ph-H), 5.48(1 H,C-H), 2.4(2H,Ph-CH₂), 1.8(1 H,Ph-CH₂-CH(CH₃)₂), 0.8(6H,-CH(CH₃)₂) | 516 |
| 10 | BisP-10 | 8.98(4H,O-H), 7.0∼7.8(18H,Ph-H), 5.4(1 H,C-H), 2.4(2H,Ph-CH₂), 1.8(1 H,Ph-CH₂-CH(CH₃)₂), 0.8(6H,-CH(CH₃)₂) | 516 |
| 11 | BisP-11 | 9.0∼9.68(5H,O-H), 6.6∼7.6(18H,Ph-H), 5.48(1 H,C-H) | 476 |
| 12 | BisP-12 | 8.98∼9.06(5H,O-H), 6.6∼7.8(18H,Ph-H), 5.4(1 H,C-H) | 476 |
| 13 | BisP-13 | 9.67(4H,O-H), 6.8∼7.6(18H,Ph-H), 1.4∼2.7(11 H,Ch-H), 5.48(1 H,C-H) | 542 |
| 14 | BisP-14 | 8.98(4H,O-H), 7.0∼7.8(18H,Ph-H), 1.4∼2.7(11 H,Ch-H), 5.4(1 H,C-H) | 542 |
| 15 | BisP-15 | 9.0∼9.7(5H,O-H), 7.0∼7.7(18H,Ph-H), 1.4∼2.7(11 H,Ch-H), 2.3(3H,C-Me) | 490 |
| 16 | BisP-16 | 9.0∼9.1(5H,O-H), 6.6∼7.8(18H,Ph-H), 2.3(3H,C- | 490 |
| 17 | BisP-17 | 9.0∼9.1(3H,O-H), 6.6∼7.6(20H,Ph-H), 5.4(1 H,C- | 444 |
| 18 | BisP-18 | 9.0∼9.1H) (3H,OH), 6.6∼7.6(20H,Ph-H), 2.3(1H,C- | 458 |

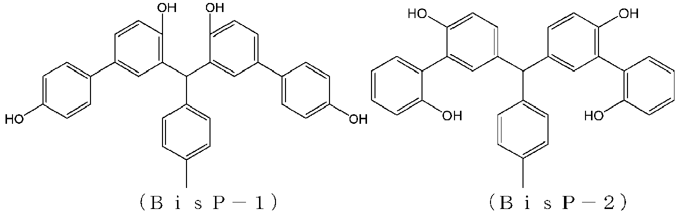

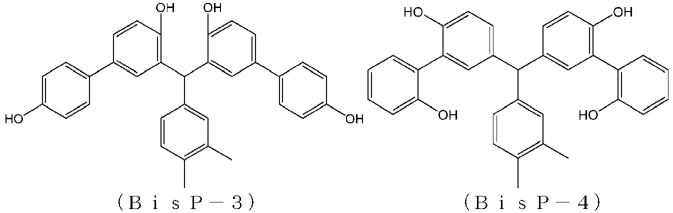

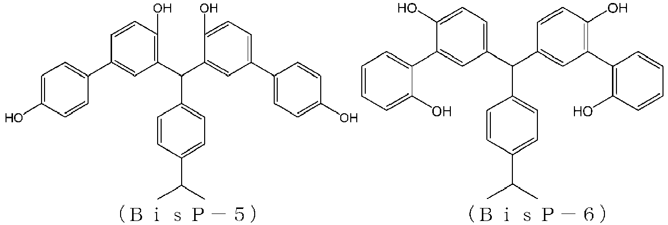

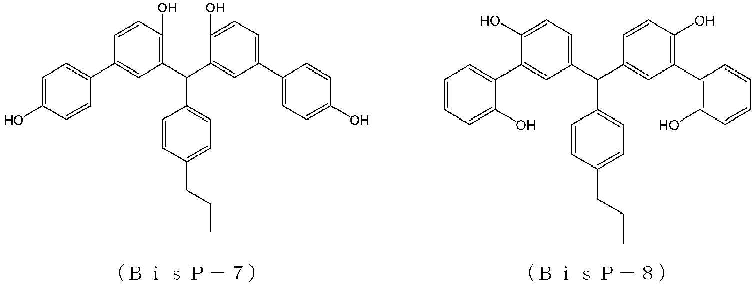

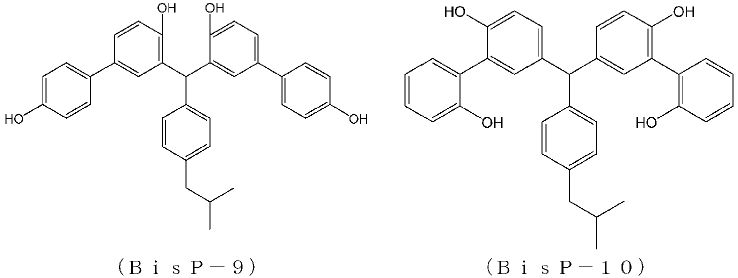

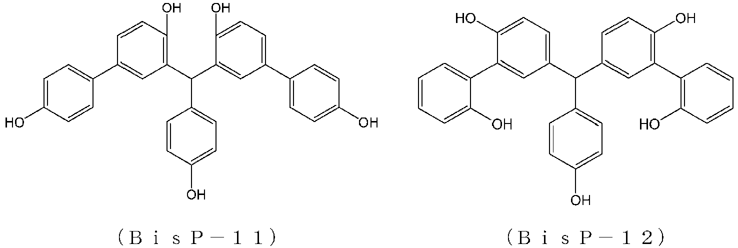

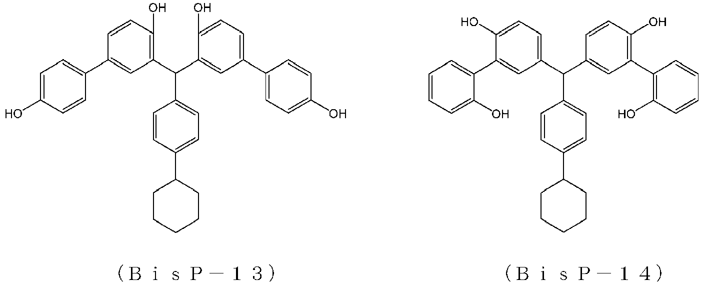

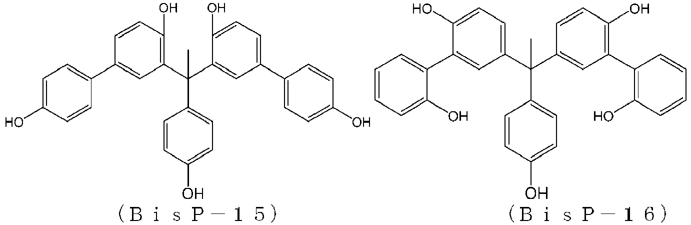

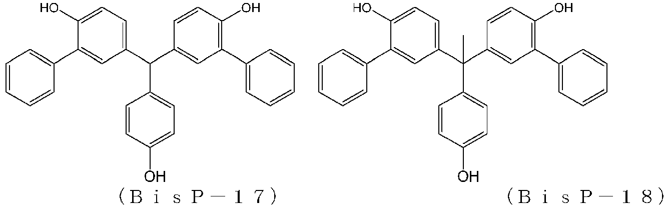

### (Synthesis Working Example 19) Synthesis of resin (R1-BisP-1)

A four necked flask (internal capacity: 1 L) equipped with a Dimroth condenser tube, a thermometer, and a stirring blade and having a detachable bottom was prepared. To this four necked flask, 37.7 g (70 mmol, manufactured by Mitsubishi Gas Chemical Company, Inc.) of the compound (BisP-1) obtained in Synthesis Example 1, 21.0 g (280 mmol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 180.0 g of o-xylene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and o-xylene was distilled off under reduced pressure to obtain 32.3 g of a brown solid resin (R1-BisP-1) .

The obtained resin (R1-BisP-1) had Mn: 2012, Mw: 3510, and Mw/Mn: 1.74.

### (Synthesis Working Example 20) Synthesis of resin (R2 - BisP-1)

A four necked flask (internal capacity: 1 L) equipped with a Dimroth condenser tube, a thermometer, and a stirring blade and having a detachable bottom was prepared. To this four necked flask, 37.7 g (70 mmol, manufactured by Mitsubishi Gas Chemical Company, Inc.) of compound (BisP-1) obtained in Synthesis Example 1, 50.9 g (280 mmol) of 4-biphenylaldehyde (manufactured by Mitsubishi Gas Chemical Company, Inc.), 100 mL of anisole (manufactured by Kanto Chemical Co., Inc.), and 10 mL of oxalic acid dihydrate (manufactured by Kanto Chemical Co., Inc.) were added in a nitrogen stream, and the mixture was reacted for 7 hours while refluxed at 100°C at normal pressure. Subsequently, 180.0 g of o-xylene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and the solvents and unreacted 4-biphenylaldehyde in the organic phase were distilled off under reduced pressure to obtain 38.5 g of a brown solid resin (R2-BisP-1).

The obtained resin (R2-BisP-1) had Mn: 1630, Mw: 2665, and Mw/Mn: 1.63.

### (Production Example 1)

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

Subsequently, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin thus obtained, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was added thereto, and the temperature was further raised to 220°C at which the mixture was reacted for 2 hours. After solvent dilution, neutralization and washing with water were performed, and the solvent was removed under reduced pressure to obtain 126.1 g of a black-brown solid modified resin (CR-1).

### [Examples 1 to 20]

Results of evaluating solubility in propylene glycol monomethyl ether (PGME) using BisP-1 to BisP-18, R1-BisP-1, and R2-BisP-2 are shown in Table 3.

### [Table 3]

**Table 3**

| | Compound | Solubility evaluation results |
|---|---|---|
| Example 1 | BisP-1 | A |
| Example 2 | BisP-2 | A |
| Example 3 | BisP-3 | A |
| Example 4 | BisP-4 | A |
| Example 5 | BisP-5 | A |
| Example 6 | BisP-6 | A |
| Example 7 | BisP-7 | A |
| Example 8 | BisP-8 | A |
| Example 9 | BisP-9 | A |
| Example 10 | BisP-10 | A |
| Example 11 | BisP-11 | A |
| Example 12 | BisP-12 | A |
| Example 13 | BisP-13 | A |
| Example 14 | BisP-14 | A |
| Example 15 | BisP-15 | A |
| Example 16 | BisP-16 | A |
| Example 17 | BisP-17 | A |
| Example 18 | BisP-18 | A |
| Example 19 | R1-BisP-1 | A |
| Example 20 | R2-BisP-1 | A |

As is evident from Table 3, all the compounds used in Example 1 to Example 20 were able to be confirmed to be excellent in solubility in a solvent.

### [Examples 21 to 38 and Comparative Example 1]

### (Heat resistance and resist performance)

Results of carrying out a heat resistance test and resist performance evaluation using BisP-1 to BisP-18 and CR-1 are shown in Table 4.

### (Preparation of resist composition)

A resist composition was prepared according to the recipe shown in Table 4 using each compound synthesized as described above. Among the components of the resist composition in Table 4, the following acid generating agent (C), acid diffusion controlling agent (E), and solvent were used.
Acid generating agent(C)
P-1: triphenylbenzenesulfonium trifluoromethanesulfonate (Midori Kagaku Co., Ltd.) Acid diffusion controlling agent(E)
Q-1: trioctylamine (Tokyo Kasei Kogyo Co., Ltd.) Solvent
S-1: propylene glycol monomethyl ether (Tokyo Kasei Kogyo Co., Ltd.)

### (Method for evaluating resist performance of resist composition)

A clean silicon wafer was spin coated with the homogeneous resist composition, and then prebaked (PB) before exposure in an oven of 110°C to form a resist film with a thickness of 60 nm. The obtained resist film was irradiated with electron beams of 1:1 line and space setting with a 50 nm interval using an electron beam lithography system (ELS-7500 manufactured by ELIONIX INC.). After irradiation, the resist film was heated at each predetermined temperature for 90 seconds, and immersed in 2.38% by mass TMAH alkaline developing solution for 60 seconds for development. Subsequently, the resist film was washed with ultrapure water for 30 seconds, and dried to form a positive type resist pattern. Concerning the formed resist pattern, the line and space were observed by a scanning electron microscope (S-4800 manufactured by Hitachi High-Technologies Corporation) to evaluate the reactivity by electron beam irradiation of the resist composition.

### [Table 4]

**Table 4**

| | Compound | Heat resistance evaluation | Resist composition | | | | Resist performance evaluation |
|---|---|---|---|---|---|---|---|
| | | | Compound [g] | P-1 [g] | Q-1 [g] | S-1 [g] | |
| Example 21 | BisP-1 | A | 1.0 | 0.3 | 0.03 | 50.0 | Good |
| Example 22 | BisP-2 | A | 1.0 | 0.3 | 0.03 | 50.0 | Good |
| Example 23 | BisP-3 | A | 1.0 | 0.3 | 0.03 | 50.0 | Good |
| Example 24 | BisP-4 | A | 1.0 | 0.3 | 0.03 | 50.0 | Good |
| Example 25 | BisP-5 | A | 1.0 | 0.3 | 0.03 | 50.0 | Good |
| Example 26 | BisP-6 | A | 1.0 | 0.3 | 0.03 | 50.0 | Good |
| Example 27 | BisP-7 | A | 1.0 | 0.3 | 0.03 | 50.0 | Good |
| Example 28 | BisP-8 | A | 1.0 | 0.3 | 0.03 | 50.0 | Good |
| Example 29 | BisP-9 | A | 1.0 | 0.3 | 0.03 | 50.0 | Good |
| Example 30 | BisP-10 | A | 1.0 | 0.3 | 0.03 | 50.0 | Good |
| Example 31 | BisP-11 | A | 1.0 | 0.3 | 0.03 | 50.0 | Good |
| Example 32 | BisP-12 | A | 1.0 | 0.3 | 0.03 | 50.0 | Good |
| Example 33 | BisP-13 | A | 1.0 | 0.3 | 0.03 | 50.0 | Good |
| Example 34 | BisP-14 | A | 1.0 | 0.3 | 0.03 | 50.0 | Good |
| Example 35 | BisP-15 | A | 1.0 | 0.3 | 0.03 | 50.0 | Good |
| Example 36 | BisP-16 | A | 1.0 | 0.3 | 0.03 | 50.0 | Good |
| Example 37 | BisP-17 | A | 1.0 | 0.3 | 0.03 | 50.0 | Good |
| Example 38 | BisP-18 | A | 1.0 | 0.3 | 0.03 | 50.0 | Good |
| Comparative Example 1 | CR-1 | C | 1.0 | 0.3 | 0.03 | 50.0 | Poor |

As is evident from Table 4, it was able to be confirmed that the compounds used in Examples 21 to 38 have good heat resistance whereas the compound used in Comparative Example 1 is inferior in heat resistance.

In resist pattern evaluation, a good resist pattern was obtained by irradiation with electron beams of 1:1 line and space setting with a 50 nm interval in Examples 21 to 38. On the other hand, no good resist pattern was able to be obtained in Comparative Example 1.

Thus, the compound that satisfies the requirements of the present invention has high heat resistance and can impart a good shape to a resist pattern, as compared with the comparative compound (CR-1). As long as the above requirements of the present invention are met, compounds other than those described in Examples also exhibit the same effects.

### [Examples 39 to 56 and Comparative Example 2]

### (Preparation of radiation-sensitive composition)

The components set forth in Table 5 were prepared and formed into homogeneous solutions, and the obtained homogeneous solutions were filtered through a Teflon (R) membrane filter with a pore diameter of 0.1 µm to prepare radiation-sensitive compositions. Each prepared radiation-sensitive composition was evaluated as described below.

### [Table 5]

**Table 5**

| | Composition | | |
|---|---|---|---|
| | Component (A) [g] | Optically active compound (B) [g] | Solvent [g] |
| Example 39 | BisP-1 | B-1 | S-1 |
| | 0.5 | 1.5 | 30.0 |
| Example 40 | BisP-2 | B-1 | S-1 |
| | 0.5 | 1.5 | 30.0 |
| Example 41 | BisP-3 | B-1 | S-1 |
| | 0.5 | 1.5 | 30.0 |
| Example 42 | BisP-4 | B-1 | S-1 |
| | 0.5 | 1.5 | 30.0 |
| Example 43 | BisP-5 | B-1 | S-1 |
| | 0.5 | 1.5 | 30.0 |
| Example 44 | BisP-6 | B-1 | S-1 |
| | 0.5 | 1.5 | 30.0 |
| Example 45 | BisP-7 | B-1 | S-1 |
| | 0.5 | 1.5 | 30.0 |
| Example 46 | BisP-8 | B-1 | S-1 |
| | 0.5 | 1.5 | 30.0 |
| Example 47 | BisP-9 | B-1 | S-1 |
| | 0.5 | 1.5 | 30.0 |
| Example 48 | BisP-10 | B-1 | S-1 |
| | 0.5 | 1.5 | 30.0 |
| Example 49 | BisP-11 | B-1 | S-1 |
| | 0.5 | 1.5 | 30.0 |
| Example 50 | BisP-12 | B-1 | S-1 |
| | 0.5 | 1.5 | 30.0 |
| Example 51 | BisP-13 | B-1 | S-1 |
| | 0.5 | 1.5 | 30.0 |
| Example 52 | BisP-14 | B-1 | S-1 |
| | 0.5 | 1.5 | 30.0 |
| Example 53 | BisP-15 | B-1 | S-1 |
| | 0.5 | 1.5 | 30.0 |
| Example 54 | BisP-16 | B-1 | S-1 |
| | 0.5 | 1.5 | 30.0 |
| Example 55 | BisP-17 | B-1 | S-1 |
| | 0.5 | 1.5 | 30.0 |
| Example 56 | BisP-18 | B-1 | S-1 |
| | 0.5 | 1.5 | 30.0 |
| Comparative Example 2 | PHS-1 | B-1 | S-1 |
| | 0.5 | 1.5 | 30.0 |

The following resist base material was used in Comparative Example 2.
PHS-1: polyhydroxystyrene Mw = 8000 (Sigma-Aldrich)

The following optically active compound (B) was used.
B-1: naphthoquinonediazide-based sensitizing agent of the chemical structural formula (G) (4NT-300, Toyo Gosei Co., Ltd.)

The following solvent was used.
S-1: propylene glycol monomethyl ether (Tokyo Kasei Kogyo Co., Ltd.)

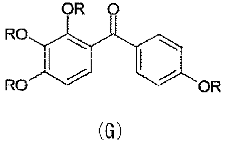

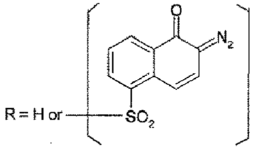

### (Evaluation of resist performance of radiation-sensitive composition)

A clean silicon wafer was spin coated with the radiation-sensitive composition obtained as described above, and then prebaked (PB) before exposure in an oven of 110°C to form a resist film with a thickness of 200 nm. The resist film was exposed to ultraviolet using an ultraviolet exposure apparatus (mask aligner MA-10 manufactured by Mikasa Co., Ltd.). The ultraviolet lamp used was a super high pressure mercury lamp (relative intensity ratio: g-ray:h-ray:i-ray:j-ray = 100:80:90:60). After irradiation, the resist film was heated at 110°C for 90 seconds, and immersed in 2.38% by mass TMAH alkaline developing solution for 60 seconds for development. Subsequently, the resist film was washed with ultrapure water for 30 seconds, and dried to form a 5 µm positive type resist pattern.

The obtained line and space were observed in the formed resist pattern by a scanning electron microscope (S-4800 manufactured by Hitachi High-Technologies Corporation). As for the line edge roughness, a pattern having asperities of less than 50 nm was evaluated as goodness.

In the case of using the radiation-sensitive compositions of Examples 39 to 56 a good resist pattern with a resolution of 5 µm was able to be obtained. The roughness of the pattern was also small and good.

On the other hand, in the case of using the radiation-sensitive composition of Comparative Example 2, a good resist pattern with a resolution of 5 µm was able to be obtained. However, the roughness of the pattern was large and poor.

As described above, it was found that a resist pattern that has small roughness and a good shape can be formed in the case of Examples 39 to 56 as compared with Comparative Example 2. As long as the above requirements of the present invention are met, radiation-sensitive compositions other than those described in Examples also exhibit the same effects.

The compounds obtained in Synthesis Working Examples 1 to 18 have a relatively low molecular weight and a low viscosity. The embedding properties and film surface flatness of underlayer film forming materials for lithography containing these compounds can be relatively advantageously enhanced. Furthermore, all of their thermal decomposition temperatures are 150°C or higher (evaluation A), and high heat resistance is retained. Therefore, the materials can be used even under high temperature baking conditions.

### [Examples 57-1 to 76-2 and Comparative Example 3]

### (Preparation of composition for forming an underlayer film for lithography)

Compositions for forming an underlayer film for lithography were prepared according to the composition shown in Table 6. Next, a silicon supporting material was spin coated with each of these compositions for forming an underlayer film for lithography, and then baked at 240°C for 60 seconds and further at 400°C for 120 seconds to prepare each underlayer film with a film thickness of 200 nm. The following acid generating agent, crosslinking agent, and organic solvent were used.
Acid generating agent: di-tertiary butyl diphenyliodonium nonafluoromethanesulfonate (DTDPI) manufactured by Midori Kagaku Co., Ltd.
Crosslinking agent: NIKALAC MX270 (NIKALAC) (Sanwa Chemical Co., Ltd.)
Organic solvent: propylene glycol monomethyl ether acetate (PGMEA)

Next, etching test was conducted under conditions shown below to evaluate etching resistance. The evaluation results are shown in Table 6.

### [Etching test]

Etching apparatus: RIE-10NR manufactured by Samco International, Inc.
Output: 50 W
Pressure: 20 Pa
Time: 2 min

Etching gas
Ar gas flow rate:CF₄ gas flow rate:O₂ gas flow rate = 50:5:5 (sccm)

### (Evaluation of etching resistance)

The evaluation of etching resistance was conducted by the following procedures. First, an underlayer film of novolac was prepared under the above conditions except that novolac (PSM4357 manufactured by Gunei Chemical Industry Co., Ltd.) was used. Then, this underlayer film of novolac was subjected to the above etching test, and the etching rate was measured.

Next, underlayer films of Examples 57-1 to 76-2 and Comparative Example 3 were subjected to the above etching test in the same way as above, and the etching rate was measured.

Then, the etching resistance was evaluated according to the following evaluation criteria on the basis of the etching rate of the underlayer film of novolac.

### [Evaluation criteria]

A: The etching rate was less than -10% as compared with the underlayer film of novolac.
B: The etching rate was -10% to +5% as compared with the underlayer film of novolac.
C: The etching rate was more than +5% as compared with the underlayer film of novolac.

### [Table 6]

**Table 6**

| | Underlayer film forming material (parts by mass) | Solvent (parts by mass) | Acid generating agent (parts by mass) | Crosslinking agent (parts by mass) | Etching resistance |
|---|---|---|---|---|---|
| Example 57-1 | BisP-1 | PGMEA | DTDPI | NIKALAC | A |
| | (10) | (90) | (0.5) | (0.5) | |
| Example 57-2 | BisP-1 | PGMEA | - | - | A |
| | (10) | (90) | | | |
| Example 58-1 | BisP-2 | PGMEA | DTDPI | NIKALAC | A |
| | (10) | (90) | (0.5) | (0.5) | |
| Example 58-2 | BisP-2 | PGMEA | - | - | A |
| | (10) | (90) | | | |
| Example 59-1 | BisP-3 | PGMEA | DTDPI | NIKALAC | A |
| | (10) | (90) | (0.5) | (0.5) | |
| Example 59-2 | BisP-3 | PGMEA | - | - | A |
| | (10) | (90) | | | |
| Example 60-1 | BisP-4 | PGMEA | DTDPI | NIKALAC | A |
| | (10) | (90) | (0.5) | (0.5) | |
| Example 60-2 | BisP-4 | PGMEA | - | - | A |
| | (10) | (90) | | | |
| Example 61-1 | BisP-5 | PGMEA | DTDPI | NIKALAC | A |
| | (10) | (90) | (0.5) | (0.5) | |
| Example 61-2 | BisP-5 | PGMEA | - | - | A |
| | (10) | (90) | | | |
| Example 62-1 | BisP-6 | PGMEA | DTDPI | NIKALAC | A |
| | (10) | (90) | (0.5) | (0.5) | |
| Example 62-2 | BisP-6 | PGMEA | - | - | A |
| | (10) | (90) | | | |
| Example 63-1 | BisP-7 | PGMEA | DTDPI | NIKALAC | A |
| | (10) | (90) | (0.5) | (0.5) | |
| Example 63-2 | BisP-7 | PGMEA | - | - | A |
| | (10) | (90) | | | |
| Example 64-1 | BisP-8 | PGMEA | DTDPI | NIKALAC | A |
| | (10) | (90) | (0.5) | (0.5) | |
| Example 64-2 | BisP-8 | PGMEA | - | - | A |
| | (10) | (90) | | | |
| Example 65-1 | BisP-9 | PGMEA | DTDPI | NIKALAC | A |
| | (10) | (90) | (0.5) | (0.5) | |
| Example 65-2 | BisP-9 | PGMEA | - | - | A |
| | (10) | (90) | | | |
| Example 66-1 | BisP-10 | PGMEA | DTDPI | NIKALAC | A |
| | (10) | (90) | (0.5) | (0.5) | |
| Example 66-2 | BisP-10 | PGMEA | - | - | A |
| | (10) | (90) | | | |
| Example 67-1 | BisP-11 | PGMEA | DTDPI | NIKALAC | A |
| | (10) | (90) | (0.5) | (0.5) | |
| Example 67-2 | BisP-11 | PGMEA | - | - | A |
| | (10) | (90) | | | |
| Example 68-1 | BisP-12 | PGMEA | DTDPI | NIKALAC | A |
| | (10) | (90) | (0.5) | (0.5) | |
| Example 68-2 | BisP-12 | PGMEA | - | - | A |
| | (10) | (90) | | | |
| Example 69-1 | BisP-13 | PGMEA | DTDPI | NIKALAC | A |
| | (10) | (90) | (0.5) | (0.5) | |
| Example 69-2 | BisP-13 | PGMEA | - | - | A |
| | (10) | (90) | | | |
| Example 70-1 | BisP-14 | PGMEA | DTDPI | NIKALAC | A |
| | (10) | (90) | (0.5) | (0.5) | |
| Example 70-2 | BisP-14 | PGMEA | - | - | A |
| | (10) | (90) | | | |
| Example 71-1 | BisP-15 | PGMEA | DTDPI | NIKALAC | A |
| | (10) | (90) | (0.5) | (0.5) | |
| Example 71-2 | BisP-15 | PGMEA | - | - | A |
| | (10) | (90) | | | |
| Example 72-1 | BisP-16 | PGMEA | DTDPI | NIKALAC | A |
| | (10) | (90) | (0.5) | (0.5) | |
| Example 72-2 | BisP-16 | PGMEA | - | - | A |
| | (10) | (90) | | | |
| Example 73-1 | BisP-17 | PGMEA | DTDPI | NIKALAC | A |
| | (10) | (90) | (0.5) | (0.5) | |
| Example 73-2 | BisP-17 | PGMEA | - | - | A |
| | (10) | (90) | | | |
| Example 74-1 | BisP-18 | PGMEA | DTDPI | NIKALAC | A |
| | (10) | (90) | (0.5) | (0.5) | |
| Example 74-2 | BisP-18 | PGMEA | - | - | A |
| | (10) | (90) | | | |
| Example 75-1 | R1-BisP-1 | PGMEA | DTDPI | NIKALAC | A |
| | (10) | (90) | (0.5) | (0.5) | |
| Example 75-2 | R1-BisP-1 | PGMEA | - | - | A |
| | (10) | (90) | | | |
| Example 76-1 | R2-BisP-1 | PGMEA | DTDPI | NIKALAC | A |
| | (10) | (90) | (0.5) | (0.5) | |
| Example 76-2 | R2-BisP-1 | PGMEA | - | - | A |
| | (10) | (90) | | | |
| Comparative Example 3 | CR-1 | PGMEA | DTDPI | NIKALAC | C |
| | (10) | (90) | (0.5) | (0.5) | |

It was found that an excellent etching rate is exerted in Examples 57-1 to 76-2 as compared with the underlayer film of novolac. On the other hand, it was found that an etching rate is poor in Comparative Example 3 as compared with the underlayer film of novolac.

### [Examples 77-1 to 96-2 and Comparative Example 4]

Next, a SiO₂ supporting material having a film thickness of 80 nm and a line and space pattern of 60 nm was coated with each of the compositions for forming an underlayer film for lithography used in Example 57-1 to Example 76-2, and baked at 240°C for 60 seconds to form a 90 nm underlayer film.

### (Evaluation of embedding properties)

The embedding properties were evaluated by the following procedures. The cross section of the film obtained under the above conditions was cut out and observed under an electron microscope to evaluate the embedding properties.

### [Evaluation criteria]

S: The underlayer film was embedded without defects in the asperities of the SiO₂ supporting material having a line and space pattern of 60 nm, and the height difference of the underlayer film in the asperities was less than 20 nm.
A: The underlayer film was embedded without defects in the asperities of the SiO₂ supporting material having a line and space pattern of 60 nm.
C: The asperities of the SiO₂ supporting material having a line and space pattern of 60 nm had defects which hindered the embedding of the underlayer film.

### [Table 7]

**Table 7**

| | Underlayer film forming material (parts by mass) | Embedding properties |
|---|---|---|
| Example 77-1 | BisP-1 | A |
| | (10) | |
| Example 77-2 | BisP-1 | S |
| | (10) | |
| Example 78-1 | BisP-2 | A |
| | (10) | |
| Example 78-2 | BisP-2 | S |
| | (10) | |
| Example 79-1 | BisP-3 | A |
| | (10) | |
| Example 79-2 | BisP-3 | S |
| | (10) | |
| Example 80-1 | BisP-4 | A |
| | (10) | |
| Example 80-2 | BisP-4 | S |
| | (10) | |
| Example 81-1 | BisP-5 | A |
| | (10) | |
| Example 81-2 | BisP-5 | S |
| | (10) | |
| Example 82-1 | BisP-6 | A |
| | (10) | |
| Example 82-2 | BisP-6 | S |
| | (10) | |
| Example 83-1 | BisP-7 | A |
| | (10) | |
| Example 83-2 | BisP-7 | S |
| | (10) | |
| Example 84-1 | BisP-8 | A |
| | (10) | |
| Example 84-2 | BisP-8 | S |
| | (10) | |
| Example 85-1 | BisP-9 | A |
| | (10) | |
| Example 85-2 | BisP-9 | S |
| | (10) | |
| Example 86-1 | BisP-10 | A |
| | (10) | |
| Example 86-2 | BisP-10 | S |
| | (10) | |
| Example 87-1 | BisP-11 | A |
| | (10) | |
| Example 87-2 | BisP-11 | S |
| | (10) | |
| Example 88-1 | BisP-12 | A |
| | (10) | |
| Example 88-2 | BisP-12 | S |
| | (10) | |
| Example 89-1 | BisP-13 | A |
| | (10) | |
| Example 89-2 | BisP-13 | S |
| | (10) | |
| Example 90-1 | BisP-14 | A |
| | (10) | |
| Example 90-2 | BisP-14 | S |
| | (10) | |
| Example 91-1 | BisP-15 | A |
| | (10) | |
| Example 91-2 | BisP-15 | S |
| | (10) | |
| Example 92-1 | BisP-16 | A |
| | (10) | |
| Example 92-2 | BisP-16 | S |
| | (10) | |
| Example 93-1 | BisP-17 | A |
| | (10) | |
| Example 93-2 | BisP-17 | S |
| | (10) | |
| Example 94-1 | BisP-18 | A |
| | (10) | |
| Example 94-2 | BisP-18 | S |
| | (10) | |
| Example 95-1 | R1-BisP-1 | A |
| | (10) | |
| Example 95-2 | R1-BisP-1 | S |
| | (10) | |
| Example 96-1 | R2-BisP-1 | A |
| | (10) | |
| Example 96-2 | R2-BisP-1 | C |
| | (10) | |
| Comparative Example 4 | CR-1 | C |
| | (10) | |

It was found that embedding properties are good in Examples 77-1 to 96-2. On the other hand, it was found that defects are seen in the asperities of the SiO₂ supporting material and embedding properties are poor in Comparative Example 4.

### [Example 97, Comparative Example 5]

Next, a SiO₂ supporting material having a film thickness of 300 nm was coated with the composition for forming an underlayer film for lithography used in Example 57-1, and baked at 240°C for 60 seconds and further at 400°C for 120 seconds to form an underlayer film having a film thickness of 85 nm. This underlayer film was coated with a resist solution for ArF and baked at 130°C for 60 seconds to form a photoresist layer with a film thickness of 140 nm.

The ArF resist solution used was prepared by containing 5 parts by mass of a compound of the formula (16) given below, 1 part by mass of triphenylsulfonium nonafluoromethanesulfonate, 2 parts by mass of tributylamine, and 92 parts by mass of PGMEA.

The compound of the formula (16) was prepared as follows. 4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure to obtain a compound represented by the following formula. 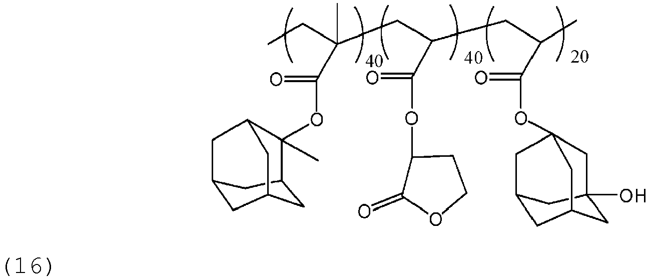 wherein 40, 40, and 20 represent the ratio of each constituent unit and do not represent a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern.

### (Comparative Example 5)

The same operations as in Example 97 were performed except that no underlayer film was formed so that a photoresist layer was formed directly on a SiO₂ supporting material to obtain a positive type resist pattern.

### [Evaluation]

Concerning each of Example 97 and Comparative Example 5, the shapes of the obtained 45 nm L/S (1:1) and 80 nm L/S (1:1) resist patterns were observed under an electron microscope manufactured by Hitachi, Ltd. (S-4800). The shapes of the resist patterns after development were evaluated as goodness when having good rectangularity without pattern collapse, and as poorness if this was not the case. The smallest line width having good rectangularity without pattern collapse as a result of this observation was used as an index for resolution evaluation. The smallest electron beam energy quantity capable of lithographing good pattern shapes was used as an index for sensitivity evaluation. The results are shown in Table 8.

### [Table 8]

**Table 8**

| | Underlayer film forming material | Resolution (nmL/S) | Sensitivity (µC/cm²) | Resist pattern shape after development |
|---|---|---|---|---|
| Example 97 | Material described in Example 57-1 | 47 | 12 | Good |
| Comparative Example 5 | None | 81 | 25 | Poor |

As is evident from Table 8, the underlayer film of Example 97 was confirmed to be significantly superior in both resolution and sensitivity to Comparative Example 5. Also, the resist pattern shapes after development were confirmed to have good rectangularity without pattern collapse. The difference in the resist pattern shapes after development indicated that the underlayer film forming material for lithography of Example 97 has good adhesiveness to a resist material.

### [Example 98]

A SiO₂ supporting material with a film thickness of 300 nm was coated with the composition for forming an underlayer film for lithography used in Example 57-1, and baked at 240°C for 60 seconds and further at 400°C for 120 seconds to form an underlayer film with a film thickness of 90 nm. This underlayer film was coated with a silicon-containing intermediate layer material and baked at 200°C for 60 seconds to form an intermediate layer film with a film thickness of 35 nm. This intermediate layer film was further coated with the above resist solution for ArF and baked at 130°C for 60 seconds to form a photoresist layer with a film thickness of 150 nm. The silicon-containing intermediate layer material used was the silicon atom-containing polymer described in <Synthesis Example 1> of Japanese Patent Laid-Open No. 2007-226170.

Subsequently, the photoresist layer was mask exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a 45 nm L/S (1:1) positive type resist pattern.

Then, the silicon-containing intermediate layer film (SOG) was dry etched with the obtained resist pattern as a mask using RIE-10NR manufactured by Samco International, Inc. Subsequently, dry etching of the underlayer film with the obtained silicon-containing intermediate layer film pattern as a mask and dry etching of the SiO₂ film with the obtained underlayer film pattern as a mask were performed in order.

Respective etching conditions are as shown below.

Conditions for etching of resist intermediate layer film with resist pattern
Output: 50 W
Pressure: 20 Pa
Time: 1 min

Etching gas
Ar gas flow rate:CF₄ gas flow rate:O₂ gas flow rate = 50:8:2 (sccm)

Conditions for etching of resist underlayer film with resist intermediate film pattern
Output: 50 W
Pressure: 20 Pa
Time: 2 min

Etching gas
Ar gas flow rate:CF₄ gas flow rate:O₂ gas flow rate = 50:5:5 (sccm)

Conditions for etching of SiO₂ film with resist underlayer film pattern
Output: 50 W
Pressure: 20 Pa
Time: 2 min

Etching gas
Ar gas flow rate:C₅F₁₂ gas flow rate:C₂F₆ gas flow rate:O₂ gas flow rate = 50:4:3:1 (sccm)

### [Evaluation]

The pattern cross section (the shape of the SiO₂ film after etching) of Example 98 obtained as described above was observed under an electron microscope manufactured by Hitachi, Ltd. (S-4800). As a result, it was confirmed that the shape of the SiO₂ film after etching in a multilayer resist process is a rectangular shape in Examples using the underlayer film of the present invention and is good without defects.

### Industrial Applicability

The present invention has industrial applicability as a compound that can be used in photoresist components, resin raw materials for materials for electric or electronic components, raw materials for curable resins such as photocurable resins, resin raw materials for structural materials, or resin curing agents, etc.

## Claims

1. A compound represented by the following formula (A): 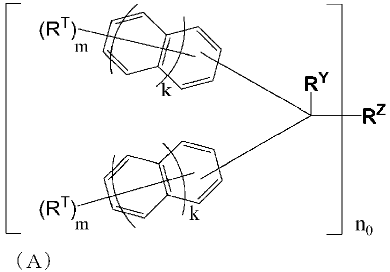 wherein
R^{Y} is a hydrogen atom, a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent or an aryl group having 6 to 30 carbon atoms optionally having a substituent;
R^{Z} is a no-valent group having 6 to 60 carbon atoms containing an aryl group having 6 to 30 carbon atoms, wherein the aryl group is substituted at least with a substituent selected from the group consisting of a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, a hydroxy group and a group in which a hydrogen atom of a hydroxy group is replaced with an acid dissociation group;
each R^{T} is independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein at least one R^{T} is a hydroxy group or a group in which a hydrogen atom of a hydroxy group is replaced with an acid dissociation group;
each m is independently an integer of 0 to 9, wherein at least one m is an integer of 1 to 9;
n₀ is an integer of 1 to 4, wherein when n₀ is an integer of 2 or larger, n₀ structural formulae within the parentheses [ ] are the same or different; and
each k is independently an integer of 0 to 2.

2. The compound according to claim 1, wherein at least one of R^{Y} and R^{Z} has a hydroxy group or a group in which a hydrogen atom of a hydroxy group is replaced with an acid dissociation group.

3. The compound according to claim 1 or 2, wherein the compound represented by the above formula (A) is a compound represented by the following formula (1): 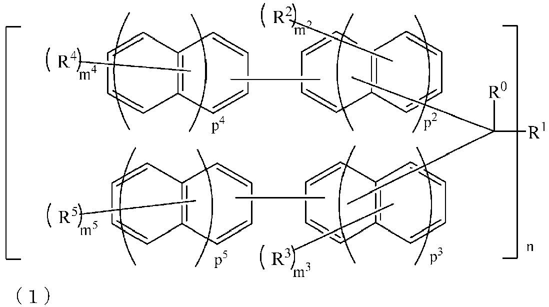 wherein
R⁰ is as defined in the above R^{Y};
R¹ is as defined in the above R^{Z};
R² to R⁵ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, an alkoxy group having 1 to 30 carbon atoms optionally having a substituent, a halogen atom, a nitro group, an amino group, a carboxyl group, a thiol group, a hydroxy group or a group in which a hydrogen atom of a hydroxy group is replaced with an acid dissociation group, wherein the alkyl group, the aryl group, the alkenyl group, and the alkoxy group each optionally have an ether bond, a ketone bond or an ester bond, wherein
at least one of R² to R⁵ is a hydroxy group or a group in which a hydrogen atom of a hydroxy group is replaced with an acid dissociation group;
m² and m³ are each independently an integer of 0 to 8;
m⁴ and m⁵ are each independently an integer of 0 to 9,
provided that m², m³, m⁴ and m⁵ are not 0 at the same time;
n is as defined in the above n₀, wherein when n is an integer of 2 or larger, n structural formulae within the parentheses [ ] are the same or different; and
p₂ to p₅ are each independently an integer of 0 to 2.

4. The compound according to claim 3, wherein at least one of R⁰ and R¹ has a hydroxy group or a group in which a hydrogen atom of a hydroxy group is replaced with an acid dissociation group.

5. The compound according to claim 3 or 4, wherein the compound represented by the above formula (1) is a compound represented by the following formula (1-1): 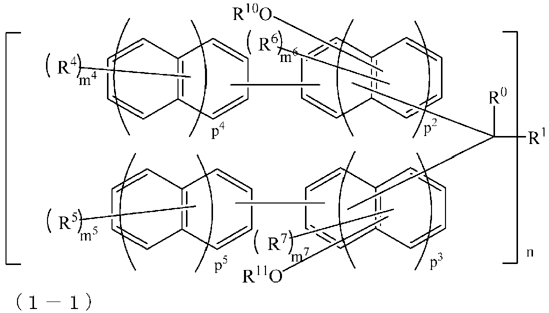 wherein
R⁰, R¹, R⁴, R⁵, n, p² to p⁵, m⁴ and m⁵ are as defined above;
R⁶ and R⁷ are each independently a linear alkyl group having 1 to 30 carbon atoms optionally having a substituent, a branched alkyl group having 3 to 30 carbon atoms optionally having a substituent, a cyclic alkyl group having 3 to 30 carbon atoms optionally having a substituent, an aryl group having 6 to 30 carbon atoms optionally having a substituent, an alkenyl group having 2 to 30 carbon atoms optionally having a substituent, a halogen atom, a nitro group, an amino group, a carboxyl group, or a thiol group;
R¹⁰ and R¹¹ are each independently an acid dissociation group or a hydrogen atom, wherein at least one of R¹⁰ and R¹¹ is a hydrogen atom; and
m⁶ and m⁷ are each independently an integer of 0 to 7.

6. The compound according to claim 5, wherein the compound represented by the above formula (1-1) is a compound represented by the following formula (1-2): 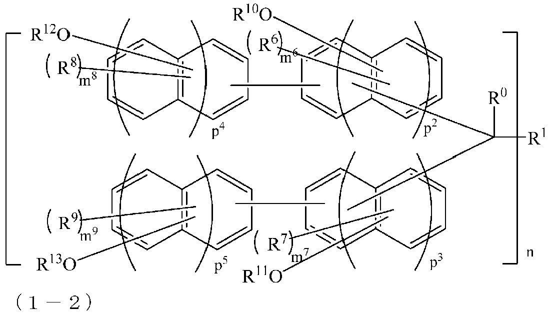 wherein
R⁰, R¹, R⁶, R⁷, R¹⁰, R¹¹, n, p² to p⁵, m⁶ and m⁷ are as defined above;
R⁸ and R⁹ are as defined in the above R⁶ and R⁷;
R¹² and R¹³ are as defined in the above R¹⁰ and R¹¹; and
m⁸ and m⁹ are each independently an integer of 0 to 8.

7. The compound according to claim 6, wherein the compound represented by the above formula (1-2) is a compound selected from the group consisting of compounds represented by the following formulae:

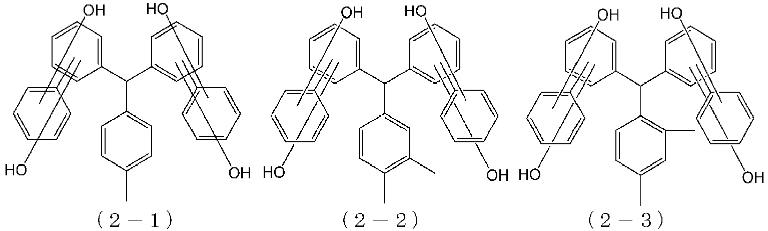

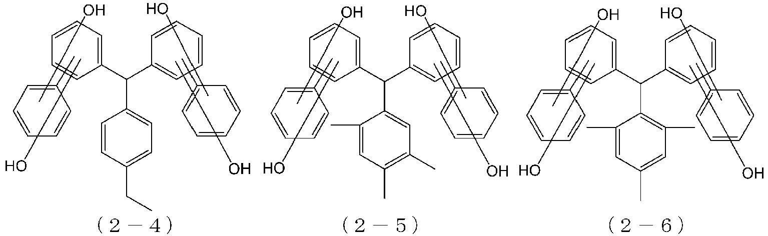

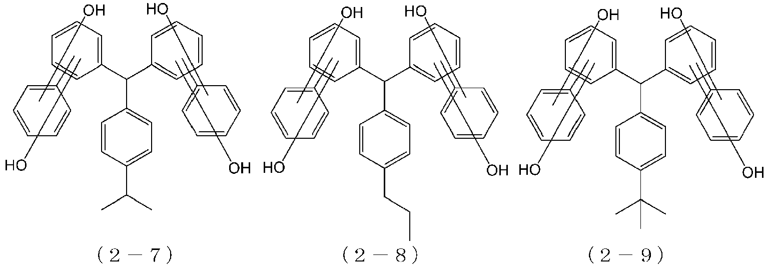

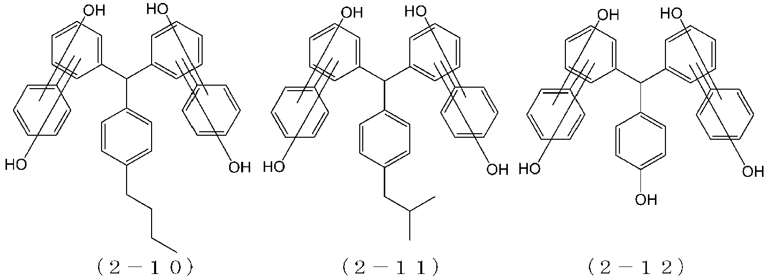

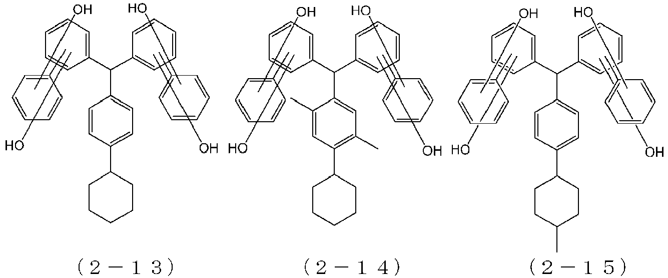

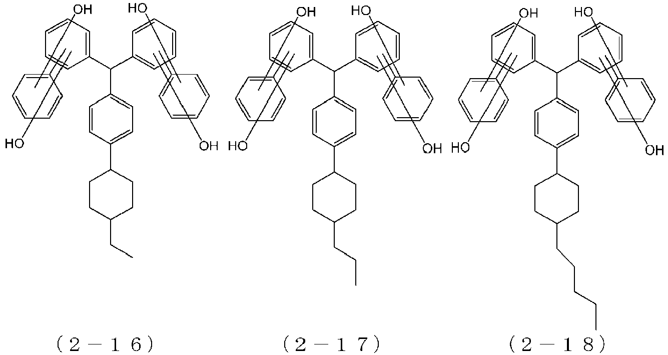

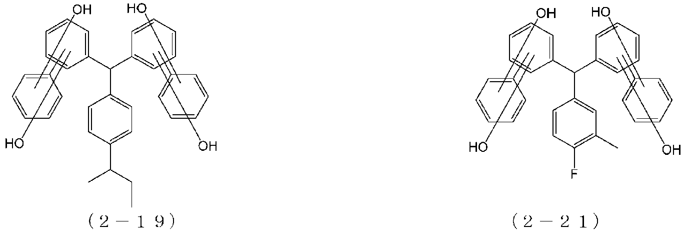

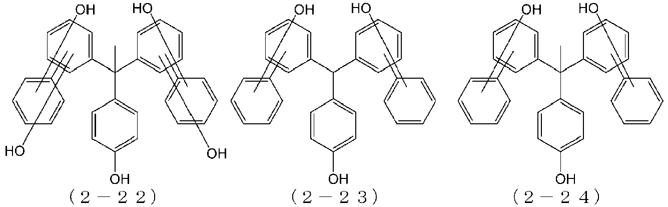

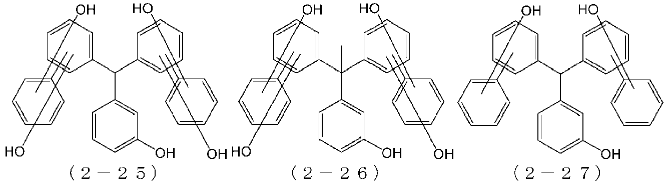

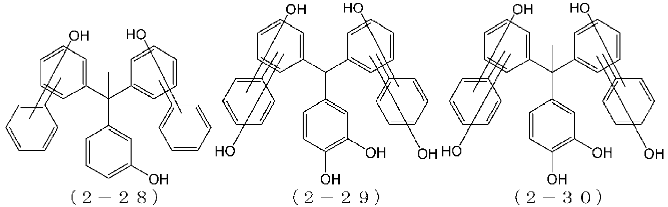

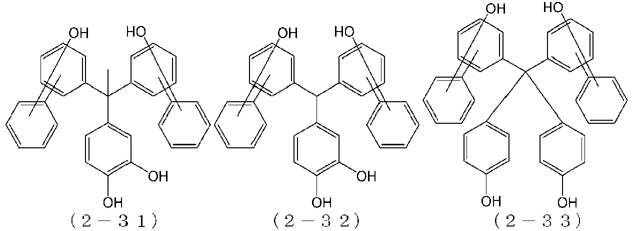

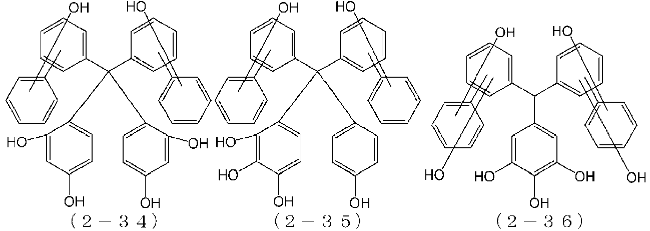

8. A resin having a constituent unit derived from the compound according to any one of claims 1 to 7.

9. A composition comprising one or more selected from the group consisting of the compound according to any one of claims 1 to 7 and the resin according to claim 8.

10. A composition for forming an optical component comprising one or more selected from the group consisting of the compound according to any one of claims 1 to 7 and the resin according to claim 8.

11. A film forming composition for lithography comprising one or more selected from the group consisting of the compound according to any one of claims 1 to 7 and the resin according to claim 8.

12. A resist composition comprising one or more selected from the group consisting of the compound according to any one of claims 1 to 7 and the resin according to claim 8.

13. The resist composition according to claim 12, further comprising a solvent.

14. The resist composition according to claim 12 or 13, further comprising an acid generating agent.

15. The resist composition according to any one of claims 12 to 14, further comprising an acid diffusion controlling agent.

16. A method for forming a resist pattern, comprising the steps of:
forming a resist film on a supporting material using the resist composition according to any one of claims 12 to 15;
exposing at least a portion of the formed resist film; and
developing the exposed resist film, thereby forming a resist pattern.

17. A radiation-sensitive composition comprising
a component (A) which is one or more selected from the group consisting of the compound according to any one of claims 1 to 7 and the resin according to claim 8,
an optically active diazonaphthoquinone compound (B), and
a solvent, wherein
the content of the solvent is 20 to 99% by mass based on 100% by mass in total of the radiation-sensitive composition, and
the content of components except for the solvent is 1 to 80% by mass based on 100% by mass in total of the radiation-sensitive composition.

18. The radiation-sensitive composition according to claim 17, wherein the content ratio among the component (A), the optically active diazonaphthoquinone compound (B), and a further optional component (D) optionally comprised in the radiation-sensitive composition ((A)/(B)/(D)) is 1 to 99% by mass/99 to 1% by mass/0 to 98% by mass based on 100% by mass of solid components of the radiation-sensitive composition.

19. The radiation-sensitive composition according to claim 17 or 18, wherein the radiation-sensitive composition is capable of forming an amorphous film by spin coating.

20. A method for producing an amorphous film, comprising the step of forming an amorphous film on a supporting material using the radiation-sensitive composition according to any one of claims 17 to 19.

21. A method for forming a resist pattern, comprising the steps of:
forming a resist film on a supporting material using the radiation-sensitive composition according to any one of claims 17 to 19;
exposing at least a portion of the formed resist film; and
developing the exposed resist film, thereby forming a resist pattern.

22. An underlayer film forming material for lithography comprising one or more selected from the group consisting of the compound according to any one of claims 1 to 7 and the resin according to claim 8.

23. A composition for forming an underlayer film for lithography comprising
the underlayer film forming material for lithography according to claim 22, and a solvent.

24. The composition for forming an underlayer film for lithography according to claim 23, further comprising an acid generating agent.

25. The composition for forming an underlayer film for lithography according to claim 23 or 24, further comprising a crosslinking agent.

26. A method for producing an underlayer film for lithography, comprising the step of forming an underlayer film on a supporting material using the composition for forming an underlayer film for lithography according to any one of claims 23 to 25.

27. A method for forming a resist pattern, comprising the steps of:
forming an underlayer film on a supporting material using the composition for forming an underlayer film for lithography according to any one of claims 23 to 25;
forming at least one photoresist layer on the underlayer film; and
irradiating a predetermined region of the photoresist layer with radiation for development, thereby forming a resist pattern.

28. A method for forming a circuit pattern, comprising the steps of:
forming an underlayer film on a supporting material using the composition for forming an underlayer film for lithography according to any one of claims 23 to 25;
forming an intermediate layer film on the underlayer film using a resist intermediate layer film material comprising a silicon atom;
forming at least one photoresist layer on the intermediate layer film;
irradiating a predetermined region of the photoresist layer with radiation for development, thereby forming a resist pattern;
etching the intermediate layer film with the resist pattern as a mask, thereby forming an intermediate layer film pattern;
etching the underlayer film with the intermediate layer film pattern as an etching mask, thereby forming an underlayer film pattern; and
etching the supporting material with the underlayer film pattern as an etching mask, thereby forming a pattern on the supporting material.

29. A purification method comprising the steps of:
obtaining a solution (S) by dissolving one or more selected from the group consisting of the compound according to any one of claims 1 to 7 and the resin according to claim 8 in a solvent; and
extracting impurities in the compound and/or the resin by bringing the obtained solution (S) into contact with an acidic aqueous solution (a first extraction step), wherein
the solvent used in the step of obtaining the solution (S) comprises a solvent that is incompatible with water.

30. The purification method according to claim 29, wherein
the acidic aqueous solution is an aqueous mineral acid solution or an aqueous organic acid solution;
the aqueous mineral acid solution is an aqueous mineral acid solution in which one or more selected from the group consisting of hydrochloric acid, sulfuric acid, nitric acid, and phosphoric acid is dissolved in water; and
the aqueous organic acid solution is an aqueous organic acid solution in which one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid is dissolved in water.

31. The purification method according to claim 29 or 30, wherein the solvent that is incompatible with water is one or more solvents selected from the group consisting of toluene, 2-heptanone, cyclohexanone, cyclopentanone, methyl isobutyl ketone, propylene glycol monomethyl ether acetate, and ethyl acetate.

32. The purification method according to any one of claims 29 to 31, comprising the step of extracting impurities in the compound and/or the resin by further bringing a solution phase comprising the compound and/or the resin into contact with water after the first extraction step (a second extraction step).
